# EUROPÄISCHE PATENTSCHRIFT

(11) **EP 2 222 844 B1**
(45) Veröffentlichungstag und Bekanntmachung des Hinweises auf die Patenterteilung: **11.02.2015**
(21) Anmeldenummer: 08860867.4
(22) Anmeldetag: 12.12.2008
(51) Int. Cl.: C12N 15/09, C08G 69/14, C08G 69/08

(54) **-AMINOCARBONSÄUREN, -AMINOCARBONSÄUREESTER ODER IHRE LACTAME HERSTELLENDE, REKOMBINANTE ZELLEN**
-AMINO CARBOXYLIC ACIDS, -AMINO CARBOXYLIC ACID ESTERS, OR RECOMBINANT CELLS WHICH PRODUCE LACTAMS THEREOF
CELLULES RECOMBINANTES FABRIQUANT DES ACIDES -AMINOCARBOXYLIQUES, DES ESTERS D'ACIDES -AMINOCARBOXYLIQUES OU LEURS LACTAMES

(30) Priorität: 17.12.2007 DE 102007060705
(43) Veröffentlichungstag der Anmeldung: 01.09.2010
(73) Patentinhaber: Evonik Degussa GmbH, 45128 Essen (DE)
(72) Erfinder: KARAU, Andreas, F-60350 Vieux Moulin (FR); SIEBER, Volker, 85405 Nandlstadt (DE); HAAS, Thomas, 48161 Münster (DE); HÄGER, Harald, 59348 Lüdinghausen (DE); GRAMMANN, Katrin, 45739 Oer-Erkenschwick (DE); BÜHLER, Bruno, 44143 Dortmund (DE); BLANK, Lars, 44227 Dortmund (DE); SCHMID, Andreas, 44229 Dortmund (DE); JACH, Guido, 53639 Königswinter (DE); LALLA, Bernd, 51065 Köln (DE); MÜLLER, Andreas, 51371 Leverkusen (DE); WELTERS, Peter, 41334 Nettetal (DE); SCHULLEHNER, Katrin, 50823 Köln (DE); EGGERT, Thorsten, 45259 Essen (DE); WECKBECKER, Andrea, 50937 Köln (DE)
(86) Internationale Anmeldenummer: PCT/EP2008/067447
(87) Internationale Veröffentlichungsnummer: WO 2009/077461

(56) Entgegenhaltungen:
- WO-A-97/44318
- BUHLER B ET AL: "Process implementation aspects for biocatalytic hydrocarbon oxyfunctionalization" JOURNAL OF BIOTECHNOLOGY, ELSEVIER SCIENCE PUBLISHERS, AMSTERDAM, NL, Bd. 113, Nr. 1-3, 30. September 2004 (2004-09-30), Seiten 183-210, XP004569608 ISSN: 0168-1656
- BÜHLER BRUNO ET AL: "Chemical biotechnology for the specific oxyfunctionalization of hydrocarbons on a technical scale." BIOTECHNOLOGY AND BIOENGINEERING 30 JUN 2003, Bd. 82, Nr. 7, 30. Juni 2003 (2003-06-30), Seiten 833-842, XP002521820 ISSN: 0006-3592
- LIU S ET AL: "Intracellular pH and metabolic activity of long-chain dicarboxylic acid-producing yeastCandida tropicalis", JOURNAL OF BIOSCIENCE AND BIOENGINEERING, ELSEVIER, AMSTERDAM, NL, vol. 96, no. 4, 1 January 2003 (2003-01-01), pages 349-353, XP004973474, ISSN: 1389-1723

### SEQUENCE LISTING

<110> Evonik Degussa GmbH
<120> omega-AMINOCARBONSÄUREN ODER IHRE LACTAME HERSTELLENDE,
   REKOMBINANTE ZELLEN
<130> 200700667
<160> 47
<170> Patentln version 3.4
<210> 1
   <211> 1380
   <212> DNA
   <213> Chromobacterium violaceum
<400> 1
   atgcagaagc aacgtacgac cagccaatgg cgcgaactgg atgccgccca tcacctgcat 60
   ccgttcaccg ataccgcatc gctgaaccag gcgggcgcgc gcgtgatgac gcgcggagag 120
   ggcgtctacc tgtgggattc ggaaggcaac aagatcatcg acggcatggc cggactgtgg 180
   tgcgtgaacg tcggctacgg ccgcaaggac tttgccgaag cggcgcgccg gcagatggaa 240
   gagctgccgt tctacaacac cttcttcaag accacccatc cggcggtggt cgagctgtcc 300
   agcctgctgg ctgaagtgac gccggccggt ttcgaccgcg tgttctatac caattccggt 360
   tccgaatcgg tggacaccat gatccgcatg gtgcgccgct actgggacgt gcagggcaag 420
   ccggagaaga agacgctgat cggccgctgg aacggctatc acggctccac catcggcggc 480
   gccagcctgg gcggcatgaa gtacatgcac gagcagggcg acttgccgat tccgggcatg 540
   gcccacatcg agcagccttg gtggtacaag cacggcaagg acatgacgcc ggacgagttc 600
   ggcgtggtgg ccgcgcgctg gctggaagag aagattctgg aaatcggcgc cgacaaggtg 660
   gccgccttcg tcggcgaacc catccagggc gccggcggcg tgatcgtccc gccggccacc 720
   tactggccgg aaatcgagcg catttgccgc aagtacgacg tgctgctggt ggccgacgaa 780
   gtgatctgcg gcttcgggcg taccggcgaa tggttcggcc atcagcattt cggcttccag 840
   cccgacctgt tcaccgccgc caagggcctg tcctccggct atctgccgat aggcgcggtc 900
   tttgtcggca agcgcgtggc cgaaggcctg atcgccggcg gcgacttcaa ccacggcttc 960
   acctactccg gccacccggt ctgcgccgcc gtcgcccacg ccaacgtggc ggcgctgcgc 1020
   gacgagggca tcgtccagcg cgtcaaggac gacatcggcc cgtacatgca aaagcgctgg 1080
   cgtgaaacct tcagccgttt cgagcatgtg gacgacgtgc gcggcgtcgg catggtgcag 1140
   gcgttcaccc tggtgaagaa caaggcgaag cgcgagctgt tccccgattt cggcgagatc 1200
   ggcacgctgt gccgcgacat cttcttccgc aacaacctga tcatgcgggc atgcggcgac 1260
   cacatcgtgt cggcgccgcc gctggtgatg acgcgggcgg aagtggacga gatgctggcg 1320
   gtggcggaac gctgtctgga ggaattcgag cagacgctga aggcgcgcgg gctggcttag 1380
<210> 2
   <211> 1137
   <212> DNA
   <213> Bacillus subtilis
<400> 2
   atgatcatag gggttcctaa agagataaaa aacaatgaaa accgtgtcgc attaacaccc 60
   gggggcgttt ctcagctcat ttcaaacggc caccgggtgc tggttgaaac aggcgcgggc 120
   cttggaagcg gatttgaaaa tgaagcctat gagtcagcag gagcggaaat cattgctgat 180
   ccgaagcagg tctgggacgc cgaaatggtc atgaaagtaa aagaaccgct gccggaagaa 240
   tatgtttatt ttcgcaaagg acttgtgctg tttacgtacc ttcatttagc agctgagcct 300
   gagcttgcac aggccttgaa ggataaagga gtaactgcca tcgcatatga aacggtcagt 360
   gaaggccgga cattgcctct tctgacgcca atgtcagagg ttgcgggcag aatggcagcg 420
   caaatcggcg ctcaattctt agaaaagcct aaaggcggaa aaggcattct gcttgccggg 480
   gtgcctggcg tttcccgcgg aaaagtaaca attatcggag gaggcgttgt cgggacaaac 540
   gcggcgaaaa tggctgtcgg cctcggtgca gatgtgacga tcattgactt aaacgcagac 600
   cgcttgcgcc agcttgatga catcttcggc catcagatta aaacgttaat ttctaatccg 660
   gtcaatattg ctgatgctgt ggcggaagcg gatctcctca tttgcgcggt attaattccg 720
   ggtgctaaag ctccgactct tgtcactgag gaaatggtaa aacaaatgaa acccggttca 780
   gttattgttg atgtagcgat cgaccaaggc ggcatcgtcg aaactgtcga ccatatcaca 840
   acacatgatc agccaacata tgaaaaacac ggggttgtgc attatgctgt agcgaacatg 900
   ccaggcgcag tccctcgtac atcaacaatc gccctgacta acgttactgt tccatacgcg 960
   ctgcaaatcg cgaacaaagg ggcagtaaaa gcgctcgcag acaatacggc actgagagcg 1020
   ggtttaaaca ccgcaaacgg acacgtgacc tatgaagctg tagcaagaga tctaggctat 1080
   gagtatgttc ctgccgagaa agctttacag gatgaatcat ctgtggcggg tgcttaa 1137
<210> 3
   <211> 1206
   <212> DNA
   <213> Pseudomonas putida
<400> 3
   atgcttgaga aacacagagt tctggattcc gctccagagt acgtagataa aaagaaatat 60
   ctctggatac tatcaacttt gtggccggct actccgatga tcggaatctg gcttgcaaat 120
   gaaactggtt gggggatttt ttatgggctg gtattgctcg tatggtacgg cgcacttcca 180
   ttgcttgatg cgatgtttgg tgaggacttt aataatccgc ctgaagaagt ggtgccgaaa 240
   ctagagaagg agcggtacta tcgagttttg acatatctaa cagttcctat gcattacgct 300
   gcattaattg tgtcagcatg gtgggtcgga actcagccaa tgtcttggct tgaaattggt 360
   gcgcttgcct tgtcactggg tatcgtgaac ggactagcgc tcaatacagg acacgaactc 420
   ggtcacaaga aggagacttt tgatcgttgg atggccaaaa ttgtgttggc tgtcgtaggg 480
   tacggtcact tctttattga gcataataag ggtcatcacc gtgatgtcgc tacaccgatg 540
   gatcctgcaa catcccggat gggagaaagc atttataagt tttcaatccg tgagatccca 600
   ggagcattta ttcgtgcttg ggggcttgag gaacaacgcc tttcgcgccg tggccaaagc 660
   gtttggagtt tcgataatga aatcctccaa ccaatgatca tcacagttat tctttacgcc 720
   gttctccttg ccttgtttgg acctaagatg ctggtgttcc tgccgattca aatggctttc 780
   ggttggtggc agctgaccag tgcgaactat attgaacatt acggcttgct ccgtcaaaaa 840
   atggaggacg gtcgatatga gcatcaaaag ccgcaccatt cttggaatag taatcacatc 900
   gtctctaatc tagtgctgtt ccaccttcag cggcactcgg atcaccacgc gcatccaaca 960
   cgttcttatc agtcacttcg ggattttccc ggcctgccgg ctcttccgac gggttaccct 1020
   ggtgcatttt tgatggcgat gattcctcag tggtttagat cagttatgga tcccaaggta 1080
   gtagattggg ctggtggtga ccttaataag atccaaattg atgattcgat gcgagaaacc 1140
   tatttgaaaa aatttggcac tagtagtgct ggtcatagtt cgagtacctc tgcggtagca 1200
   tcgtag 1206
<210> 4
   <211> 519
   <212> DNA
   <213> Pseudomonas putida
<400> 4
   atggctagct ataaatgccc ggattgtaat tatgtttatg atgagagtgc gggtaatgtg 60
   catgaggggt tttctccagg tacgccttgg caccttattc ctgaggattg gtgctgcccc 120
   gattgcgccg ttcgagacaa gcttgacttc atgttaattg agagcggcgt aggtgaaaag 180
   ggcgtcacct caacccatac ttcgccaaat ttatccgagg ttagtggcac aagtttaact 240
   gctgaagcag tggttgcgcc gacaagctta gagaaattgc ctagtgccga cgttaaaggc 300
   caagatctat ataaaactca acctccaagg tctgatgccc aaggcgggaa agcatacttg 360
   aagtggatat gtattacttg tggccatata tatgatgagg cgttgggcga tgaggccgag 420
   ggttttactc caggtactcg ctttgaggat attcctgatg actggtgctg tccggattgc 480
   ggggctacga aagaagacta tgtgctctac gaggaaaag 519
<210> 5
   <211> 1677
   <212> DNA
   <213> Pseudomonas putida
<400> 5
   atgtacgact atataatcgt tggtgctgga tctgcaggat gtgtgcttgc taatcgtctt 60
   tcggccgacc cctctaaaag agtttgttta cttgaagctg ggccgcgaga tacgaatccg 120
   ctaattcata tgccgttagg tattgctttg ctttcaaata gtaaaaagtt gaattgggct 180
   tttcaaactg cgccacagca aaatctcaac ggccggagcc ttttctggcc acgaggaaaa 240
   acgttaggtg gttcaagctc aatcaacgca atggtctata tccgagggca tgaagacgat 300
   taccacgcat gggagcaggc ggccggccgc tactggggtt ggtaccgggc tcttgagttg 360
   ttcaaaaggc ttgaatgcaa ccagcgattc gataagtccg agcaccatgg ggttgacgga 420
   gaattagctg ttagtgattt aaaatatatc aatccgctta gcaaagcatt cgtgcaagcc 480
   ggcatggagg ccaatattaa tttcaacgga gatttcaacg gcgagtacca ggacggcgta 540
   gggttctatc aagtaaccca aaaaaatgga caacgctgga gctcggcgcg tgcattcttg 600
   cacggtgtac tttccagacc aaatctagac atcattactg atgcgcatgc atcaaaaatt 660
   ctttttgaag accgtaaggc ggttggtgtt tcttatataa agaaaaatat gcaccatcaa 720
   gtcaagacaa cgagtggtgg tgaagtactt cttagtcttg gcgcagtcgg cacgcctcac 780
   cttctaatgc tttctggtgt tggggctgca gccgagctta aggaacatgg tgtttctcta 840
   gtccatgatc ttcctgaggt ggggaaaaat cttcaagatc atttggacat cacattgatg 900
   tgcgcagcaa attcgagaga gccgataggt gttgctcttt ctttcatccc tcgtggtgtc 960
   tcgggtttgt tttcatatgt gtttaagcgc gaggggtttc tcactagtaa cgtggcagag 1020
   tcgggtggtt ttgtaaaaag ttctcctgat cgtgatcggc ccaatttgca gtttcatttc 1080
   cttccaactt atcttaaaga tcacggtcga aaaatagcgg gtggttatgg ttatacgcta 1140
   catatatgtg atcttttgcc taagagccga ggcagaattg gcctaaaaag cgccaatcca 1200
   ttacagccgc ctttaattga cccgaactat cttagcgatc atgaagatat taaaaccatg 1260
   attgcgggta ttaagatagg gcgcgctatt ttgcaggccc catcgatggc gaagcatttt 1320
   aagcatgaag tagtaccggg ccaggctgtt aaaactgatg atgaaataat cgaagatatt 1380
   cgtaggcgag ctgagactat ataccatccg gtaggtactt gtaggatggg taaagatcca 1440
   gcgtcagttg ttgatccgtg cctgaagatc cgtgggttgg caaatattag agtcgttgat 1500
   gcgtcaatta tgccgcactt ggtcgcgggt aacacaaacg ctccaactat tatgattgca 1560
   gaaaatgcgg cagaaataat tatgcggaat cttgatgtgg aagcattaga ggctagcgct 1620
   gagtttgctc gcgagggtgc agagctagag ttggccatga tagctgtctg catgtaa 1677
<210> 6
   <211> 2649
   <212> DNA
   <213> Pseudomonas putida
<400> 6
   atgaaaataa taataaataa tgatttcccg gtcgctaagg tcggagcgga tcaaattacg 60
   actctagtaa gtgccaaagt tcatagttgc atatatcggc caagattgag tatcgcggat 120
   ggagccgctc ccagagtatg cctttacaga gccccacctg gatatgggaa aaccgttgct 180
   cttgcgttcg agtggctacg ccacagaaca gccggacgtc ctgcagtgtg gctttcttta 240
   agagccagtt cttacagtga atttgatatc tgcgcagaga ttattgagca gcttgaaact 300
   ttcgaaatgg taaaattcag ccgtgtgaga gagggtgtga gcaagcctgc gctcttgcga 360
   gaccttgcat ctagtctttg gcagagcacc tcgaataacg agatagaaac gctagtttgt 420
   ttggataata ttaatcatga cttagacttg ccgttgttgc acgcacttat ggagtttatg 480
   ttaaatacac caaaaaatat caggtttgca gttgcaggca atacaataaa agggttctcg 540
   cagcttaaac ttgcaggcgc tatgcgggag tacaccgaga aagacttggc ctttagcgca 600
   gaagaggcgg tggcgttagc ggaggcagag tctgttcttg gagttcctga agaacagata 660
   gagaccttgg tgcaagaagt tgaggggtgg cctgctcttg tagttttttt gttaaagcgt 720
   gagttgccgg ccaagcatat ttcagcagta gttgaagtag acaattactt tagggatgaa 780
   atatttgagg cgattcccga gcgctatcgt gtttttcttg caaattcttc attgctcgat 840
   ttcgtgacgc ctgatcaata caattatgta ttcaaatgcg tcaatggggt ctcatgtatt 900
   aagtatttaa gcactaatta catgttgctt cgccatgtga gcggtgagcc agcgcagttt 960
   acactgcatc cagtactgcg taattttcta cgagaaatta cttggactga aaatcctgct 1020
   aaaagatcct acctgcttaa gcgtgcagct ttctggcatt ggcgtagagg tgaataccag 1080
   tatgcaatac gaatatccct acgggcgaat gactgtcgct gggcagtcag catgtctgag 1140
   agaataattt tagatttgtc atttcgtcag ggcgaaatag atgcgctgag acagtggctg 1200
   ttagagctgc cgaagcaggc ctggcaccaa aaacccatag tgcttattag ttacgcgtgg 1260
   gtattgtatt tcagtcagca aggcgcgcga gcagagaagt taattaaaga cctatcttca 1320
   caatccgata aaaaaaataa atggcaagaa aaggaatggc tgcagcttgt gcttgcaata 1380
   ggtaaagcaa ccaaagatga aatgctttcg agtgaggagc tctgtaataa gtggattagt 1440
   ttatttgggg attcaaacgc agttggaaaa ggggccgcgc taacctgttt ggcttttatt 1500
   tttgccagtg agtatagatt tgcagagttg gagaaggtgc tggctcaggc ccaagccgtg 1560
   aataaatttg caaaacaaaa ttttgctttt ggttggctgt atgtcgcgag gtttcaacaa 1620
   gccctagcaa gcggaaaaat gggctgggcg aggcagatta taactcaagc acgcacagac 1680
   agtcgcgcgc agatgatgga atccgagttt acttcgaaaa tgtttgacgc tctagagctt 1740
   gagttacatt atgaattgcg ctgcttggac acctcagaag aaaagctctc caaaatttta 1800
   gagttcattt ccaatcacgg ggtgacagac gtgttttttt ccgtatgccg tgctgtgtca 1860
   gcttggcggc ttggaaggag tgacctaaat ggctccattg agatattgga gtgggcgaag 1920
   gcgcatgcgg ttgaaaaaaa tctaccaaga ttggaagtta tgagccaaat tgagatctat 1980
   cagcgcttag tctgtcaagg cataacgggc ataaataatt taaaaactct tgaagatcat 2040
   aagattttct ccggacagca ctcagccccc ctaaaagcac gcctgctgct tgttcaatca 2100
   ctagtgcttt cccgagatcg gaactttcat agtgccgcgc acagagcgtt attggctatt 2160
   cagcaagccc gtaaaattaa cgcgggccag ctggaagtcc gtggattatt gtgtttggcc 2220
   ggagcgcagg caggtgccgg tgatttaaaa aaggctcagc ttaacattgt ttatgcagtg 2280
   gagatagcaa aacagcttca atgctttcaa acagttcttg atgaagtatg tttaattgag 2340
   cgaataatac cggcttcatg tgaagccttc acagcagtta atttagatca agcgattggg 2400
   gcttttagtc ttccgcgaat agttgagatt ggaaagtccg cagagaataa agctgacgct 2460
   ttattgacac ggaagcagat tgctgtcttg aggcttgtaa aagaggggtg ctcaaacaaa 2520
   caaatagcaa caaatatgca tgtcaccgaa gatgctataa agtggcatat gaggaaaata 2580
   tttgccacct tgaatgtagt gaatcgcacg caagcaacaa ttgaagctga gcgtcaagga 2640
   attatctaa 2649
<210> 7
   <211> 1158
   <212> DNA
   <213> Pseudomonas putida
<400> 7
   atggcaatcg ttgttgttgg cgctggtaca gctggagtaa atgctgcgtt ctggcttcgt 60
   caatatggtt ataaagggga aattaggatt tttagcaggg agtctgtggc gccttatcag 120
   cggcctcctc tatccaaggc ttttctgaca agtgagattg cagaatccgc agtgccatta 180
   aagccagaag gtttttatac gaataacaat attaccattt cgttaaatac accgattgta 240
   tcaatcgacg tggggcgtaa gatagtttct tctaaagatg gaaaagaata cgcgtatgaa 300
   aaattgattc ttgcaacacc tgctagcgca cgtaggttaa cctgcgaggg gtctgaactg 360
   tctggggtct gctatttacg cagtatggaa gacgccaaaa atttacgtag gaaacttgtg 420
   gagagtgcgt ctgttgttgt gttgggcggc ggagtaatcg ggcttgaagt cgcctcagct 480
   gcggtgggct tagggaagag ggtcacagtg atagaagcca ccccgcgtgt aatggcgcgc 540
   gtggttacgc cggcagcagc aaacttagtc agagcccgcc tggaggctga aggaattgag 600
   ttcaagctga atgcgaaatt aacgtctata aagggcagga atggccatgt tgaacaatgc 660
   gtacttgaaa gtggagaaga aattcaggcg gatctgattg tagttggaat cggtgctatc 720
   ccagagctag agctggcaac tgaggcggcc cttgaagtga gtaatggtgt tgtggtcgat 780
   gatcagatgt gtacatcgga tacaagtata tatgcaatcg gcgactgcgc aatggctaga 840
   aatccttttt ggggaacgat ggtacgttta gagacaattc ataatgcggt tacacacgct 900
   caaattgtcg caagtagcat ctgtggcaca tcaacaccag caccaacccc accacggttc 960
   tggtctgatc ttaaagggat ggcgctgcaa ggacttggtg ctctaaagga ctacgataaa 1020
   ctcgttgttg caattaataa cgaaactctt gaactagaag tccttgcgta caagcaggag 1080
   cgactgattg caactgagac aataaatttg cctaaacgtc aaggtgcgct tgcagggagt 1140
   ataaaattac ctgattag 1158
<210> 8
   <211> 33
   <212> DNA
   <213> Artificial Sequence
<220>
   <223> Primer
<400> 8
   acgtagcggc cgcctaatca ggtaatttta tac 33
<210> 9
   <211> 16
   <212> DNA
   <213> Artificial Sequence
<220>
   <223> Primer
<400> 9
   gagcgagcta tctggt 16
<210> 10
   <211> 32
   <212> DNA
   <213> Artificial Sequence
<220>
   <223> Primer
<400> 10
   tcgtagcggc cgcccagcag acgacggagc aa 32
<210> 11
   <211> 37
   <212> DNA
   <213> Artificial Sequence
<220>
   <223> Primer
<400> 11
   attttatctt ctcgaggctt ttcctcgtag agcacat 37
<210> 12
   <211> 38
   <212> DNA
   <213> Artificial Sequence
<220>
   <223> Primer
<400> 12
   tgctctaacg aggaaaagcc tcgagaagat aaaatgta 38
<210> 13
   <211> 34
   <212> DNA
   <213> Artificial Sequence
<220>
   <223> Primer
<400> 13
   attgacgcgg ccgcttacat gcagacagct atca 34
<210> 14
   <211> 42
   <212> DNA
   <213> Artificial Sequence
<220>
   <223> Primer
<400> 14
   cgaggagctc aggaggatcc aagcatgcag aagcaacgta cg 42
<210> 15
   <211> 31
   <212> DNA
   <213> Artificial Sequence
<220>
   <223> Primer
<400> 15
   gtcatgtacc cctaagccag cccgcgcgcc t 31
<210> 16
   <211> 41
   <212> DNA
   <213> Artificial Sequence
<220>
   <223> Primer
<400> 16
   acctatctag aaggaggacg catatgatca taggggttcc t 41
<210> 17
   <211> 26
   <212> DNA
   <213> Artificial Sequence
<220>
   <223> Primer
<400> 17
   aacctctgca gttaagcacc cgccac 26
<210> 18
   <211> 1853
   <212> DNA
   <213> Pseudomonas fluoreszenz
<400> 18
   atgggtgtgt atgactacaa aaacttcggc acggcggatt ccaaggcgtt gttcagcgat 60
   gccatggcga tcacgctgta ttcctaccac aacctcgata acggttttgc cgccggttat 120
   agcacaacgg ttttggcctt ggcctgccgg cgacgctggt cacggcgttg ctcggcggta 180
   ccgattccca gggcgtcatc cccggcattc cgtggaatcc cgattcggaa aaactcgccc 240
   tcgaagccgt gaaaaaggcc ggctggacgc cgatcacggc ctcgcaactg ggctacgacg 300
   gcaagaccga cgcacgcgga accttctttg gcgagaaggc cggttactcg acagcgcagg 360
   tcgagattct cggcaagtac gacgcccagg gccatctcac agaaatcggc atcgcctttc 420
   gcggcaccag cggcccgcgc gagaacctga tccttgattc catcggcgac gtgatcaacg 480
   acttgctcgc cgcgttcggc cccaaggatt acgccaagaa ctacgtcggc gaagcgttcg 540
   gcaacctgct caatgacgtg gtcgcctttg ccaaggccaa tggcctcagc ggcaaggacg 600
   tgctggtcag cggccacagc ctcggcgggc tggcggtcaa cagcatggcg gatttgagcg 660
   gcggcaagtg gggcgggttc ttcgccgact ccaactacat cgcctatgcc tcgccgaccc 720
   agagcagcac cgacaaagtg ctcaacgtcg gctacgagaa cgacccggtg ttccgcgccc 780
   tcgacggttc gaatttcacc ggcgcctcga ttggcgtgca cgacgcgccg aaggaatcgg 840
   ccaccgacaa catcgtcagc ttcaacgatc actacgcctc gacggcgtgg aatctgctgc 900
   cgttctccat cctcaacatc ccgacctgga tctcgcacct gccaaccgct tacggcgacg 960
   gcatgaaccg ggtgatcgag tcgaagttct acgacctgac cagcaaggac tcgacgatca 1020
   tcgtcgccaa cctgtcggat ccggcgcggg ccaacacctg ggtgcaggat ctcaaccgca 1080
   acgccgaaac ccacaagggc agcaccttca tcatcggcag cgacgccaac gatctgattc 1140
   agggtggcag cggcaatgac tatctggaag gtcgcgccgg caacgacacc tttcgcgaca 1200
   gcggcggcta caacatcatc ctgggcgggc agggcagcaa tacgctggac ttgcagaagt 1260
   cggtgaatac cttcgacttc gccaacgacg gcgccggcaa tctgtacatt cgcgatgcca 1320
   acggcgggat cagcatcacc cgcgacatcg gcgccatcgt caccaaagag ccgggcttcc 1380
   tctggggtct gttcaaggac gacgtgaccc acagcgtcac ggccagtggc ttgaaggtcg 1440
   gcaacaacct gaccgcctac gagtcgagcg tgaagggcag caacggcgcc gacacgctca 1500
   aggcgcatgc cggcggcgac tggttgttcg gcctcgacgg caacgatcat ctgatcggcg 1560
   gggcgggcaa cgatgtgttt gttggcggcg ccggtaacga tctgatggag tccgggggcg 1620
   gggcggatac gttcctgttc aacggcgcgt tcggccagga tcgggtggtg ggattcacgt 1680
   ccaacgacaa actggtgttt ctcggcgtgc agggtgtgtt gcctggcgat gacttccgag 1740
   cgcatgcctc ggcagccggg caggataccg tgctgaagtt cggcggcgat tcggtgacat 1800
   tggttggcgt ttcgctgggg agtttgagtg gcgatggaat tgtgatcgcc tga 1853
<210> 19
   <211> 503
   <212> PRT
   <213> Pseudomonas putida W619
<400> 19
   Met Ser Lys Ser Leu Thr Trp Ala Arg Gly Ser Gly Arg Arg Leu Ile
   1 5 10 15
   Ser Leu Val Leu Arg Tyr Leu Lys Ile Arg Asn Val Val Phe Phe Arg
   20 25 30
   Leu Tyr Pro Arg Ala Asn Leu Ala Ala Thr Thr Ser Ile Val Ala Tyr
   35 40 45
   Ser Phe Phe Glu Glu Pro Pro Met Asn Met Pro Glu Thr Ala Pro Ala
   50 55 60
   Gly Ile Ala Ser Gln Leu Lys Leu Asp Ala His Trp Met Pro Tyr Thr
   65 70 75 80
   Ala Asn Arg Asn Phe His Arg Asp Pro Arg Leu Ile Val Ala Ala Glu
   85 90 95
   Gly Asn Tyr Leu Val Asp Asp Lys Gly Arg Arg Ile Phe Asp Ala Leu
   100 105 110
   Ser Gly Leu Trp Thr Cys Gly Ala Gly His Thr Arg Lys Glu Ile Thr
   115 120 125
   Glu Ala Val Ala Arg Gln Leu Gly Thr Leu Asp Tyr Ser Pro Ala Phe
   130 135 140
   Gln Phe Gly His Pro Leu Ser Phe Gln Leu Ala Glu Lys Ile Thr Ala
   145 150 155 160
   Leu Thr Pro Gly Asp Leu Asn His Val Phe Tyr Thr Asn Ser Gly Ser
   165 170 175
   Glu Cys Ala Asp Thr Ala Leu Lys Met Val Arg Ala Tyr Trp Arg Leu
   180 185 190
   Lys Gly Gln Ala Thr Lys Thr Lys Ile Ile Gly Arg Ala Arg Gly Tyr
   195 200 205
   His Gly Val Asn Ile Ala Gly Thr Ser Leu Gly Gly Val Asn Gly Asn
   210 215 220
   Arg Lys Met Phe Gly Gln Leu Leu Asp Val Asp His Leu Pro His Thr
   225 230 235 240
   Val Leu Pro Val Asn Ala Phe Ser Lys Gly Leu Pro Glu Glu Gly Gly
   245 250 255
   Ile Ala Leu Ala Asp Glu Met Leu Lys Leu Ile Glu Leu His Asp Ala
   260 265 270
   Ser Asn Ile Ala Ala Val Ile Val Glu Pro Leu Ala Gly Ser Ala Gly
   275 280 285
   Val Leu Pro Pro Pro Lys Gly Tyr Leu Lys Arg Leu Arg Glu Ile Cys
   290 295 300
   Thr Gln His Asn Ile Leu Leu Ile Phe Asp Glu Val Ile Thr Gly Phe
   305 310 315 320
   Gly Arg Met Gly Ala Met Thr Gly Ala Glu Ala Phe Gly Val Thr Pro
   325 330 335
   Asp Leu Met Cys Ile Ala Lys Gln Val Thr Asn Gly Ala Ile Pro Met
   340 345 350
   Gly Ala Val Ile Ala Ser Ser Glu Ile Tyr Gln Thr Phe Met Asn Gln
   355 360 365
   Pro Thr Pro Glu Tyr Ala Val Glu Phe Pro His Gly Tyr Thr Tyr Ser
   370 375 380
   Ala His Pro Val Ala Cys Ala Ala Gly Ile Ala Ala Leu Asp Leu Leu
   385 390 395 400
   Gln Arg Glu Asn Leu Val Gln Ser Ala Ala Glu Leu Ala Pro His Phe
   405 410 415
   Glu Lys Leu Leu His Gly Val Lys Gly Thr Lys Asn Val Val Asp Ile
   420 425 430
   Arg Asn Tyr Gly Leu Ala Gly Ala Ile Gln Ile Ala Ala Arg Asp Gly
   435 440 445
   Asp Ala Ile Val Arg Pro Tyr Glu Val Ala Met Lys Leu Trp Lys Ala
   450 455 460
   Gly Phe Tyr Val Arg Phe Gly Gly Asp Thr Leu Gln Phe Gly Pro Thr
   465 470 475 480
   Phe Asn Thr Thr Pro Gln Gln Leu Asp Arg Leu Phe Asp Ala Val Gly
   485 490 495
   Glu Asn Leu Asn Leu Ile Asp
   500
<210> 20
   <211> 460
   <212> PRT
   <213> Pseudomonas aeruginosa PA01
<400> 20
   Met Thr Ala Gln Leu Asn Pro Gln Arg Asp Thr Arg Asp Tyr Gln Gln
   1 5 10 15
   Leu Asp Ala Ala His His Ile His Ala Phe Leu Asp Gln Lys Ala Leu
   20 25 30
   Asn Arg Glu Gly Pro Arg Val Met Val Arg Gly Asp Gly Leu Gln Leu
   35 40 45
   Trp Asp Asn Asp Gly Lys Arg Tyr Leu Asp Gly Met Ser Gly Leu Trp
   50 55 60
   Cys Thr Asn Leu Gly Tyr Gly Arg Gln Asp Leu Ala Ala Ala Ala Ser
   65 70 75 80
   Arg Gln Leu Glu Gln Leu Pro Tyr Tyr Asn Met Phe Phe His Thr Thr
   85 90 95
   His Pro Ala Val Val Glu Leu Ser Glu Met Leu Phe Ser Leu Leu Pro
   100 105 110
   Asp His Tyr Ser His Ala Ile Tyr Thr Asn Ser Gly Ser Glu Ala Asn
   115 120 125
   Glu Val Leu Ile Arg Thr Val Arg Arg Tyr Trp Gln Ile Leu Gly Lys
   130 135 140
   Pro Gln Lys Lys Ile Met Ile Gly Arg Trp Asn Gly Tyr His Gly Ser
   145 150 155 160
   Thr Leu Gly Ser Thr Ala Leu Gly Gly Met Lys Phe Met His Glu Met
   165 170 175
   Gly Gly Met Leu Pro Asp Phe Ala His Ile Asp Glu Pro Tyr Trp Tyr
   180 185 190
   Ala Asn Gly Gly Glu Leu Ser Pro Ala Glu Phe Gly Arg Arg Ala Ala
   195 200 205
   Leu Gln Leu Glu Glu Lys Ile Leu Glu Leu Gly Ala Glu Asn Val Ala
   210 215 220
   Ala Phe Val Ala Glu Pro Phe Gln Gly Ala Gly Gly Met Ile Phe Pro
   225 230 235 240
   Pro Gln Ser Tyr Trp Pro Glu Ile Gln Arg Ile Cys Arg Gln Tyr Asp
   245 250 255
   Val Leu Leu Cys Ala Asp Glu Val Ile Gly Gly Phe Gly Arg Thr Gly
   260 265 270
   Glu Trp Phe Ala His Glu His Phe Gly Phe Gln Pro Asp Thr Leu Ser
   275 280 285
   Ile Ala Lys Gly Leu Thr Ser Gly Tyr Ile Pro Met Gly Gly Leu Val
   290 295 300
   Leu Gly Lys Arg Ile Ala Glu Val Leu Val Glu Gln Gly Gly Val Phe
   305 310 315 320
   Ala His Gly Leu Thr Tyr Ser Gly His Pro Val Ala Ala Ala Val Ala
   325 330 335
   Ile Ala Asn Leu Lys Ala Leu Arg Asp Glu Gly Val Val Thr Arg Val
   340 345 350
   Arg Glu Glu Thr Gly Pro Tyr Leu Gln Arg Cys Leu Arg Glu Val Phe
   355 360 365
   Gly Asp His Pro Leu Val Gly Glu Val Gln Gly Ala Gly Phe Val Ala
   370 375 380
   Ala Leu Gln Phe Ala Glu Asp Lys Val Thr Arg Lys Arg Phe Ala Asn
   385 390 395 400
   Glu Asn Asp Leu Ala Trp Arg Cys Arg Thr Ile Gly Phe Glu Glu Gly
   405 410 415
   Val Ile Ile Arg Ser Thr Leu Gly Arg Met Ile Met Ala Pro Ala Leu
   420 425 430
   Val Ala Gly Arg Ala Glu Ile Asp Glu Leu Ile Asp Lys Thr Arg Ile
   435 440 445
   Ala Val Asp Arg Thr Ala Arg Glu Ile Gly Val Leu
   450 455 460
<210> 21
   <211> 448
   <212> PRT
   <213> Pseudomonas aeruginosa PA01
<400> 21
   Met Asn Gln Pro Leu Asn Val Ala Pro Pro Val Ser Ser Glu Leu Asn
   1 5 10 15
   Leu Arg Ala His Trp Met Pro Phe Ser Ala Asn Arg Asn Phe Gln Lys
   20 25 30
   Asp Pro Arg Ile Ile Val Ala Ala Glu Gly Ser Trp Leu Thr Asp Asp
   35 40 45
   Lys Gly Arg Lys Val Tyr Asp Ser Leu Ser Gly Leu Trp Thr Cys Gly
   50 55 60
   Ala Gly His Ser Arg Lys Glu Ile Gln Glu Ala Val Ala Arg Gln Leu
   65 70 75 80
   Gly Thr Leu Asp Tyr Ser Pro Gly Phe Gln Tyr Gly His Pro Leu Ser
   85 90 95
   Phe Gln Leu Ala Glu Lys Ile Ala Gly Leu Leu Pro Gly Glu Leu Asn
   100 105 110
   His Val Phe Phe Thr Gly Ser Gly Ser Glu Cys Ala Asp Thr Ser Ile
   115 120 125
   Lys Met Ala Arg Ala Tyr Trp Arg Leu Lys Gly Gln Pro Gln Lys Thr
   130 135 140
   Lys Leu Ile Gly Arg Ala Arg Gly Tyr His Gly Val Asn Val Ala Gly
   145 150 155 160
   Thr Ser Leu Gly Gly Ile Gly Gly Asn Arg Lys Met Phe Gly Gln Leu
   165 170 175
   Met Asp Val Asp His Leu Pro His Thr Leu Gln Pro Gly Met Ala Phe
   180 185 190
   Thr Arg Gly Met Ala Gln Thr Gly Gly Val Glu Leu Ala Asn Glu Leu
   195 200 205
   Leu Lys Leu Ile Glu Leu His Asp Ala Ser Asn Ile Ala Ala Val Ile
   210 215 220
   Val Glu Pro Met Ser Gly Ser Ala Gly Val Leu Val Pro Pro Val Gly
   225 230 235 240
   Tyr Leu Gln Arg Leu Arg Glu Ile Cys Asp Gln His Asn Ile Leu Leu
   245 250 255
   Ile Phe Asp Glu Val Ile Thr Ala Phe Gly Arg Leu Gly Thr Tyr Ser
   260 265 270
   Gly Ala Glu Tyr Phe Gly Val Thr Pro Asp Leu Met Asn Val Ala Lys
   275 280 285
   Gln Val Thr Asn Gly Ala Val Pro Met Gly Ala Val Ile Ala Ser Ser
   290 295 300
   Glu Ile Tyr Asp Thr Phe Met Asn Gln Ala Leu Pro Glu His Ala Val
   305 310 315 320
   Glu Phe Ser His Gly Tyr Thr Tyr Ser Ala His Pro Val Ala Cys Ala
   325 330 335
   Ala Gly Leu Ala Ala Leu Asp Ile Leu Ala Arg Asp Asn Leu Val Gln
   340 345 350
   Gln Ser Ala Glu Leu Ala Pro His Phe Glu Lys Gly Leu His Gly Leu
   355 360 365
   Gln Gly Ala Lys Asn Val Ile Asp Ile Arg Asn Cys Gly Leu Ala Gly
   370 375 380
   Ala Ile Gln Ile Ala Pro Arg Asp Gly Asp Pro Thr Val Arg Pro Phe
   385 390 395 400
   Glu Ala Gly Met Lys Leu Trp Gln Gln Gly Phe Tyr Val Arg Phe Gly
   405 410 415
   Gly Asp Thr Leu Gln Phe Gly Pro Thr Phe Asn Ala Arg Pro Glu Glu
   420 425 430
   Leu Asp Arg Leu Phe Asp Ala Val Gly Glu Ala Leu Asn Gly Ile Ala
   435 440 445
<210> 22
   <211> 444
   <212> PRT
   <213> Pseudomonas aeruginosa PA01
<400> 22
   Met Thr Met Asn Asp Glu Pro Gln Ser Ser Ser Leu Asp Asn Phe Trp
   1 5 10 15
   Met Pro Phe Thr Ala Asn Arg Gln Phe Lys Ala Arg Pro Arg Leu Leu
   20 25 30
   Glu Ser Ala Glu Gly Ile His Tyr Ile Ala Gln Gly Gly Arg Arg Ile
   35 40 45
   Leu Asp Gly Thr Ala Gly Leu Trp Cys Cys Asn Ala Gly His Gly Arg
   50 55 60
   Arg Glu Ile Ser Glu Ala Val Ala Arg Gln Ile Ala Thr Leu Asp Tyr
   65 70 75 80
   Ala Pro Pro Phe Gln Met Gly His Pro Leu Pro Phe Glu Leu Ala Ala
   85 90 95
   Arg Leu Thr Glu Ile Ala Pro Pro Ser Leu Asn Lys Val Phe Phe Thr
   100 105 110
   Asn Ser Gly Ser Glu Ser Ala Asp Thr Ala Leu Lys Ile Ala Leu Ala
   115 120 125
   Tyr Gln Arg Ala Ile Gly Gln Gly Thr Arg Thr Arg Leu Ile Gly Arg
   130 135 140
   Glu Leu Gly Tyr His Gly Val Gly Phe Gly Gly Leu Ser Val Gly Gly
   145 150 155 160
   Met Val Asn Asn Arg Lys Ala Phe Ser Ala Asn Leu Leu Pro Gly Val
   165 170 175
   Asp His Leu Pro His Thr Leu Asp Val Ala Arg Asn Ala Phe Thr Val
   180 185 190
   Gly Leu Pro Glu His Gly Val Glu Lys Ala Glu Glu Leu Glu Arg Leu
   195 200 205
   Val Thr Leu His Gly Ala Glu Asn Ile Ala Ala Val Ile Val Glu Pro
   210 215 220
   Met Ser Gly Ser Ala Gly Val Val Leu Pro Pro Lys Gly Tyr Leu Gln
   225 230 235 240
   Arg Leu Arg Glu Ile Thr Arg Lys His Gly Ile Leu Leu Ile Phe Asp
   245 250 255
   Glu Val Ile Thr Gly Phe Gly Arg Val Gly Glu Ala Phe Ala Ala Gln
   260 265 270
   Arg Trp Gly Val Val Pro Asp Leu Leu Thr Cys Ala Lys Gly Leu Thr
   275 280 285
   Asn Gly Ser Ile Pro Met Gly Ala Val Phe Val Asp Glu Lys Ile His
   290 295 300
   Ala Ala Phe Met Gln Gly Pro Gln Gly Ala Ile Glu Phe Phe His Gly
   305 310 315 320
   Tyr Thr Tyr Ser Gly His Pro Val Ala Cys Ala Ala Ala Leu Ala Thr
   325 330 335
   Leu Asp Ile Tyr Arg Arg Asp Asp Leu Phe Gln Arg Ala Val Glu Leu
   340 345 350
   Glu Gly Tyr Trp Gln Asp Ala Leu Phe Ser Leu Arg Asp Leu Pro Asn
   355 360 365
   Val Val Asp Ile Arg Ala Val Gly Leu Val Gly Gly Val Gln Leu Ala
   370 375 380
   Pro His Ala Asp Gly Pro Gly Lys Arg Gly Tyr Asp Val Phe Glu Arg
   385 390 395 400
   Cys Phe Trp Glu His Asp Leu Met Val Arg Val Thr Gly Asp Ile Ile
   405 410 415
   Ala Met Ser Pro Pro Leu Ile Ile Asp Lys Pro His Ile Asp Gln Ile
   420 425 430
   Val Glu Arg Leu Ala Gln Ala Ile Arg Ala Ser Val
   435 440
<210> 23
   <211> 468
   <212> PRT
   <213> Pseudomonas aeruginosa PA01
<400> 23
   Met Asn Ala Arg Leu His Ala Thr Ser Pro Leu Gly Asp Ala Asp Leu
   1 5 10 15
   Val Arg Ala Asp Gln Ala His Tyr Met His Gly Tyr His Val Phe Asp
   20 25 30
   Asp His Arg Val Asn Gly Ser Leu Asn Ile Ala Ala Gly Asp Gly Ala
   35 40 45
   Tyr Ile Tyr Asp Thr Ala Gly Asn Arg Tyr Leu Asp Ala Val Gly Gly
   50 55 60
   Met Trp Cys Thr Asn Ile Gly Leu Gly Arg Glu Glu Met Ala Arg Thr
   65 70 75 80
   Val Ala Glu Gln Thr Arg Leu Leu Ala Tyr Ser Asn Pro Phe Cys Asp
   85 90 95
   Met Ala Asn Pro Arg Ala Ile Glu Leu Cys Arg Lys Leu Ala Glu Leu
   100 105 110
   Ala Pro Gly Asp Leu Asp His Val Phe Leu Thr Thr Gly Gly Ser Thr
   115 120 125
   Ala Val Asp Thr Ala Ile Arg Leu Met His Tyr Tyr Gln Asn Cys Arg
   130 135 140
   Gly Lys Arg Ala Lys Lys His Val Ile Thr Arg Ile Asn Ala Tyr His
   145 150 155 160
   Gly Ser Thr Phe Leu Gly Met Ser Leu Gly Gly Lys Ser Ala Asp Arg
   165 170 175
   Pro Ala Glu Phe Asp Phe Leu Asp Glu Arg Ile His His Leu Ala Cys
   180 185 190
   Pro Tyr Tyr Tyr Arg Ala Pro Glu Gly Leu Gly Glu Ala Glu Phe Leu
   195 200 205
   Asp Gly Leu Val Asp Glu Phe Glu Arg Lys Ile Leu Glu Leu Gly Ala
   210 215 220
   Asp Arg Val Gly Ala Phe Ile Ser Glu Pro Val Phe Gly Ser Gly Gly
   225 230 235 240
   Val Ile Val Pro Pro Ala Gly Tyr His Arg Arg Met Trp Glu Leu Cys
   245 250 255
   Gln Arg Tyr Asp Val Leu Tyr Ile Ser Asp Glu Val Val Thr Ser Phe
   260 265 270
   Gly Arg Leu Gly His Phe Phe Ala Ser Gln Ala Val Phe Gly Val Gln
   275 280 285
   Pro Asp Ile Ile Leu Thr Ala Lys Gly Leu Thr Ser Gly Tyr Gln Pro
   290 295 300
   Leu Gly Ala Cys Ile Phe Ser Arg Arg Ile Trp Glu Val Ile Ala Glu
   305 310 315 320
   Pro Asp Lys Gly Arg Cys Phe Ser His Gly Phe Thr Tyr Ser Gly His
   325 330 335
   Pro Val Ala Cys Ala Ala Ala Leu Lys Asn Ile Glu Ile Ile Glu Arg
   340 345 350
   Glu Gly Leu Leu Ala His Ala Asp Glu Val Gly Arg Tyr Phe Glu Glu
   355 360 365
   Arg Leu Gln Ser Leu Arg Asp Leu Pro Ile Val Gly Asp Val Arg Gly
   370 375 380
   Met Arg Phe Met Ala Cys Val Glu Phe Val Ala Asp Lys Ala Ser Lys
   385 390 395 400
   Ala Leu Phe Pro Glu Ser Leu Asn Ile Gly Glu Trp Val His Leu Arg
   405 410 415
   Ala Gln Lys Arg Gly Leu Leu Val Arg Pro Ile Val His Leu Asn Val
   420 425 430
   Met Ser Pro Pro Leu Ile Leu Thr Arg Glu Gln Val Asp Thr Val Val
   435 440 445
   Arg Val Leu Arg Glu Ser Ile Glu Glu Thr Val Glu Asp Leu Val Arg
   450 455 460
   Ala Gly His Arg
   465
<210> 24
   <211> 460
   <212> PRT
   <213> Pseudomonas putida W619
<400> 24
   Met Asn Ala Pro Phe Gln Pro Gln Arg Asp Thr Arg Asp Tyr Gln Ala
   1 5 10 15
   Ser Asp Ala Ala His His Ile His Ala Phe Leu Asp Gln Lys Ala Leu
   20 25 30
   Asn Ala Glu Gly Pro Arg Val Ile Thr Arg Gly Glu Arg Leu Tyr Leu
   35 40 45
   Trp Asp Asn Asp Gly Arg Arg Tyr Leu Asp Gly Met Ser Gly Leu Trp
   50 55 60
   Cys Thr Gln Leu Gly Tyr Gly Arg Lys Asp Leu Thr Ala Ala Ala Ala
   65 70 75 80
   Ala Gln Met Asp Gln Leu Ala Tyr Tyr Asn Met Phe Phe His Thr Thr
   85 90 95
   His Pro Ala Val Ile Glu Leu Ser Glu Leu Leu Phe Ser Leu Leu Pro
   100 105 110
   Gly His Tyr Ser His Ala Ile Tyr Thr Asn Ser Gly Ser Glu Ala Asn
   115 120 125
   Glu Val Leu Ile Arg Thr Val Arg Arg Tyr Trp Gln Val Val Gly Gln
   130 135 140
   Pro Asn Lys Lys Val Met Ile Gly Arg Trp Asn Gly Tyr His Gly Ser
   145 150 155 160
   Thr Leu Ala Ala Thr Ala Leu Gly Gly Met Lys Phe Met His Glu Met
   165 170 175
   Gly Gly Leu Ile Pro Asp Val Ala His Ile Asp Glu Pro Tyr Trp Tyr
   180 185 190
   Ala Glu Gly Gly Glu Leu Thr Pro Ala Glu Phe Gly Arg Arg Cys Ala
   195 200 205
   Leu Gln Leu Glu Glu Lys Ile Leu Glu Leu Gly Ala Glu Asn Val Ala
   210 215 220
   Gly Phe Val Ala Glu Pro Phe Gln Gly Ala Gly Gly Met Ile Phe Pro
   225 230 235 240
   Pro Glu Ser Tyr Trp Pro Glu Ile Gln Arg Ile Cys Arg Gln Tyr Asp
   245 250 255
   Val Leu Leu Cys Ala Asp Glu Val Ile Gly Gly Phe Gly Arg Thr Gly
   260 265 270
   Glu Trp Phe Ala His Glu Tyr Phe Gly Phe Glu Pro Asp Thr Leu Ser
   275 280 285
   Ile Ala Lys Gly Leu Thr Ser Gly Tyr Val Pro Met Gly Gly Leu Val
   290 295 300
   Leu Ser Lys Arg Ile Ala Glu Ala Leu Val Glu Arg Gly Gly Val Phe
   305 310 315 320
   Ala His Gly Leu Thr Tyr Ser Gly His Pro Val Ala Ala Ala Val Ala
   325 330 335
   Ile Ala Asn Leu Lys Ala Leu Arg Asp Glu Gly Ile Val Arg Gln Val
   340 345 350
   Lys Asp Asp Thr Gly Pro Tyr Leu Gln Arg Ile Leu Arg Glu Val Phe
   355 360 365
   Ala Asn His Pro Leu Ile Gly Gln Val Gln Gly Ala Gly Leu Val Ala
   370 375 380
   Ala Leu Gln Phe Ala Glu Asp Lys Gly Ser Arg Lys Arg Tyr Ala Asn
   385 390 395 400
   Glu Asn Asp Leu Ala Trp Gln Cys Arg Thr Tyr Gly Phe Glu Glu Gly
   405 410 415
   Val Ile Ile Arg Ser Thr Leu Gly Arg Met Ile Met Ala Pro Ala Leu
   420 425 430
   Val Ala Thr His Gly Glu Leu Asp Glu Leu Val Asp Lys Thr Arg Ile
   435 440 445
   Ala Val Asp Arg Thr Ala Arg Gly Leu Gly Ile Leu
   450 455 460
<210> 25
   <211> 459
   <212> PRT
   <213> Pseudomonas putida KT2440
<400> 25
   Met Ser Thr His Ser Ser Thr Val Gln Asn Asp Leu Ala Ala Leu Ile
   1 5 10 15
   His Pro Asn Thr Asn Leu Ala Gln His Arg Glu Val Gly Pro Leu Val
   20 25 30
   Ile Ala Arg Gly Asp Gly Val Arg Val Phe Asp Glu Gln Gly Asn Ala
   35 40 45
   Tyr Ile Glu Ala Met Ser Gly Leu Trp Ser Ala Ala Leu Gly Phe Ser
   50 55 60
   Glu Gln Arg Leu Val Asp Ala Ala Val Glu Gln Phe Lys Gln Leu Pro
   65 70 75 80
   Tyr Tyr His Ser Phe Ser His Lys Thr Asn Ala Pro Ala Ala Ala Leu
   85 90 95
   Ala Ala Lys Leu Ala Ala Leu Ala Pro Gly Asp Leu Asn His Val Phe
   100 105 110
   Phe Thr Asn Ser Gly Ser Glu Ala Asn Asp Ser Val Val Lys Met Val
   115 120 125
   Trp Tyr Val Asn Asn Ala Leu Gly Arg Pro Ala Lys Lys Lys Phe Ile
   130 135 140
   Ser Arg Gln Gln Ala Tyr His Gly Ala Thr Val Ala Ala Ala Ser Leu
   145 150 155 160
   Thr Gly Ile Pro Ser Met His Arg Asp Phe Asp Leu Pro Ala Ile Pro
   165 170 175
   Val His His Leu Thr Cys Pro Asn Phe Tyr Arg Phe Ala Arg Pro Gly
   180 185 190
   Glu Ser Gln Glu Ala Phe Thr Val Arg Leu Ala Asn Glu Leu Glu Arg
   195 200 205
   Tyr Ile Leu Ala Glu Gly Pro Glu Thr Ile Ala Ala Phe Ile Gly Glu
   210 215 220
   Pro Val Ile Ala Ala Gly Gly Val Ile Pro Pro Pro Thr Gly Tyr Trp
   225 230 235 240
   Ala Ala Ile Gln Ala Val Cys Lys Arg Tyr Asp Ile Leu Val Val Ile
   245 250 255
   Asp Glu Ile Ile Thr Gly Phe Gly Arg Leu Gly Thr Met Phe Gly Ser
   260 265 270
   Gln Leu Tyr Gly Ile Gln Pro Asp Ile Met Val Leu Ser Lys Gln Leu
   275 280 285
   Thr Ser Ser Tyr Gln Pro Leu Ala Ala Val Val Val Ser Asp Ala Met
   290 295 300
   Asn Asp Val Leu Val Ser Gln Ser Gln Arg Leu Gly Ala Phe Ala His
   305 310 315 320
   Gly Leu Thr Cys Thr Gly His Pro Val Ala Thr Ala Val Ala Leu Glu
   325 330 335
   Asn Ile Arg Ile Ile Glu Glu Arg Asp Leu Val Gly His Val Gln His
   340 345 350
   Leu Ala Pro Val Phe Gln Arg His Leu Arg Ala Phe Glu Asp His Pro
   355 360 365
   Leu Val Gly Asn Val Arg Gly Val Gly Leu Met Gly Gly Ile Glu Leu
   370 375 380
   Val Ala Asp Lys Ala Thr Arg Gln Pro Phe Ala Gln Pro Gly Thr Leu
   385 390 395 400
   Gly Gly Tyr Val Phe Lys Gln Ala His Lys His Gly Leu Ile Ile Arg
   405 410 415
   Ala Ile Tyr Asp Thr Ile Ala Phe Cys Pro Pro Leu Ile Thr Thr Gln
   420 425 430
   Asp Asp Ile Glu Ala Ile Phe Ser Ala Phe Glu Arg Thr Leu Ala Asp
   435 440 445
   Ala Thr Asp Trp Ala Arg Ser Gln His Leu Leu
   450 455
<210> 26
   <211> 490
   <212> PRT
   <213> Pseudomonas putida W619
<400> 26
   Met Arg Ile Ser Ser Val Ser Ser Ala Pro Gly Ser Val Ser Ser Cys
   1 5 10 15
   Cys Leu Leu Cys Asp Gln Pro Leu Arg Pro Pro Ala Gly Gly Leu Trp
   20 25 30
   Thr Leu Glu Lys His Met Ser Val Lys Asn Pro Gln Thr Arg Asp Trp
   35 40 45
   Gln Thr Leu Ser Gly Glu His His Leu Ala Pro Phe Ser Asp Tyr Lys
   50 55 60
   Gln Leu Lys Glu Lys Gly Pro Arg Ile Ile Thr Lys Ala Gln Gly Val
   65 70 75 80
   His Leu Trp Asp Ser Glu Gly His Lys Ile Leu Asp Gly Met Ala Gly
   85 90 95
   Leu Trp Cys Val Ala Val Gly Tyr Gly Arg Glu Glu Leu Val Gln Ala
   100 105 110
   Ala Glu Lys Gln Met Arg Glu Leu Pro Tyr Tyr Asn Leu Phe Phe Gln
   115 120 125
   Thr Ala His Pro Pro Ala Leu Glu Leu Ala Lys Ala Ile Thr Asp Val
   130 135 140
   Ala Pro Glu Gly Met Thr His Val Phe Phe Thr Gly Ser Gly Ser Glu
   145 150 155 160
   Gly Asn Asp Thr Val Leu Arg Met Val Arg His Tyr Trp Ala Leu Lys
   165 170 175
   Gly Lys Pro Gln Lys Gln Thr Ile Ile Gly Arg Ile Asn Gly Tyr His
   180 185 190
   Gly Ser Thr Val Ala Gly Ala Ser Leu Gly Gly Met Ser Gly Met His
   195 200 205
   Glu Gln Gly Gly Leu Pro Ile Pro Gly Ile Val His Ile Pro Gln Pro
   210 215 220
   Tyr Trp Phe Gly Glu Gly Gly Asp Met Ser Pro Asp Asp Phe Gly Val
   225 230 235 240
   Trp Ala Ala Glu Gln Leu Glu Lys Lys Ile Leu Glu Val Gly Glu Asp
   245 250 255
   Asn Val Ala Ala Phe Ile Ala Glu Pro Ile Gln Gly Ala Gly Gly Val
   260 265 270
   Ile Ile Pro Pro Glu Thr Tyr Trp Pro Lys Val Lys Glu Ile Leu Ala
   275 280 285
   Lys Tyr Asp Ile Leu Phe Val Ala Asp Glu Val Ile Cys Gly Phe Gly
   290 295 300
   Arg Thr Gly Glu Trp Phe Gly Ser Asp Tyr Tyr Asp Leu Lys Pro Asp
   305 310 315 320
   Leu Met Thr Ile Ala Lys Gly Leu Thr Ser Gly Tyr Ile Pro Met Gly
   325 330 335
   Gly Val Ile Val Arg Asp Lys Val Ala Lys Val Leu Ser Glu Gly Gly
   340 345 350
   Asp Phe Asn His Gly Phe Thr Tyr Ser Gly His Pro Val Ala Ala Ala
   355 360 365
   Val Gly Leu Glu Asn Leu Arg Ile Leu Arg Glu Glu Lys Ile Val Glu
   370 375 380
   Lys Val Arg Thr Glu Val Ala Pro Tyr Leu Gln Lys Arg Leu Arg Glu
   385 390 395 400
   Leu Gln Asp His Pro Leu Val Gly Glu Val Arg Gly Leu Gly Leu Leu
   405 410 415
   Gly Ala Ile Glu Leu Val Lys Asp Lys Ala Ser Arg Ser Arg Tyr Glu
   420 425 430
   Gly Lys Gly Val Gly Met Val Cys Arg Asn Phe Cys Phe Asp Asn Gly
   435 440 445
   Leu Ile Met Arg Ala Val Gly Asp Thr Met Ile Ile Ala Pro Pro Leu
   450 455 460
   Val Ile Ser His Ala Glu Val Asp Glu Leu Val Glu Lys Ala Arg Lys
   465 470 475 480
   Cys Leu Asp Leu Thr Leu Glu Ala Ile Arg
   485 490
<210> 27
   <211> 457
   <212> PRT
   <213> Streptomyces coelicolor A3(2)
<400> 27
   Met Ser Thr Asp Ser Pro Lys Asp Leu Ser Arg Thr Ala Tyr Asp His
   1 5 10 15
   Leu Trp Met His Phe Thr Arg Met Ser Ser Tyr Glu Asn Ala Pro Val
   20 25 30
   Pro Thr Ile Val Arg Gly Glu Gly Thr His Ile Tyr Asp Asp Lys Gly
   35 40 45
   Arg Arg Tyr Leu Asp Gly Leu Ala Gly Leu Phe Val Val Gln Ala Gly
   50 55 60
   His Gly Arg Gln Glu Leu Ala Glu Thr Ala Ser Lys Gln Ala Gln Glu
   65 70 75 80
   Leu Ala Phe Phe Pro Val Trp Ser Tyr Ala His Pro Lys Ala Val Glu
   85 90 95
   Leu Ala Glu Arg Leu Ala Asn Glu Ala Pro Gly Asp Leu Asn Lys Val
   100 105 110
   Phe Phe Thr Thr Gly Gly Gly Glu Ala Val Glu Thr Ala Trp Lys Leu
   115 120 125
   Ala Lys Gln Tyr Phe Lys Leu Thr Gly Lys Pro Thr Lys Tyr Lys Val
   130 135 140
   Ile Ser Arg Ala Val Ala Tyr His Gly Thr Pro Gln Gly Ala Leu Ser
   145 150 155 160
   Ile Thr Gly Leu Pro Ala Leu Lys Ala Pro Phe Glu Pro Leu Val Pro
   165 170 175
   Gly Ala His Lys Val Pro Asn Thr Asn Ile Tyr Arg Ala Pro Ile His
   180 185 190
   Gly Asp Asp Pro Glu Ala Tyr Gly Arg Trp Ala Ala Asp Gln Ile Glu
   195 200 205
   Gln Gln Ile Leu Phe Glu Gly Pro Glu Thr Val Ala Ala Val Phe Leu
   210 215 220
   Glu Pro Val Gln Asn Ala Gly Gly Cys Phe Pro Pro Pro Pro Gly Tyr
   225 230 235 240
   Phe Gln Arg Val Arg Glu Ile Cys Asp Gln Tyr Asp Val Leu Leu Val
   245 250 255
   Ser Asp Glu Val Ile Cys Ala Phe Gly Arg Leu Gly Thr Thr Phe Ala
   260 265 270
   Cys Asp Lys Phe Gly Tyr Val Pro Asp Met Ile Thr Cys Ala Lys Gly
   275 280 285
   Met Thr Ser Gly Tyr Ser Pro Ile Gly Ala Cys Val Ile Ser Asp Arg
   290 295 300
   Leu Ala Glu Pro Phe Tyr Lys Gly Asp Asn Thr Phe Leu His Gly Tyr
   305 310 315 320
   Thr Phe Gly Gly His Pro Val Ser Ala Ala Val Gly Ile Ala Asn Leu
   325 330 335
   Asp Leu Phe Glu Arg Glu Gly Leu Asn Gln His Val Leu Asp Asn Glu
   340 345 350
   Gly Ala Phe Arg Ala Thr Leu Glu Lys Leu His Asp Leu Pro Ile Val
   355 360 365
   Gly Asp Val Arg Gly Asn Gly Phe Phe Tyr Gly Ile Glu Leu Val Lys
   370 375 380
   Asp Lys Ala Thr Lys Glu Ser Phe Asp Glu Glu Glu Thr Glu Arg Val
   385 390 395 400
   Leu Tyr Gly Phe Leu Ser Lys Lys Leu Phe Glu Asn Gly Leu Tyr Cys
   405 410 415
   Arg Ala Asp Asp Arg Gly Asp Pro Val Ile Gln Leu Ala Pro Pro Leu
   420 425 430
   Ile Ser Asn Gln Glu Thr Phe Asp Glu Ile Glu Gln Ile Leu Arg Ala
   435 440 445
   Thr Leu Thr Glu Ala Trp Thr Lys Leu
   450 455
<210> 28
   <211> 459
   <212> PRT
   <213> Streptomyces avermitilis MA 4680
<400> 28
   Met Gly Asn Pro Ile Ala Val Ser Lys Asp Leu Ser Arg Thr Ala Tyr
   1 5 10 15
   Asp His Leu Trp Met His Phe Thr Arg Met Ser Ser Tyr Glu Asn Ala
   20 25 30
   Pro Val Pro Thr Ile Val Arg Gly Glu Gly Thr Tyr Ile Tyr Asp Asp
   35 40 45
   Lys Gly Lys Arg Tyr Leu Asp Gly Leu Ser Gly Leu Phe Val Val Gln
   50 55 60
   Ala Gly His Gly Arg Thr Glu Leu Ala Glu Thr Ala Phe Lys Gln Ala
   65 70 75 80
   Gln Glu Leu Ala Phe Phe Pro Val Trp Ser Tyr Ala His Pro Lys Ala
   85 90 95
   Val Glu Leu Ala Glu Arg Leu Ala Asn Tyr Ala Pro Gly Asp Leu Asn
   100 105 110
   Lys Val Phe Phe Thr Thr Gly Gly Gly Glu Ala Val Glu Thr Ala Trp
   115 120 125
   Lys Leu Ala Lys Gln Tyr Phe Lys Leu Gln Gly Lys Pro Thr Lys Tyr
   130 135 140
   Lys Val Ile Ser Arg Ala Val Ala Tyr His Gly Thr Pro Gln Gly Ala
   145 150 155 160
   Leu Ser Ile Thr Gly Leu Pro Ala Leu Lys Ala Pro Phe Glu Pro Leu
   165 170 175
   Val Pro Gly Ala His Lys Val Pro Asn Thr Asn Ile Tyr Arg Ala Pro
   180 185 190
   Leu Phe Gly Asp Asp Pro Glu Ala Phe Gly Arg Trp Ala Ala Asp Gln
   195 200 205
   Ile Glu Gln Gln Ile Leu Phe Glu Gly Pro Glu Thr Val Ala Ala Val
   210 215 220
   Phe Leu Glu Pro Val Gln Asn Ala Gly Gly Cys Phe Pro Pro Pro Pro
   225 230 235 240
   Gly Tyr Phe Gln Arg Val Arg Glu Ile Cys Asp Gln Tyr Asp Val Leu
   245 250 255
   Leu Val Ser Asp Glu Val Ile Cys Ala Phe Gly Arg Leu Gly Thr Met
   260 265 270
   Phe Ala Cys Asp Lys Phe Gly Tyr Val Pro Asp Met Ile Thr Cys Ala
   275 280 285
   Lys Gly Met Thr Ser Gly Tyr Ser Pro Ile Gly Ala Cys Ile Val Ser
   290 295 300
   Asp Arg Ile Ala Glu Pro Phe Tyr Lys Gly Asp Asn Thr Phe Leu His
   305 310 315 320
   Gly Tyr Thr Phe Gly Gly His Pro Val Ser Ala Ala Val Gly Val Ala
   325 330 335
   Asn Leu Asp Leu Phe Glu Arg Glu Gly Leu Asn Gln His Val Leu Asp
   340 345 350
   Asn Glu Ser Ala Phe Leu Thr Thr Leu Gln Lys Leu His Asp Leu Pro
   355 360 365
   Ile Val Gly Asp Val Arg Gly Asn Gly Phe Phe Tyr Gly Ile Glu Leu
   370 375 380
   Val Lys Asp Lys Ala Thr Lys Glu Thr Phe Thr Asp Glu Glu Ser Glu
   385 390 395 400
   Arg Val Leu Tyr Gly Phe Val Ser Lys Lys Leu Phe Glu Tyr Gly Leu
   405 410 415
   Tyr Cys Arg Ala Asp Asp Arg Gly Asp Pro Val Ile Gln Leu Ser Pro
   420 425 430
   Pro Leu Ile Ser Asn Gln Ser Thr Phe Asp Glu Ile Glu Ser Ile Ile
   435 440 445
   Arg Gln Val Leu Thr Glu Ala Trp Thr Lys Leu
   450 455
<210> 29
   <211> 450
   <212> PRT
   <213> Streptomyces avermitilis MA 4680
<400> 29
   Met Thr Pro Gln Pro Asn Pro Gln Val Gly Ala Ala Val Lys Ala Ala
   1 5 10 15
   Asp Arg Ala His Val Phe His Ser Trp Ser Ala Gln Glu Leu Ile Asp
   20 25 30
   Pro Leu Ala Val Ala Gly Ala Glu Gly Ser Tyr Phe Trp Asp Tyr Asp
   35 40 45
   Gly Arg Arg Tyr Leu Asp Phe Thr Ser Gly Leu Val Phe Thr Asn Ile
   50 55 60
   Gly Tyr Gln His Pro Lys Val Val Ala Ala Ile Gln Glu Gln Ala Ala
   65 70 75 80
   Ser Leu Thr Thr Phe Ala Pro Ala Phe Ala Val Glu Ala Arg Ser Glu
   85 90 95
   Ala Ala Arg Leu Ile Ala Glu Arg Thr Pro Gly Asp Leu Asp Lys Ile
   100 105 110
   Phe Phe Thr Asn Gly Gly Ala Asp Ala Ile Glu His Ala Val Arg Met
   115 120 125
   Ala Arg Ile His Thr Gly Arg Pro Lys Val Leu Ser Ala Tyr Arg Ser
   130 135 140
   Tyr His Gly Gly Thr Gln Gln Ala Val Asn Ile Thr Gly Asp Pro Arg
   145 150 155 160
   Arg Trp Ala Ser Asp Ser Ala Ser Ala Gly Val Val His Phe Trp Ala
   165 170 175
   Pro Tyr Leu Tyr Arg Ser Arg Phe Tyr Ala Glu Thr Glu Gln Gln Glu
   180 185 190
   Cys Glu Arg Ala Leu Glu His Leu Glu Thr Thr Ile Ala Phe Glu Gly
   195 200 205
   Pro Gly Thr Ile Ala Ala Ile Val Leu Glu Thr Val Pro Gly Thr Ala
   210 215 220
   Gly Ile Met Val Pro Pro Pro Gly Tyr Leu Ala Gly Val Arg Glu Leu
   225 230 235 240
   Cys Asp Lys Tyr Gly Ile Val Phe Val Leu Asp Glu Val Met Ala Gly
   245 250 255
   Phe Gly Arg Thr Gly Glu Trp Phe Ala Ala Asp Leu Phe Asp Val Thr
   260 265 270
   Pro Asp Leu Met Thr Phe Ala Lys Gly Val Asn Ser Gly Tyr Val Pro
   275 280 285
   Leu Gly Gly Val Ala Ile Ser Gly Lys Ile Ala Glu Thr Phe Gly Lys
   290 295 300
   Arg Ala Tyr Pro Gly Gly Leu Thr Tyr Ser Gly His Pro Leu Ala Cys
   305 310 315 320
   Ala Ala Ala Val Ala Thr Ile Asn Val Met Ala Glu Glu Gly Val Val
   325 330 335
   Glu Asn Ala Ala Asn Leu Gly Ala Arg Val Ile Glu Pro Gly Leu Arg
   340 345 350
   Glu Leu Ala Glu Arg His Pro Ser Val Gly Glu Val Arg Gly Val Gly
   355 360 365
   Met Phe Trp Ala Leu Glu Leu Val Lys Asp Arg Glu Thr Arg Glu Pro
   370 375 380
   Leu Val Pro Tyr Asn Ala Ala Gly Glu Ala Asn Ala Pro Met Ala Ala
   385 390 395 400
   Phe Gly Ala Ala Ala Lys Ala Asn Gly Leu Trp Pro Phe Ile Asn Met
   405 410 415
   Asn Arg Thr His Val Val Pro Pro Cys Asn Val Thr Glu Ala Glu Ala
   420 425 430
   Lys Glu Gly Leu Ala Ala Leu Asp Ala Ala Leu Ser Val Ala Asp Glu
   435 440 445
   Tyr Thr
   450
<210> 30
   <211> 446
   <212> PRT
   <213> Chromobacterium violaceum ATCC 12472
<400> 30
   Met Asn His Pro Leu Thr Thr Arg Ser Glu Phe Asp His Leu Asp Leu
   1 5 10 15
   Arg Ala His Trp Met Pro Phe Ser Ala Asn Arg Asn Phe Gln Arg Asp
   20 25 30
   Pro Arg Leu Ile Val Ser Gly Glu Gly Asn Tyr Leu Thr Asp Ala Asp
   35 40 45
   Gly Arg Arg Ile Phe Asp Ser Leu Ser Gly Leu Trp Cys Cys Gly Ala
   50 55 60
   Gly His Ser Arg Lys Glu Ile Ala Glu Ala Ala Tyr Arg Gln Leu Ser
   65 70 75 80
   Thr Leu Asp Tyr Ser Pro Gly Phe Gln Phe Gly His Pro Leu Ser Phe
   85 90 95
   Arg Leu Ala Glu Arg Val Ala Ala Met Ala Pro Gly Ala Leu Asn His
   100 105 110
   Val Phe Phe Thr Asn Ser Gly Ser Glu Cys Ala Asp Thr Ala Val Lys
   115 120 125
   Met Ala Arg Ala Tyr Trp Arg Leu Lys Gly Gln Ala Ser Lys Thr Lys
   130 135 140
   Leu Ile Gly Arg Ala Arg Gly Tyr His Gly Val Asn Ile Ala Gly Thr
   145 150 155 160
   Ser Leu Gly Gly Met Asn Gly Asn Arg Lys Leu Phe Gly Pro Leu Met
   165 170 175
   Asp Ala Asp His Leu Pro His Thr Leu Leu Pro Ala Asn Ala Phe Ser
   180 185 190
   Arg Gly Leu Pro Glu Gln Gly Ala Glu Leu Ala Asp Asp Leu Leu Arg
   195 200 205
   Leu Ile Glu Leu His Asp Ala Ser Asn Ile Ala Ala Val Ile Val Glu
   210 215 220
   Pro Met Ala Gly Ser Ala Gly Val Ile Val Pro Pro Gln Gly Tyr Leu
   225 230 235 240
   Gln Arg Leu Arg Glu Ile Cys Thr Gln His Gly Ile Leu Leu Ile Phe
   245 250 255
   Asp Glu Val Ile Thr Gly Phe Gly Arg Thr Gly Ser Leu Phe Gly Ala
   260 265 270
   Asp His Phe Gly Val Thr Pro Asp Ile Met Asn Leu Ala Lys Gln Leu
   275 280 285
   Thr Asn Gly Ala Val Pro Met Gly Ala Val Val Ala Ser Ser Glu Ile
   290 295 300
   Tyr Asp Ala Phe Met Ala Gln Ala Thr Pro Glu Tyr Ala Val Glu Phe
   305 310 315 320
   Ala His Gly Tyr Thr Tyr Ser Ala His Pro Val Ala Cys Ala Ala Ala
   325 330 335
   Leu Ala Ala Leu Asp Val Leu Glu Gln Glu Asn Leu Val Ala Arg Ala
   340 345 350
   Ala Glu Leu Ala Pro His Phe Glu Arg Gly Ile His Gly Leu Lys Gly
   355 360 365
   Leu Pro His Val Ile Asp Ile Arg Asn Cys Gly Leu Ala Gly Ala Val
   370 375 380
   Gln Ile Ala Pro Ser Gly Gly Asp Ala Ile Val Arg Pro Tyr Glu Ala
   385 390 395 400
   Ala Met Ala Leu Trp Arg Lys Gly Phe Tyr Val Arg Tyr Gly Gly Asp
   405 410 415
   Ala Leu Gln Phe Gly Pro Pro Phe Thr Ala Thr Pro Gln Glu Leu Asp
   420 425 430
   Ser Leu Phe Asp Ala Val Gly Glu Thr Leu Ala Lys Leu Ala
   435 440 445
<210> 31
   <211> 11539
   <212> DNA
   <213> Artificial
<220>
   <223> Vector
<400> 31
   gaaaaccgcc actgcgccgt taccaccgct gcgttcggtc aaggttctgg accagttgcg 60
   tgagcgcata cgctacttgc attacagttt acgaaccgaa caggcttatg tcaattcgcc 120
   tctcaggcgc cgctggtgcc gctggttgga cgccaagggt gaatccgcct cgataccctg 180
   attactcgct tcctgcgccc tctcaggcgg cgatagggga ctggtaaaac ggggattgcc 240
   cagacgcctc ccccgcccct tcaggggcac aaatgcggcc ccaacggggc cacgtagtgg 300
   tgcgtttttt gcgtttccac ccttttcttc cttttccctt ttaaaccttt taggacgtct 360
   acaggccacg taatccgtgg cctgtagagt ttaaaaaggg acggatttgt tgccattaag 420
   ggacggattt gttgttaaga agggacggat ttgttgttgt aaagggacgg atttgttgta 480
   ttgtgggacg cagatacagt gtccccttat acacaaggaa tgtcgaacgt ggcctcaccc 540
   ccaatggttt acaaaagcaa tgccctggtc gaggccgcgt atcgcctcag tgttcaggaa 600
   cagcggatcg ttctggcctg tattagccag gtgaagagga gcgagcctgt caccgatgaa 660
   gtgatgtatt cagtgacggc ggaggacata gcgacgatgg cgggtgtccc tatcgaatct 720
   tcctacaacc agctcaaaga agcggccctg cgcctgaaac ggcgggaagt ccggttaacc 780
   caagagccca atggcaaggg gaaaagaccg agtgtgatga ttaccggctg ggtgcaaaca 840
   atcatctacc gggagggtga gggccgtgta gaactcaggt tcaccaaaga catgctgccg 900
   tacctgacgg aactcaccaa acagttcacc aaatacgcct tggctgacgt ggccaagatg 960
   gacagcaccc acgcgatcag gctttacgag ctgctcatgc aatgggacag catcggccag 1020
   cgcgaaatag aaattgacca gctgcgaaag tggtttcaac tggaaggccg gtatccctcg 1080
   atcaaggact tcaagttgcg agtgcttgat ccagccgtga cgcagatcaa cgagcacagc 1140
   ccgctacagg tggagtgggc gcagcgaaag accgggcgca aggtcacaca tctgttgttc 1200
   agttttggac cgaagaagcc cgccaaggcg gtgggtaagg ccccagcgaa gcgcaaggcc 1260
   gggaagattt cagatgctga gatcgcgaaa caggctcgcc ctggtgagac atgggaagcg 1320
   gcccgcgctc gactaaccca gatgccgctg gatctggcct agaggccgtg gccaccacgg 1380
   cccggcctgc ctttcaggct gcattattga agcatttatc agggttattg tctcatgagc 1440
   ggatacatat ttgaatgtat ttagaaaaat aaacaaaaga gtttgtagaa acgcaaaaag 1500
   gccatccgtc aggatggcct tctgcttaat ttgatgcctg gcagtttatg gcgggcgtcc 1560
   tgcccgccac cctccgggcc gttgcttcgc aacgttcaaa tccgctcccg gcggatttgt 1620
   cctactcagg agagcgttca ccgacaaaca acagataaaa cgaaaggccc agtctttcga 1680
   ctgagccttt cgttttattt gatgcctggc agttccctac tctcgcatgg ggagacccca 1740
   cactaccatc ggcgctacgg cgtttcactt ctgagttcgg catggggtca ggtgggacca 1800
   ccgcgctact gccgccaggc aaattctgtt ttatcagacc gcttctgcgt tctgatttaa 1860
   tctgtatcag gctgaaaatc ttctctcatc cgccaaaaca gaagcttggc tgcaggtcga 1920
   caagcgctga atgggtatcg gcactagagt ttaacttggc taggctaatt ggtatggtca 1980
   tttttatttt gtcctgaatt acctagaatg acttgaagtt ttaatcttca cttttcctcg 2040
   tagagcacat agtcttcttt cgtagccccg caatccggac agcaccagtc atcaggaata 2100
   tcctcaaagc gagtacctgg agtaaaaccc tcggcctcat cgcccaacgc ctcatcatat 2160
   atatggccac aagtaataca tatccacttc aagtatgctt tcccgccttg ggcatcagac 2220
   cttggaggtt gagttttata tagatcttgg cctttaacgt cggcactagg caatttctct 2280
   aagcttgtcg gcgcaaccac tgcttcagca gttaaacttg tgccactaac ctcggataaa 2340
   tttggcgaag tatgggttga ggtgacgccc ttttcaccta cgccgctctc aattaacatg 2400
   aagtcaagct tgtctcgaac ggcgcaatcg gggcagcacc aatcctcagg aataaggtgc 2460
   caaggcgtac ctggagaaaa cccctcatgc acattacccg cactctcatc ataaacataa 2520
   ttacaatccg ggcatttata gctagccata ctcatcacca tgtcaaattg ttattttgcg 2580
   ttgcggcaaa tttgcgttat ttatttctca tcttcttcct tgtggaagat tttcttacaa 2640
   cctgtggggt gctcgcctga tcaagaactt cagtcttagc cgcacagccc tcatttttat 2700
   tttcttgatt aagtgggatc actaattctg cggccaaaac tgccattcca gttggagtag 2760
   catctgaaat accactttcc gagttggcaa gctgactatt cacccctagc tgtgtttctt 2820
   tcgagggaga atccgcgaga aagataaagt ccaccttgtc tcgaactgcg cagtccgggc 2880
   atgcccaatc tttggggata tcattccagc tggtgttcgg gtggaaacct tcgtgcggct   2940
   cccccttatt ttcatcatag atatactgac aatctggaca ctggtacctt gacattctcc   3000
   cactctccta ttaactcagc gttagtcgct gctccgacaa cttcatcaga gtgtaactat 3060
   cggagcaggt cgccttcaaa gcaattacag agaggcaatc aaccctcagc actttagcga 3120
   agtgcactct ttgtgagagc tttcaacgcc gcacataaaa ttgaagcact tattgtaaat 3180
   atcgaccgcc gggctctgcg tgctcacata actacgatgc taccgcagag gtactcgaac 3240
   tatgaccagc actactagtg ccaaattttt tcaaataggt ttctcgcatc gaatcatcaa 3300
   tttggatctt attaaggtca ccaccagccc aatctactac cttgggatcc ataactgatc 3360
   taaaccactg aggaatcatc gccatcaaaa atgcaccagg gtaacccgtc ggaagagccg 3420
   gcaggccggg aaaatcccga agtgactgat aagaacgtgt tggatgcgcg tggtgatccg 3480
   agtgccgctg aaggtggaac agcactagat tagagacgat gtgattacta ttccaagaat 3540
   ggtgcggctt ttgatgctca tatcgaccgt cctccatttt ttgacggagc aagccgtaat 3600
   gttcaatata gttcgcactg gtcagctgcc accaaccgaa agccatttga atcggcagga 3660
   acaccagcat cttaggtcca aacaaggcaa ggagaacggc gtaaagaata actgtgatga 3720
   tcattggttg gaggatttca ttatcgaaac tccaaacgct ttggccacgg cgcgaaaggc 3780
   gttgttcctc aagcccccaa gcacgaataa atgctcctgg gatctcacgg attgaaaact 3840
   tataaatgct ttctcccatc cgggatgttg caggatccat cggtgtagcg acatcacggt 3900
   gatgaccctt attatgctca ataaagaagt gaccgtaccc tacgacagcc aacacaattt 3960
   tggccatcca acgatcaaaa gtctccttct tgtgaccgag ttcgtgtcct gtattgagcg 4020
   ctagtccgtt cacgataccc agtgacaagg caagcgcacc aatttcaagc caagacattg 4080
   gctgagttcc gacccaccat gctgacacaa ttaatgcagc gtaatgcata ggaactgtta 4140
   gatatgtcaa aactcgatag taccgctcct tctctagttt cggcaccact tcttcaggcg 4200
   gattattaaa gtcctcacca aacatcgcat caagcaatgg aagtgcgccg taccatacga 4260
   gcaataccag cccataaaaa atcccccaac cagtttcatt tgcaagccag attccgatca 4320
   tcggagtagc cggccacaaa gttgatagta tccagagata tttcttttta tctacgtact 4380
   ctggagcgga atccagaact ctgtgtttct caagcatatg gaattctcca atttttatta 4440
   aattagtcgc tacgagattt aagacgtaat tttatgccta actgagaaag ttaagccgcc 4500
   cactctcact ctcgacatct taaacctgag ctaatcggac gcttgcgcca actacaccta 4560
   cgggtagttt ttgctccgtc gtctgctgga aaaacacgag ctggccgcaa gcatgccagg 4620
   taccgcgagc tactcgcgac ggctgaaagc accgaaatga gcgagctatc tggtcgattt 4680
   tgacccggtg cccgtcttca aaatcggcga aggccgaagt cggccagaaa tagcggccta 4740
   cttcagacct tccctagtaa atattttgca ccaccgatca tgccgactac acttaagtgt 4800
   agttttaata tttaacaccg taacctatgg tgaaaatttc cagtcagctg gcgcgagaat 4860
   agcataatga aaataataat aaataatgat ttcccggtcg ctaaggtcgg agcggatcaa 4920
   attacgactc tagtaagtgc caaagttcat agttgcatat atcggccaag attgagtatc 4980
   gcggatggag ccgctcccag agtatgcctt tacagagccc cacctggata tgggaaaacc 5040
   gttgctcttg cgttcgagtg gctacgccac agaacagccg gacgtcctgc agtgtggctt 5100
   tctttaagag ccagttctta cagtgaattt gatatctgcg cagagattat tgagcagctt 5160
   gaaactttcg aaatggtaaa attcagccgt gtgagagagg gtgtgagcaa gcctgcgctc 5220
   ttgcgagacc ttgcatctag tctttggcag agcacctcga ataacgagat agaaacgcta 5280
   gtttgtttgg ataatattaa tcatgactta gacttgccgt tgttgcacgc acttatggag 5340
   tttatgttaa atacaccaaa aaatatcagg tttgcagttg caggcaatac aataaaaggg 5400
   ttctcgcagc ttaaacttgc aggcgctatg cgggagtaca ccgagaaaga cttggccttt 5460
   agcgcagaag aggcggtggc gttagcggag gcagagtctg ttcttggagt tcctgaagaa 5520
   cagatagaga ccttggtgca agaagttgag gggtggcctg ctcttgtagt ttttttgtta 5580
   aagcgtgagt tgccggccaa gcatatttca gcagtagttg aagtagacaa ttactttagg 5640
   gatgaaatat ttgaggcgat tcccgagcgc tatcgtgttt ttcttgcaaa ttcttcattg 5700
   ctcgatttcg tgacgcctga tcaatacaat tatgtattca aatgcgtcaa tggggtctca 5760
   tgtattaagt atttaagcac taattacatg ttgcttcgcc atgtgagcgg tgagccagcg 5820
   cagtttacac tgcatccagt actgcgtaat tttctacgag aaattacttg gactgaaaat 5880
   cctgctaaaa gatcctacct gcttaagcgt gcagctttct ggcattggcg tagaggtgaa 5940
   taccagtatg caatacgaat atccctacgg gcgaatgact gtcgctgggc agtcagcatg 6000
   tctgagagaa taattttaga tttgtcattt cgtcagggcg aaatagatgc gctgagacag 6060
   tggctgttag agctgccgaa gcaggcctgg caccaaaaac ccatagtgct tattagttac 6120
   gcgtgggtat tgtatttcag tcagcaaggc gcgcgagcag agaagttaat taaagaccta 6180
   tcttcacaat ccgataaaaa aaataaatgg caagaaaagg aatggctgca gcttgtgctt 6240
   gcaataggta aagcaaccaa agatgaaatg ctttcgagtg aggagctctg taataagtgg 6300
   attagtttat ttggggattc aaacgcagtt ggaaaagggg ccgcgctaac ctgtttggct 6360
   tttatttttg ccagtgagta tagatttgca gagttggaga aggtgctggc tcaggcccaa 6420
   gccgtgaata aatttgcaaa acaaaatttt gcttttggtt ggctgtatgt cgcgaggttt 6480
   caacaagccc tagcaagcgg aaaaatgggc tgggcgaggc agattataac tcaagcacgc 6540
   acagacagtc gcgcgcagat gatggaatcc gagtttactt cgaaaatgtt tgacgctcta 6600
   gagcttgagt tacattatga attgcgctgc ttggacacct cagaagaaaa gctctccaaa 6660
   attttagagt tcatttccaa tcacggggtg acagacgtgt ttttttccgt atgccgtgct 6720
   gtgtcagctt ggcggcttgg aaggagtgac ctaaatggct ccattgagat attggagtgg 6780
   gcgaaggcgc atgcggttga aaaaaatcta ccaagattgg aagttatgag ccaaattgag 6840
   atctatcagc gcttagtctg tcaaggcata acgggcataa ataatttaaa aactcttgaa 6900
   gatcataaga ttttctccgg acagcactca gcccccctaa aagcacgcct gctgcttgtt 6960
   caatcactag tgctttcccg agatcggaac tttcatagtg ccgcgcacag agcgttattg 7020
   gctattcagc aagcccgtaa aattaacgcg ggccagctgg aagtccgtgg attattgtgt 7080
   ttggccggag cgcaggcagg tgccggtgat ttaaaaaagg ctcagcttaa cattgtttat 7140
   gcagtggaga tagcaaaaca gcttcaatgc tttcaaacag ttcttgatga agtatgttta 7200
   attgagcgaa taataccggc ttcatgtgaa gccttcacag cagttaattt agatcaagcg 7260
   attggggctt ttagtcttcc gcgaatagtt gagattggaa agtccgcaga gaataaagct 7320
   gacgctttat tgacacggaa gcagattgct gtcttgaggc ttgtaaaaga ggggtgctca 7380
   aacaaacaaa tagcaacaaa tatgcatgtc accgaagatg ctataaagtg gcatatgagg 7440
   aaaatatttg ccaccttgaa tgtagtgaat cgcacgcaag caacaattga agctgagcgt 7500
   caaggaatta tctaaaataa tcggcattaa gtgatatagt gaaaagtata ccggagagag 7560
   aattatggca atcgttgttg ttggcgctgg tacagctgga gtaaatgctg cgttctggct 7620
   tcgtcaatat ggttataaag gggaaattag gatttttagc agggagtctg tggcgcctta 7680
   tcagcggcct cctctatcca aggcttttct gacaagtgag attgcagaat ccgcagtgcc 7740
   attaaagcca gaaggttttt atacgaataa caatattacc atttcgttaa atacaccgat 7800
   tgtatcaatc gacgtggggc gtaagatagt ttcttctaaa gatggaaaag aatacgcgta 7860
   tgaaaaattg attcttgcaa cacctgctag cgcacgtagg ttaacctgcg aggggtctga 7920
   actgtctggg gtctgctatt tacgcagtat ggaagacgcc aaaaatttac gtaggaaact 7980
   tgtggagagt gcgtctgttg ttgtgttggg cggcggagta atcgggcttg aagtcgcctc 8040
   agctgcggtg ggcttaggga agagggtcac agtgatagaa gccaccccgc gtgtaatggc 8100
   gcgcgtggtt acgccggcag cagcaaactt agtcagagcc cgcctggagg ctgaaggaat 8160
   tgagttcaag ctgaatgcga aattaacgtc tataaagggc aggaatggcc atgttgaaca 8220
   atgcgtactt gaaagtggag aagaaattca ggcggatctg attgtagttg gaatcggtgc 8280
   tatcccagag ctagagctgg caactgaggc ggcccttgaa gtgagtaatg gtgttgtggt 8340
   cgatgatcag atgtgtacat cggatacaag tatatatgca atcggcgact gcgcaatggc 8400
   tagaaatcct ttttggggaa cgatggtacg tttagagaca attcataatg cggttacaca 8460
   cgctcaaatt gtcgcaagta gcatctgtgg cacatcaaca ccagcaccaa ccccaccacg 8520
   gttctggtct gatcttaaag ggatggcgct gcaaggactt ggtgctctaa aggactacga 8580
   taaactcgtt gttgcaatta ataacgaaac tcttgaacta gaagtccttg cgtacaagca 8640
   ggagcgactg attgcaactg agacaataaa tttgcctaaa cgtcaaggtg cgcttgcagg 8700
   gagtataaaa ttacctgatt agcaatgatg ctcagccact cgaaccaacg gtcgcgatag 8760
   ggacggcagt tacctgccgc cccccgcact ccgtacgtgc ggaactaccg cgtaaaatgt 8820
   ggcccaggct gttatgtggc gcttgggcgg ggaagtattg ccatatttgg tgatgaccgt 8880
   tttctacgcc acataaatcg gtggtggcta tggtgggatt tcccttgctg aaatgggaga 8940
   tccgatcatg ttcgagctct tattcaaata cactgctgtg ttggcggtaa gcgttctcga 9000
   gctcatagtc cacgacgccc gtgattttgt agccctggcc gacggccagc aggtaggccg 9060
   acaggctcat gccggccgcc gccgcctttt cctcaatcgc tcttcgttcg tctggaaggc 9120
   agtacacctt gataggtggg ctgcccttcc tggttggctt ggtttcatca gccatccgct 9180
   tgccctcatc tgttacgccg gcggtagccg gccagcctcg cagagcagga ttcccgttga 9240
   gcaccgccag gtgcgaataa gggacagtga agaaggaaca cccgctcgcg ggtgggccta 9300
   cttcacctat cctgcccggc tgacgccgtt ggatacacca aggaaagtct acacgaaccc 9360
   tttggcaaaa tcctgtatat cgtgcgaaaa aggatggata taccgaaaaa atcgctataa 9420
   tgaccccgaa gcagggttat gcagcggaaa agcgctgctt ccctgctgtt ttgtggaata 9480
   tctaccgact ggaaacaggc aaatgcagga aattactgaa ctgaggggac aggcgagaga 9540
   ggatcaatgg ctatctgggg gaccgagggc tgtcgctgcg ccaaggcacg attggagatc 9600
   ccctatgcgg tgtgaaatac cgcacagatg cgtaaggaga aaataccgca tcaggcgctc 9660
   ttccgcttcc tcgctcactg actcgctgcg ctcggtcgtt cggctgcggc gagcggtatc 9720
   agctcactca aaggcggtaa tacggttatc cacagaatca ggggataacg caggaaagaa 9780
   catgtgagca aaaggccagc aaaaggccag gaaccgtaaa aaggccgcgt tgctggcgtt 9840
   tttccatagg ctccgccccc ctgacgagca tcacaaaaat cgacgctcaa gtcagaggtg 9900
   gcgaaacccg acaggactat aaagatacca ggcgtttccc cctggaagct ccctcgtgcg 9960
   ctctcctgtt ccgaccctgc cgcttaccgg atacctgtcc gcctttctcc cttcgggaag 10020
   cgtggcgctt tctcatagct cacgctgtag gtatctcagt tcggtgtagg tcgttcgctc 10080
   caagctgggc tgtgtgcacg aaccccccgt tcagcccgac cgctgcgcct tatccggtaa 10140
   ctatcgtctt gagtccaacc cggtaagaca cgacttatcg ccactggcag cagccactgg 10200
   taacaggatt agcagagcga ggtatgtagg cggtgctaca gagttcttga agtggtggcc 10260
   taactacggc tacactagaa ggacagtatt tggtatctgc gctctgctga agccagttac 10320
   cttcggaaaa agagttggta gctcttgatc cggcaaacaa accaccgctg gtagcggtgg 10380
   tttttttgtt tgcaagcagc agattacgcg cagaaaaaaa ggatctcaag aagatccttt 10440
   gatcttttct acggggtctg acgctcagtg gaacgaaaac tcacgttaag ggattttggt 10500
   catgagatta tcaaaaagga tcttcaccta gatcctttta aattaaaaat gaagttttaa 10560
   atcaatctaa agtatatatg agtaaacttg gtctgacagt taccaatcga ttggtcggtc 10620
   atttcgaacc ccagagtccc gctcagaaga actcgtcaag aaggcgatag aaggcgatgc 10680
   gctgcgaatc gggagcggcg ataccgtaaa gcacgaggaa gcggtcagcc cattcgccgc 10740
   caagctcttc agcaatatca cgggtagcca acgctatgtc ctgatagcgg tccgccacac 10800
   ccagccggcc acagtcgatg aatccagaaa agcggccatt ttccaccatg atattcggca 10860
   agcaggcatc gccatgggtc acgacgagat cctcgccgtc gggcatgcgc gccttgagcc 10920
   tggcgaacag ttcggctggc gcgagcccct gatgctcttc gtccagatca tcctgatcga 10980
   caagaccggc ttccatccga gtacgtgctc gctcgatgcg atgtttcgct tggtggtcga 11040
   atgggcaggt agccggatca agcgtatgca gccgccgcat tgcatcagcc atgatggata 11100
   ctttctcggc aggagcaagg tgagatgaca ggagatcctg ccccggcact tcgcccaata 11160
   gcagccagtc ccttcccgct tcagtgacaa cgtcgagcac agctgcgcaa ggaacgcccg 11220
   tcgtggccag ccacgatagc cgcgctgcct cgtcctgcag ttcattcagg gcaccggaca 11280
   ggtcggtctt gacaaaaaga accgggcgcc cctgcgctga cagccggaac acggcggcat 11340
   cagagcagcc gattgtctgt tgtgcccagt catagccgaa tagcctctcc acccaagcgg 11400
   ccggagaacc tgcgtgcaat ccatcttgtt caatcatgcg aaacgatcct catcctgtct 11460
   cttgatcaga tcttgatccc ctgcgccatc agatccttgg cggcaagaaa gccatccagt 11520
   ttactttgca gggcttccc 11539
<210> 32
   <211> 35
   <212> DNA
   <213> Artificial
<220>
   <223> Primer
<400> 32
   aagggaattc catatgcttg agaaacacag agttc 35
<210> 33
   <211> 35
   <212> DNA
   <213> Artificial
<220>
   <223> Primer
<400> 33
   aaaattcgcg tcgacaagcg ctgaatgggt atcgg 35
<210> 34
   <211> 20
   <212> DNA
   <213> Artificial
<220>
   <223> Primer
<400> 34
   tgagacagtg gctgttagag 20
<210> 35
   <211> 35
   <212> DNA
   <213> Artificial
<220>
   <223> Primer
<400> 35
   taataaccgc tcgagaacgc ttaccgccaa cacag 35
<210> 36
   <211> 47
   <212> DNA
   <213> Artificial
<220>
   <223> Primer
<400> 36
   gccggcgaga acctgtactt tcagatggca agtaagtatg ccacttg 47
<210> 37
   <211> 28
   <212> DNA
   <213> Artificial
<220>
   <223> Primer
<400> 37
   ggatcctcac ttcttgtgct gagccttg 28
<210> 38
   <211> 446
   <212> PRT
   <213> Arabidopsis thaliana
<400> 38
   Met Val Val Ile Asn Ser Leu Arg Arg Leu Ala Arg Thr Thr Gln Val
   1 5 10 15
   His Leu His Ser Lys Tyr Ala Thr Cys Met Ser Gly Asn Ser Thr Ser
   20 25 30
   Arg Arg Ile Phe Thr Thr Glu Ala Ala Pro Glu Lys Gly Asn Glu Pro
   35 40 45
   Arg Leu Val Ser Ala Ala Val Glu Gln Leu Asn Thr Leu Pro Phe Tyr
   50 55 60
   His Ser Phe Trp Asn Arg Thr Thr Lys Pro Ser Leu Asp Leu Ala Lys
   65 70 75 80
   Val Leu Leu Glu Met Phe Thr Ala Asn Lys Met Ala Lys Ala Phe Phe
   85 90 95
   Thr Ser Gly Gly Ser Asp Ala Asn Asp Thr Gln Val Lys Leu Val Trp
   100 105 110
   Tyr Tyr Asn Asn Ala Leu Gly Arg Pro Glu Lys Lys Lys Phe Ile Ala
   115 120 125
   Arg Lys Lys Ser Tyr His Gly Ser Thr Leu Ile Ser Ala Ser Leu Ser
   130 135 140
   Gly Leu Pro Pro Leu His Gln Asn Phe Asp Leu Pro Ala Pro Phe Val
   145 150 155 160
   Leu His Thr Asp Cys Pro His Tyr Trp Arg Phe His Leu Pro Gly Glu
   165 170 175
   Thr Glu Glu Glu Phe Ser Thr Arg Leu Ala Lys Asn Leu Glu Asp Leu
   180 185 190
   Ile Ile Lys Glu Gly Pro Glu Thr Ile Gly Ala Phe Ile Ala Glu Pro
   195 200 205
   Val Met Gly Ala Gly Gly Val Ile Pro Pro Pro Ala Thr Tyr Phe Glu
   210 215 220
   Lys Val Gln Ala Val Val Lys Lys Tyr Asp Ile Leu Phe Ile Ala Asp
   225 230 235 240
   Glu Val Ile Cys Ala Phe Gly Arg Leu Gly Thr Met Phe Gly Cys Asp
   245 250 255
   Lys Tyr Asn Ile Lys Pro Asp Leu Val Thr Leu Ala Lys Ala Leu Ser
   260 265 270
   Ser Ala Tyr Met Pro Ile Gly Ala Ile Leu Met Ser Gln Glu Val Ala
   275 280 285
   Asp Val Ile Asn Ser His Ser Ser Lys Leu Gly Val Phe Ser His Gly
   290 295 300
   Phe Thr Tyr Ser Gly His Pro Val Ser Cys Ala Val Ala Ile Glu Ala
   305 310 315 320
   Leu Lys Ile Tyr Lys Glu Arg Asn Ile Pro Glu Tyr Val Ala Lys Val
   325 330 335
   Ala Pro Arg Phe Gln Asp Gly Val Lys Ala Phe Ala Ser Gly Ser Pro
   340 345 350
   Ile Ile Gly Glu Thr Arg Gly Thr Gly Leu Ile Leu Gly Thr Glu Phe
   355 360 365
   Val Asp Asn Lys Ser Pro Asn Glu Pro Phe Pro Pro Glu Trp Gly Val
   370 375 380
   Gly Ala Phe Phe Gly Ala Glu Cys Gln Lys His Gly Met Leu Val Arg
   385 390 395 400
   Val Ala Gly Asp Gly Ile Leu Met Ser Pro Pro Leu Ile Ile Ser Pro
   405 410 415
   Glu Glu Ile Asp Glu Leu Ile Ser Ile Tyr Gly Lys Ala Leu Lys Ala
   420 425 430
   Thr Glu Glu Lys Val Lys Glu Leu Lys Ala Gln His Lys Lys
   435 440 445
<210> 39
   <211> 1515
   <212> DNA
   <213> Arabidopsis thaliana
<400> 39
   atggtcgtta tcaacagtct ccgacgcttg gcgcgtacca ctcaggttca tttgcacagt 60
   aagtatgcca cttgcatgtc tgggaactcc acttccagga ggattttcac tactgaggca 120
   gcacctgaga agaaaaacac tgttgggtct aaagggcatg atatgcttgc accttttact 180
   gctggatggc agagtgctga tttagatccc ttggtcattg caaagtctga gggaagttat 240
   gtgtatgatg atactgggaa aaaatatctt gactctctcg ctggtttatg gtgtactgcc 300
   ttaggaggaa atgagccaag gcttgtttct gccgctgttg aacagttgaa caccttgccg 360
   ttttatcact ccttttggaa ccgtactact aaaccttctc tggatcttgc taaggttctt 420
   ttagagatgt tcacggccaa caaaatggcc aaagcatttt ttacaagcgg tggatcagat 480
   gccaacgata cccaggtcaa gctggtttgg tattacaata acgcacttgg aaggcccgag 540
   aagaaaaagt ttatcgcgag aaagaaatcg taccatggct ccactctaat atcagcaagt 600
   ttgtccggcc ttcccccgct acaccaaaat tttgatttac ctgcaccatt tgtgttgcac 660
   acagattgcc ctcattattg gcgttttcat cttccaggcg aaacggaaga ggagttctca 720
   accagattag ccaagaattt agaggatcta atcatcaaag aaggaccaga aactattggt 780
   gcttttatag ctgaaccagt catgggtgct gggggtgtga tacctccacc tgctacctac 840
   tttgaaaagg ttcaagctgt tgttaagaaa tatgatatct tgttcattgc tgatgaggtg 900
   atatgtgcat ttggaaggct cgggacaatg tttggctgtg acaaatacaa cattaagcca 960
   gatcttgtga ccttagctaa ggcactgtct tcagcatata tgccgattgg agccattctt 1020
   atgagccaag aagtggcaga tgtcataaat tctcatagca gcaagctagg cgttttctcc 1080
   catggattta cttattctgg tcatccagtt tcgtgtgctg tagcaattga agcgttaaag 1140
   atatacaagg agaggaacat accagagtat gtcgccaaag ttgccccaag gtttcaagat 1200
   ggagttaaag cgtttgcctc tggtagtcct attattggag agacaagagg aacaggtttg 1260
   attcttggga ctgagtttgt agacaataaa tctccgaacg aaccatttcc accagaatgg 1320
   ggtgttggcg cattctttgg agccgagtgc cagaagcacg ggatgttagt ccgtgttgca 1380
   ggtgatggca ttttgatgtc tccaccgctc attatctcac ctgaagagat tgatgagttg 1440
   atttctatct atgggaaagc attgaaggca acggaagaga aggtaaaaga actcaaggct 1500
   cagcacaaga agtga 1515
<210> 40
   <211> 1362
   <212> DNA
   <213> Pseudomonas sp.
<400> 40
   atgagcgtca acaacccgca aacccgtgaa tggcaaaccc tgagcgggga gcaccatctc 60
   gcacctttca gtgactacaa gcagctgaag gagaaggggc cgcgcatcat caccaaggcc 120
   cagggggtgc atttgtggga cagcgagggg cacaagatcc tcgacggcat ggcgggcctg 180
   tggtgcgtgg cggtgggtta cggccgtgaa gagctggttc aggcagcaga aaagcagatg 240
   cgcgagctgc cgtactacaa cctgttcttc cagacggccc acccgcctgc actggaactg 300
   gccaaggcca ttaccgatgt ggcgcccgag ggcatgaccc atgtgttctt caccggctcc 360
   ggctccgaag gcaacgacac cgtgctgcgc atggtgcgcc actactgggc gttgaagggc 420
   aagccgcaca agcagaccat catcggccgt atcaacggct accacggctc caccttcgcc 480
   ggtgcttgcc tgggcggcat gagcggcatg cacgagcagg gcggcctgcc gatcccgggc 540
   atcgtgcaca tcccgcagcc gtactggttc ggcgaaggcg gtgacatgac cccggatgcg 600
   ttcggtatct gggcggccga acagctggag aagaaaatcc tcgaagtcgg cgaagacaac 660
   gtcgccgcct tcatcgccga gcctatccag ggcgcaggcg gcgtgatcat cccgccggaa 720
   acctactggc cgaaggtgaa ggagattctc gccaagtacg acatcctgtt cgttgccgac 780
   gaagtcatct gtggtttcgg ccgtaccggc gagtggttcg gctctgatta ctacgacctc 840
   aagcccgacc tgatgaccat cgccaagggc ctgacctccg gttacatccc catgggcggt 900
   gtgatcgtgc gtgacaaagt ggccaaggtg atcagcgaag gcggtgactt caaccacggc 960
   ttcacctatt cgggccaccc ggtagcggcg gcggtgggcc tggaaaacct gcgcatcctg 1020
   cgcgacgagc aaattgtcga gaaggcgcgt actgaagcgg caccgtattt gcaaaagcgt 1080
   ttgcgtgagc tgcaggacca cccgctggtg ggtgaagtgc gcggccttgg catgcttggc 1140
   gcgatcgagc tggtgaaaga caaggccacc cgcagccgtt acgagggcaa gggcgtgggc 1200
   atgatctgcc gcaccttctg cttcgaaaac ggcctgatca tgcgtgcggt gggtgacacc 1260
   atgatcatcg cgccgccgct ggtcatcagc catgcggaaa tcgacgaact ggtggaaaag 1320
   gcacgcaaat gcctcgacct gacccttgag gcgattcgat aa 1362
<210> 41
   <211> 1359
   <212> DNA
   <213> Pseudomonas sp.
<400> 41
   atgagcgatt cgcaaaccct gcactggcaa gcgctgagcc gcgaccatca tctgccgccg 60
   ttcaccgatt acaaggcgct gaacgccaaa ggcacgcgca tcatcaccaa ggcgtcaggc 120
   gtctacctgt gggacagcga aggccacaag atcctcgacg ccatggccgg gctctggtgc 180
   gtcaacctgg gctatggccg cgaggagctg gtcgaggccg cgaccaggca gatgcgcgag 240
   ctgccgtact acaacctgtt cttccagacc gcccacccgc cggccgtggc gctggccaag 300
   gcgattgccg acatcgcgcc ggccgggatg aaccatgtgt tcttcaccgg ctccggctcg 360
   gaggccaacg acaccgtgct gcgcatggtg cgccattact gggcgatcaa gggccagccg 420
   gcgaagaagg tggtcatcgg ccgctggaat ggctatcacg gctcgaccat cgctggcgca 480
   agcctcggtg gcatgaaggc catgcacgag cagggcgacg gcccgatccc cggcatcgag 540
   catatcgacc agccctactg gttcggcgag ggtggcgaca tgagcccgga agagttcggc 600
   gtgcgcatcg ccgaccagct ggagcagaag atccttgagg tcggcgagga caaggtcgcc 660
   gccttcatcg ccgaacctat ccagggcgcc ggcggcgtga tcatcccgcc cgagagctac 720
   tggccgcggg tcaaggaaat cctcgcgcgc tacgacattc tcttcatcgc cgacgaggtc 780
   atctgcggct tcggccgtac cggtgagtgg ttcggcagcg actactacgg cctcgagccg 840
   gacctgatgc cgatcgccaa gggcctgacc tctggctaca tccccatggg cggcgtagtg 900
   gtgcgcgacg aagtggtgca cacgctcaac gagggcggcg agttctacca cggcttcacc 960
   tactcggggc accccgtggc cgccgcggtg gcgctggaga acatccgcat cctgcgcgag 1020
   gagaagatcg tcgagcgggt gaagacgaag acggcaccct atttgcagtc ccgttggcag 1080
   gaactgctcg agcatccgct ggtaggcgag gcgcgcggcg tcggcctgct tggtgcgctg 1140
   gagttggtga agaacaagaa gacccgcgaa cgctttgccg atcccggtgt gggcatgctc 1200
   tgtcgcgagc actgcttccg caacggcctg gtgatgcgtg cggttggcga caccatgatc 1260
   atttcgccgc cgctggtgat cagcgaagag cagatcgacg agctggttgg caaggtgcgg 1320
   ttgtgcctcg acgccacggc caaggatgtg ctgggctga 1359
<210> 42
   <211> 1380
   <212> DNA
   <213> Artificial
<220>
   <223> codon optimized gene for expression in E coli
<400> 42
   atgcagaaac agcgtaccac ctctcagtgg cgtgaactgg atgcggcgca tcatctgcat 60
   ccgtttaccg ataccgcgag cctgaatcag gcgggtgcgc gtgtgatgac ccgtggcgaa 120
   ggcgtgtatc tgtgggatag cgaaggcaac aaaattattg atggcatggc gggcctgtgg 180
   tgcgtgaacg tgggctatgg ccgtaaagat tttgcggaag cggcgcgtcg tcagatggaa 240
   gaactgccgt tttataacac cttctttaaa accacccatc cggcggtggt ggaactgagc 300
   agcctgctgg ccgaagttac cccggcaggt tttgatcgtg tgttttatac caacagcggc 360
   agcgaaagcg tggataccat gattcgtatg gtgcgtcgtt attgggatgt gcagggcaaa 420
   ccggaaaaaa aaaccctgat tggccgttgg aacggctatc acggcagcac cattggcggt 480
   gcgagcctgg gcggcatgaa atatatgcat gaacagggcg atctgccgat tccgggcatg 540
   gcgcatattg aacagccgtg gtggtataaa catggcaaag atatgacccc ggatgaattt 600
   ggcgtggttg cggcgcgttg gctggaagaa aaaattctgg aaatcggcgc ggataaagtg 660
   gcggcgtttg tgggcgaacc gattcagggt gcgggcggtg tgattgttcc gccggcaacc 720
   tattggccgg aaattgaacg tatttgccgc aaatatgatg tgctgctggt tgcggatgaa 780
   gtgatttgcg gctttggccg taccggcgaa tggtttggcc atcagcattt tggctttcag 840
   ccggacctgt ttaccgcggc gaaaggcctg agcagcggct atctgccgat tggcgcggtg 900
   tttgtgggca aacgtgttgc ggaaggtctg attgcgggcg gtgattttaa ccatggcttt 960
   acctatagcg gccatccggt gtgtgcggcg gtggcgcatg cgaatgttgc ggcgctgcgt 1020
   gatgaaggca ttgtgcagcg tgtgaaagat gatattggcc cgtatatgca gaaacgttgg 1080
   cgtgaaacct ttagccgttt tgaacatgtg gatgatgtgc gtggcgtggg catggtgcag 1140
   gcgtttaccc tggtgaaaaa caaagcgaaa cgtgaactgt ttccggattt tggcgaaatt 1200
   ggcaccctgt gccgcgatat tttttttcgc aacaacctga ttatgcgtgc gtgcggcgat 1260
   cacattgtgt ctgcaccgcc gctggttatg acccgtgcgg aagtggatga aatgctggcc 1320
   gtggcggaac gttgcctgga agaatttgaa cagaccctga aagcgcgtgg cctggcctaa 1380
<210> 43
   <211> 5143
   <212> DNA
   <213> Artificial
<220>
   <223> Vector
<400> 43
   tttaagaagg agatataccc atgacacaga gggcccacca tcaccatcac cattccatgg 60
   cctcctccga ggacgtcatc aaggagttca tgcgcttcaa ggtgcgcatg gagggctccg 120
   tgaacggcca cgagttcgag atcgagggcg agggcgaggg ccgcccctac gagggcaccc 180
   agaccgccaa gctgaaggtg accaagggcg gccccctgcc cttcgcctgg gacatcctgt 240
   cccctcagtt ccagtacggc tccaaggcct acgtgaagca ccccgccgac atccccgact 300
   acttgaagct gtccttcccc gagggcttca agtgggagcg cgtgatgaac ttcgaggacg 360
   gcggcgtggt gaccgtgacc caggactcct ccctgcagga cggcgagttc atctacaagg 420
   tgaagctgcg cggcaccaac ttcccctccg acggccccgt aatgcagaag aagactatgg 480
   gttgggaggc ctccaccgag cggatgtacc ccgaggacgg cgccctgaag ggcgagatca 540
   agatgaggct gaagctgaag gacggcggcc actacgacgc cgaggtcaag accacctaca 600
   tggccaagaa gcccgtgcag ctgcccggcg cctacaagac cgacatcaag ctggacatca 660
   cctcccacaa cgaggactac accatcgtgg aacagtacga gcgcgccgag ggccgccact 720
   ccaccggcgc cggcgagaac ctgtactttc agatggcaag taagtatgcc acttgcatgt 780
   ctgggaactc cacttccagg aggattttca ctactgaggc agcacctgag aagaaaaaca 840
   ctgttgggtc taaagggcat gatatgcttg caccttttac tgctggatgg cagagtgctg 900
   atttagatcc cttggtcatt gcaaagtctg agggaagtta tgtgtatgat gatactggga 960
   aaaaatatct tgactctctc gctggtttat ggtgtactgc cttaggagga aatgagccaa 1020
   ggcttgtttc tgccgctgtt gaacagttga acaccttgcc gttttatcac tccttttgga 1080
   accgtactac taaaccttct ctggatcttg ctaaggttct tttagagatg ttcacggcca 1140
   acaaaatggc caaagcattt tttacaagcg gtggatcaga tgccaacgat acccaggtca 1200
   agctggtttg gtattacaat aacgcacttg gaaggcccga gaagaaaaag tttatcgcga 1260
   gaaagaaatc gtaccatggc tccactctaa tatcagcaag tttgtccggc cttcccccgc 1320
   tacaccaaaa ttttgattta cctgcaccat ttgtgttgca cacagattgc cctcattatt 1380
   ggcgttttca tcttccaggc gaaacggaag aggagttctc aaccagatta gccaagaatt 1440
   tagaggatct aatcatcaaa gaaggaccag aaactattgg tgcttttata gctgaaccag 1500
   tcatgggtgc tgggggtgtg atacctccac ctgctaccta ctttgaaaag gttcaagctg 1560
   ttgttaagaa atatgatatc ttgttcattg ctgatgaggt gatatgtgca tttggaaggc 1620
   tcgggacaat gtttggctgt gacaaataca acattaagcc agatcttgtg accttagcta 1680
   aggcactgtc ttcagcatat atgccgattg gagccattct tatgagccaa gaagtggcag 1740
   atgtcataaa ttctcatagc agcaagctag gcgttttctc ccatggattt acttattctg 1800
   gtcatccagt ttcgtgtgct gtagcaattg aagcgttaaa gatatacaag gagaggaaca 1860
   taccagagta tgtcgccaaa gttgccccaa ggtttcaaga tggagttaaa gcgtttgcct 1920
   ctggtagtcc tattattgga gagacaagag gaacaggttt gattcttggg actgagtttg 1980
   tagacaataa atctccgaac gaaccatttc caccagaatg gggtgttggc gcattctttg 2040
   gagccgagtg ccagaagcac gggatgttag tccgtgttgc aggtgatggc attttgatgt 2100
   ctccaccgct cattatctca cctgaagaga ttgatgagtt gatttctatc tatgggaaag 2160
   cattgaaggc aacggaagag aaggtaaaag aactcaaggc tcagcacaag aagtgaggat 2220
   ccggctgcta acaaagcccg aaaggaagct gagttggctg ctgccaccgc tgagcaataa 2280
   ctagcataac cccttggggc ctctaaacgg gtcttgaggg gttttttgct gaaaggagga 2340
   actatatccg gccggatatc cacaggacgg gtgtggtcgc catgatcgcg tagtcgatag 2400
   tggctccaag tagcgaagcg agcaggactg ggcggcggcc aaagcggtcg gacagtgctc 2460
   cgagaacggg tgcgcataga aattgcatca acgcatatag cgctagcagc acgccatagt 2520
   gactggcgat gctgtcggaa tggacgatat cccgcaagag gcccggcagt accggcataa 2580
   ccaagcctat gcctacagca tccagggtga cggtgccgag gatgacgatg agcgcattgt 2640
   tagatttcat acacggtgcc tgactgcgtt agcaatttaa ctgtgataaa ctaccgcatt 2700
   aaagcttatc gatgataagc tgtcaaacat gagaattctt gaagacgaaa gggcctcgtg 2760
   atacgcctat ttttataggt taatgtcatg catgagacaa taaccctgat aaatgcttca 2820
   ataatattga aaaaggaaga gtatgagtat tcaacatttc cgtgtcgccc ttattccctt 2880
   ttttgcggca ttttgccttc ctgtttttgc tcacccagaa acgctggtga aagtaaaaga 2940
   tgctgaagat cagttgggtg cacgagtggg ttacatcgaa ctggatctca acagcggtaa 3000
   gatccttgag agttttcgcc ccgaagaacg ttttccaatg atgagcactt ttaaagttct 3060
   gctatgtggc gcggtattat cccgtgttga cgccgggcaa gagcaactcg gtcgccgcat 3120
   acactattct cagaatgact tggttgacgc gtcaccagtc acagaaaagc atcttacgga 3180
   tggcatgaca gtaagagaat tatgcagtgc tgccataacc atgagtgata acactgcggc 3240
   caacttactt ctgacaacga tcggaggacc gaaggagcta accgcttttt tgcacaacat 3300
   gggggatcat gtaactcgcc ttgatcgttg ggaaccggag ctgaatgaag ccataccaaa 3360
   cgacgagcgt gacaccacga tgcctgcagc aatggcaaca acgttgcgca aactattaac 3420
   tggcgaacta cttactctag cttcccggca acaattaata gactggatgg aggcggataa 3480
   agttgcagga ccacttctgc gctcggccct tccggctggc tggtttattg ctgataaatc 3540
   tggagccggt gagcgtgggt ctcgcggtat cattgcagca ctggggccag atggtaagcc 3600
   ctcccgtatc gtagttatct acacgacggg gagtcaggca actatggatg aacgaaatag 3660
   acagatcgct gagataggtg cctcactgat taagcattgg taactgtcag accaagttta 3720
   ctcatatata ctttagattg atttaaaact tcatttttaa tttaaaagga tctaggtgaa 3780
   gatccttttt gataatctca tgcatgacca aaatccctta acgtgagttt tcgttccact 3840
   gagcgtcaga ccccgtagaa aagatcaaag gatcttcttg agatcctttt tttctgcgcg 3900
   taatctgctg cttgcaaaca aaaaaaccac cgctaccagc ggtggtttgt ttgccggatc 3960
   aagagctacc aactcttttt ccgaaggtaa ctggcttcag cagagcgcag ataccaaata 4020
   ctgtccttct agtgtagccg tagttaggcc accacttcaa gaactctgta gcaccgccta 4080
   catacctcgc tctgctaatc ctgttaccag tggctgctgc cagtggcgat aagtcgtgtc 4140
   ttaccgggtt ggactcaaga cgatagttac cggataaggc gcagcggtcg ggctgaacgg 4200
   ggggttcgtg cacacagccc agcttggagc gaacgaccta caccgaactg agatacctac 4260
   agcgtgagct atgagaaagc gccacgcttc ccgaagggag aaaggcggac aggtatccgg 4320
   taagcggcag ggtcggaaca ggagagcgca cgagggagct tccaggggga aacgcctggt 4380
   atctttatag tcctgtcggg tttcgccacc tctgacttga gcgtcgattt ttgtgatgct 4440
   cgtcaggggg gcggagccta tggaaaaacg ccagcaacgc ggccttttta cggttcctgg 4500
   ccttttgctg gccttttgct cacatgttct ttcctgcgtt atcccctgat tctgtggata 4560
   accgtattac cgcctttgag tgagctgata ccgctcgccg cagccgaacg accgagcgca 4620
   gcgagtcagt gagcgaggaa gcggaagagc gcctgatgcg gtattttctc cttacgcatc 4680
   tgtgcggtat ttcacaccgc atatatggtg cactctcagt acaatctgct ctgatgccgc 4740
   atagttaagc cagtatacac tccgctatcg ctacgtgact gggtcatggc tgcgccccga 4800
   cacccgccaa cacccgctga cgcgccctga cgggcttgtc tgctcccggc atccgcttac 4860
   agacaagctg tgaccgtctc cgggagctgc atgtgtcaga ggttttcacc gtcatcaccg 4920
   aaacgcgcga ggcagctggc acgacaggtt tcccgactgg aaagcgggca gtgagcgcaa 4980
   cgcaattaat gtgagttagc tcactcatta ggcaccccag gctttacact ttatgcttcc 5040
   ggctcgtatg ttgtgtggaa ttgtgagcgg ataacaattt cacacaggaa acagctatga 5100
   ccatgattac gccaagctct agctagaaat aattttgttt aac 5143
<210> 44
   <211> 36
   <212> DNA
   <213> Artificial
<220>
   <223> Primer
<400> 44
   ggaattccat atgagcgtca acaacccgca aacccg 36
<210> 45
   <211> 37
   <212> DNA
   <213> Artificial
<220>
   <223> Primer
<400> 45
   ccgctcgagt tatcgaatcg cctcaagggt caggtcc 37
<210> 46
   <211> 38
   <212> DNA
   <213> Artificial
<220>
   <223> Primer
<400> 46
   ggaattccat atgagcgatt cgcaaaccct gcactggc 38
<210> 47
   <211> 35
   <212> DNA
   <213> Artificial
<220>
   <223> Primer
<400> 47
   cgcggatcct cagcccagca catccttggc tgtcg 35

## Beschreibung

Die vorliegende Erfindung betrifft gegenüber ihrem Wildtyp gentechnisch veränderte Zellen, ein Verfahren zur Herstellung von ω-Aminocarbonsäuren, ω-Aminocarbonsäureestern oder von aus ω-Aminocarbonsäuren abgeleiteten Lactamen und, ein Verfahren zur Herstellung von auf ω-Aminocarbonsäuren oder auf Lactamen basierenden Polyamiden.

Polyamide sind Polymere, deren Wiederholungseinheiten (Monomere) als charakteristisches Merkmal die Amid-Gruppe besitzen. Die Bezeichnung "Polyamide" wird üblicherweise als Bezeichnung für synthetische, technisch verwendbare thermoplastische Kunststoffe verwendet und grenzt diese Stoffklasse damit von den chemisch verwandten Proteinen ab. Fast alle bedeutsamen Polyamide leiten sich von primären Aminen ab, d.h. in ihren Wiederholeinheiten kommt die funktionelle Gruppe -CO-NH- vor. Daneben existieren auch Polyamide von sekundären Aminen (-CO-NR-, R = organischer Rest). Als Monomere für die Polyamide finden besonders Aminocarbonsäuren, Lactame und/oder Diamine und Dicarbonsäuren Verwendung.

Besondere Bedeutung kommt insbesondere der Herstellung von Polyamiden auf der Basis von Lactamen zu. So wird durch Ringöffnungspolymerisation von ω-Caprolactam das technisch häufig verwendete Produkt "Polyamid 6" erhalten, während durch Ringöffnungspolymerisation von Laurinlactam das technisch ebenfalls wichtige "Polyamid 12" erhalten wird. Auch Copolymere aus Lactamen, wie etwa Copolymere aus ω-Caprolactam und Laurinlactam ("Polyamid 6/12") sind von großer, technischer Bedeutung.

Die Herstellung von ω-Caprolactam erfolgt üblicherweise durch Umsetzung von Cyclohexanon mit dem Hydrogensulfat öder dem Hydrochlorid des Hydroxylamins unter Bildung von Cyclohexanonoxim. Dieses wird durch eine Beckmann-Umlagerung in ε-Caprolactam umgewandelt, wobei häufig konzentrierte Schwefelsäure als Katalysator eingesetzt wird. Die Herstellung des Cyclohexanons erfolgt üblicherweise durch katalytische Oxidation von Cyclohexan mit Luftsauerstoff, wobei Cyclohexan wiederum durch Hydrierung von Benzol erhalten wird.

Besonders aufwendig ist die Herstellung von Laurinlactam. Diese erfolgt großtechnisch dadurch, dass zunächst Butadien unter Bildung von Cyclododecatrien trimerisiert wird. Das Cyclododecatrien wird anschließend unter Bildung von Cyclododecan hydriert und das so erhaltene Cyclododecan unter Bildung von Cyclododecanon oxidiert. Das auf diese Weise erhaltene Cyclododecanon wird sodann mit Hydroxylamin zu Cyclododecanoxim umgesetzt, welches dann in einer Beckmann-Umlagerung in Laurinlactam umgewandelt wird.

Der Nachteil dieser aus dem Stand der Technik bekannten Verfahren zur Herstellung von Lactamen mittels Beckmann-Umlagerung von Oximen besteht unter anderem darin, dass als Nebenprodukt große Mengen Salze wie beispielsweise Natriumsulfat gebildet werden, die entsorgt werden müssen. Im Stand der Technik werden daher auch andere Verfahren zur Herstellung von Lactamen beschrieben, welche diese Nachteile nicht aufweisen. So beschreibt EP-A-0 748 797 ein Verfahren zur Herstellung von Lactamen aus Dinitrilen, bei dem das Dinitril zum Aminonitril hydriert und das Aminonitril durch cyclisierende Hydrolyse zum Lactam umgesetzt wird. Als Katalysator für die cyclisierende Hydrolyse sind Molekularsiebe, wie saure Zeolithe, Silikate und nicht-zeolithische Molekularsiebe, Metallphosphate und Metalloxide oder Metallmischoxide offenbart. Dieses Verfahren weist jedoch unter anderem den Nachteil auf, dass die Selektivität der Umsetzung des Aminonitrils durch die cyclisierende Hydrolyse eher gering ist und daher große Mengen an Nebenprodukten gebildet werden. Darüber hinaus werden bei den aus diesem Stand der Technik beschriebenen Verfahren zur Herstellung von Lactamen Kohlenwasserstoffe wie Benzol oder Butadien eingesetzt, welche durch Cracken von Benzin oder Erdöl gewonnen werden und sich daher von nicht nachwachsenden Rohstoffen ableiten. Die Herstellung von Polyamiden, welche auf derart hergestellten Lactamen basieren, ist daher aus ökologischer sicht als nachteilig anzusehen.

Der vorliegenden Erfindung lag die Aufgabe zugrunde, die sich aus dem Stand der Technik ergebenden Nachteile zu überwinden.

Insbesondere lag der vorliegenden Erfindung die Aufgabe zugrunde, ein Verfahren anzugeben, mit dem Lactame, insbesondere Laurinlactam, in möglichst wenigen Verfahrensschritten und unter Bildung von möglichst wenigen Nebenprodukten gebildet werden können.

Weiterhin war es eine Aufgabe der vorliegenden Verbindung, ein Verfahren anzugeben, mit dem Lactame, insbesondere Laurinlactam, aus nachwachsenden Rohstoffen hergestellt werden kann.

Einen Beitrag zur Lösung der vorstehend genannten Aufgaben leistet eine Zelle, welche gegenüber ihrem Wildtyp derart gentechnisch verändert worden ist, dass sie im Vergleich zu ihrem Wildtyp mehr ω-Aminocarbonsäuren, ω-Aminocarbonsäureester oder mehr aus ω-Aminocarbonsäuren abgeleitete Lactame aus Carbonsäuren oder Carbonsäureestern herzustellen vermag, wobei die Zelle eine im Vergleich zu ihrem Wildtyp gesteigerte Aktivität der Enzyme E_{I} und E_{III} oder der Enzyme E_{I}, E_{II} und E_{III} aufweist:
i) eines Enzyms E_{I}, welches die Umsetzung von Carbonsäuren oder Carbonsäureestern zu den entsprechenden ω-Hydroxycarbonsäuren oder ω-Hydroxycarbonsäureestern katalysiert, ausgewählt aus der Gruppe bestehend aus den Alkanmonooxygenasen:
   *alkBGT* kodierte Alkanmonoxygenase aus *Pseudomonas putida* GPo1 und Cytochrom-P450-Monoxygenasen aus *Candida tropicalis;*
ii) eines Enzyms E_{II}, welches die Umsetzung von ω-Hydroxycarbonsäuren oder ω-Hydroxycarbonsäureestem zu den entsprechenden ω-Oxocarbonsäuren oder ω-Oxocarbonsäureestem katalysiert, ausgewählt aus der Gruppe bestehend aus den *alk*J*-*Gen kodierten Alkoholdehydrogenasen;
ii) eines Enzyms E_{III}, welches die Umsetzung von ω-Oxocarbonsäuren oder ω-Oxocarbonsäureestem zu den entsprechenden ω-Aminocarbonsäuren oder ω-Aminocarbonsäureestern katalysiert ausgewählt aus der Gruppe der ω-Transaminasen.

Eine solche Zelle kann eingesetzt werden, um ω-Aminocarbonsäuren, ω-Aminocarbonsäureester oder aber aus ω-Aminocarbonsäuren abgeleitete Lactame auf fermentativem Weg aus Carbonsäuren oder Carbonsäureestern, beispielsweise aus Laurinsäure oder Laurinsäureestern, herzustellen.

Die Formulierung *"dass sie im Vergleich zu ihrem Wildtyp mehr* ω*-Aminocarbonsäuren, ω-Aminocarbonsäureester oder mehr aus ω*-*aminocarbonsäuren abgeleitete Lactame aus Carbonsäuren oder Carbonsäureestern herzustellen vermag"* betrifft auch den Fall, dass der Wildtyp der gentechnisch veränderten Zelle überhaupt keine ω-Aminocarbonsäuren, ω-Aminocarbonsäureester oder keine aus ω-Aminocarbonsäuren abgeleiteten Lactame, zumindest aber keine nachweisbaren Mengen dieser Verbindungen zu bilden vermag und erst nach der gentechnischen Veränderung nachweisbare Mengen dieser Komponenten gebildet werden können.

Unter einem *"Wildtyp"* einer Zelle wird vorzugsweise eine Zelle bezeichnet, deren Genom in einem Zustand vorliegt, wie er natürlicherweise durch die Evolution entstanden ist. Der Begriff wird sowohl für die gesamte Zelle als auch für einzelne Gene verwendet. Unter den Begriff *"Wildtyp"* fallen daher insbesondere nicht solche Zellen bzw. solche Gene, deren Gensequenzen zumindest teilweise durch den Menschen mittels rekombinanter Verfahren verändert worden sind.

Dabei ist es erfindungsgemäß bevorzugt, dass die gentechnisch veränderte Zelle derart gentechnisch verändert ist, dass sie in einem definierten Zeitintervall, vorzugsweise innerhalb von 2 Stunden, noch mehr bevorzugt innerhalb von 8 Stunden und am meisten bevorzugt innerhalb von 24 Stunden, mindestens 2mal, besonders bevorzugt mindestens 10mal, darüber hinaus bevorzugt mindestens 100mal, darüber hinaus noch mehr bevorzugt mindestens 1.000mal und am meisten bevorzugt mindestens 10.000mal mehr ω-Aminocarbonsäuren, ω-Aminocarbonsäureester oder aus ω**-**Aminocarbonsäuren abgeleitete Lactame bildet als der Wildtyp der Zelle. Die Zunahme der Produktbildung kann dabei beispielsweise dadurch bestimmt werden, dass die erfindungsgemäße Zelle und die Wildtyp-Zelle jeweils getrennt unter gleichen Bedingungen (gleiche Zelldichte, gleiches Nährmedium, gleiche Kulturbedingungen) für ein bestimmtes Zeitintervall in einem geeigneten Nährmedium kultiviert werden und anschließend die Menge an Zielprodukt (ω-Aminocarbonsäuren, ω-Aminocarbonsäureester oder aus ω**-**Aminocarbonsäuren abgeleiteten Lactame) im Nährmedium bestimmt wird.

Die erfindungsgemäßen Zellen können Prokaryonten oder Eukaryonten sein. Dabei kann es sich um Säugetierzellen (wie etwa Zellen aus dem Menschen), um pflanzliche Zellen oder um Mikroorganismen wie Hefen, Pilze oder Bakterien handeln, wobei Mikroorganismen besonders bevorzugt und Bakterien und Hefen am meisten bevorzugt sind.

Als Bakterien, Hefen oder Pilze sind insbesondere diejenigen Bakterien, Hefen oder Pilze geeignet, die bei der Deutschen Sammlung von Mikroorganismen und Zellkulturen GmbH (DSMZ), Braunschweig, Deutschland, als Bakterien-, Hefe- oder Pilz-Stämme hinterlegt sind. Erfindungsgemäß geeignete Bakterien gehören zu den Gattungen, die unter http://www.dsmz.de/species/bacteria.htm
aufgeführt sind, erfindungsgemäß geeignete Hefen gehören zu denjenigen Gattungen, die unter
http://www.dsmz.de/species/yeasts.htm
aufgeführt sind und erfindungsgemäß geeignete Pilze sind diejenigen, die unter
http://www.dsmz.de/species/fungi.htm
aufgeführt sind.

Erfindungsgemäß besonders bevorzugte Zellen leiten sich ab von Zellen der Gattungen *Corynebacterium, Brevibacterium, Bacillus, Lactobacillus, Lactococcus, Candida, Pichia, Kluveromyces*, *Saccharomyces*, *Escherichia*, *Zymomonas, Yarrowia, Methylobacterium, Ralstonia*, *Pseudomonas*, *Burkholderia* und *Clostridium,* wobei *Escherichia coli, Corynebakterium glactamicum* und *Pseudomonas putida* besonders bevorzugt und *Escherichia coli* am meisten bevorzugt sind.

Der Begriff *"gesteigerte Aktivität eines Enzyms",* wie er vorstehend im Zusammenhang mit dem Enzym E_{I} und in den nachfolgenden Ausführungen im Zusammenhang mit den Enzymen E_{II} usw. verwendet wird, ist vorzugsweise als gesteigerte intrazelluläre Aktivität zu verstehen.

Die nun folgenden Ausführungen zur Erhöhung der Enzymaktivität in Zellen gelten sowohl für die Erhöhung der Aktivität des Enzyms E_{I} als auch für alle nachfolgend genannten Enzyme, deren Aktivität gegebenenfalls erhöht werden kann.

Grundsätzlich lässt sich eine Steigerung der enzymatischen Aktivität dadurch erzielen, dass man die Kopienzahl der Gensequenz bzw. der Gensequenzen erhöht, welche für das Enzym kodieren, einen starken Promotor verwendet oder ein Gen oder Allel nutzt, das für ein entsprechendes Enzym mit einer gesteigerten Aktivität kodiert und gegebenenfalls diese Maßnahmen kombiniert. Erfindungsgemäß gentechnisch veränderte Zellen werden beispielsweise durch Transformation, Transduktion, Konjugation oder einer Kombination dieser Methoden mit einem Vektor erzeugt, der das gewünschte Gen, ein Allel dieses Gens oder Teile davon und einen die Expression des Gens ermöglichenden Vektor enthält. Die heterologe Expression wird insbesondere durch Integration des Gens oder der Allele in das Chromosom der Zelle oder einem extrachromosomal replizierenden Vektor erzielt.

Einen Überblick über die Möglichkeiten zur Erhöhung der Enzym-Aktivität in Zellen am Beispiel der Pyruvat-Carboxylase gibt DE-A-100 31 999, die hiermit als Referenz eingeführt wird und deren Offenbarungsgehalt hinsichtlich der Möglichkeiten zur Erhöhung der Enzym-Aktivität in Zellen einen Teil der Offenbarung der vorliegenden Erfindung bildet.

Die Expression der vorstehend und aller nachfolgend genannten Enzyme bzw. Gene ist mit Hilfe von 1- und 2-dimensionaler Proteingelauftrennung und anschließender optischer Identifizierung der Proteinkonzentration mit entsprechender Auswertesoftware im Gel nachweisbar. Wenn die Erhöhung einer Enzymaktivität ausschließlich auf einer Erhöhung der Expression des entsprechenden Gens basiert, so kann die Quantifizierung der Erhöhung der Enzymaktivität in einfacher Weise durch einen Vergleich der 1- oder 2-dimensionalen Proteinauftrennungen zwischen Wildtyp und gentechnisch veränderter Zelle bestimmt werden. Eine gebräuchliche Methode zur Präparation der Proteingele bei coryneformen Bakterien und zur Identifizierung der Proteine ist die von Hermann et al. (Electrophoresis, 22: 1712.23 (2001) beschriebene Vorgehensweise. Die Proteinkonzentration kann ebenfalls durch Western-Blot-Hybridisierung mit einem für das nachzuweisende Protein spezifischen Antikörper (Sambrook et al., Molecular Cloning: a laboratory manual, 2nd Ed. Cold Spring Harbor Laboratory Press, Cold Spring Harbor, N.Y. USA, 1989) und anschließender optische Auswertung mit entsprechender Software zur Konzentrationsbestimmung (Lohaus und Meyer (1989) Biospektrum, 5: 32-39; Lottspeich (1999), Angewandte Chemie 111: 2630-2647) analysiert werden. Die Aktivität von DNA-bindenden Proteinen kann mittels DNA-Band-Shift-Assays (auch als Gelretardation bezeichnet) gemessen werden (Wilson et al. (2001) Journal of Bacteriology, 183: 2151-2155). Die Wirkung von DNA-bindenden Proteinen auf die Expression anderer Gene kann durch verschiedene gut beschriebene Methoden des Reportergen-Assays nachgewiesen werden (Sambrook et al., Molecular Cloning: a laboratory manual, 2nd Ed. Cold Spring Harbor Laboratory Press, Cold Spring Harbor, N.Y. USA, 1989). Die intrazellulären enzymatischen Aktivitäten können nach verschiedenen beschriebenen Methoden (Donahue et al. (2000) Journal of Bacteriology 182 (19): 5624-5627; Ray et al. (2000) Journal ofBacteriology 182 (8): 2277-2284; Freedberg et al. (1973) Journal of Bacteriology 115 (3): 816-823) bestimmt werden. Sofern in den nachfolgenden Ausführungen keine konkreten Methoden zur Bestimmung der Aktivität eines bestimmten Enzyms angegeben werden, erfolgt die Bestimmung der Steigerung der Enzymaktivität und auch die Bestimmung der Verminderung einer Enzymaktivität vorzugsweise mittels der in Hermann et al., Electophoresis, 22: 1712-23 (2001), Lohaus et al., Biospektrum 5 32-39 (1998), Lottspeich, Angewandte Chemie 111: 2630-2647 (1999) und Wilson et al., Journal of Bacteriology 183: 2151-2155 (2001) beschriebenen Methoden.

Wird die Erhöhung der Enzymaktivität durch Mutation des endogenen Gens bewerkstelligt, so können derartige Mutationen entweder nach klassischen Methoden ungerichtet erzeugt werden, wie etwa durch UV-Bestrahlung oder durch mutationsauslösende Chemikalien, oder gezielt mittels gentechnologischer Methoden wie Deletion(en), Insertion(en) und/oder Nukleotidaustausch(e). Durch diese Mutationen werden gentechnisch veränderte Zellen erhalten. Besonders bevorzugte Mutanten von Enzymen sind insbesondere auch solche Enzyme, die nicht mehr oder zumindest im Vergleich zum Wildtyp-Enzym vermindert *feedback*-inhibierbar sind.

Wird die Erhöhung der Enzymaktivität durch Erhöhung der Expression eines Enzyms bewerkstelligt, so erhöht man beispielsweise die Kopienzahl der entsprechenden Gene oder mutiert die Promotor- und Regulationsregion oder die Ribosomenbindungsstelle, die sich stromaufwärts des Strukturgens befindet. In gleicher Weise wirken Expressionskassetten, die stromaufwärts des Strukturgens eingebaut werden. Durch induzierbare Promotoren ist es zusätzlich möglich, die Expression zu jedem beliebigen Zeitpunkt zu steigern. Des Weiteren können dem Enzym-Gen als regulatorische Sequenzen aber auch sogenannte *"Enhancer"* zugeordnet sein, die über eine verbesserte Wechselwirkung zwischen RNA-Polymerase und DNA ebenfalls eine erhöhte Genexpression bewirken. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte liegen dabei entweder in Plasmiden mit unterschiedlicher Kopienzahl vor oder sind im Chromosom integriert und amplifiziert. Alternativ kann weiterhin eine Überexpression der betreffenden Gene durch Veränderung der Medienzusammensetzung und Kulturfiihrung erreicht werden. Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in EP-A-0 472 869, im US 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in WO-A-96/15246, bei Malumbres et al. (Gene 134, 15-24 (1993), in JP-A-10-229891, bei Jensen und Hammer (Biotechnology and Bioengineering 58, 191-195 (1998)) und in bekannten Lehrbüchern der Genetik und Molekularbiologie. Die vorstehend beschriebenen Maßnahmen führen ebenso wie die Mutationen zu gentechnisch veränderten Zellen.

Zur Erhöhung der Expression der jeweiligen Gene werden zum Beispiel episomale Plasmide eingesetzt. Als Plasmide eignen sich insbesondere solche, die in coryneformen Bakterien repliziert werden. Zahlreiche bekannte Plasmidvektoren, wie zum Beispiel pZ1 (Menkel et al., Applied and Environmental Microbiology 64: 549-554 (1989)), pEKEx1 (Eikmanns et al., Gene 107: 69-74 (1991)) oder pHS2-1 (Sonnen et al., Gene 107: 69-74 (1991)) beruhen auf den kryptischen Plasmiden pHM1519, pBL1 oder pGA1. Andere Plasmidvektoren, wie zum Beispiel solche, die aufpCG4 (US 4,489,160) oder pNG2 (Serwold-Davis et al., FEMS Microbiology Letters 66: 119-124 (1990)) oder pAG1 (US 5,158,891) beruhen, können in gleicher Weise eingesetzt werden.

Weiterhin eignen sich auch solche Plasmidevektoren, mit Hilfe derer man das Verfahren der Genamplifikation durch Integration in das Chromosom anwenden kann, so wie es beispielsweise von Reinscheid et al. (Applied and Environmental Microbiology 60: 126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise *Escherichia coli*)*,* nicht aber in *Corynebacterium glutamicum* repliziert werden kann. Als Vektoren kommen beispielsweise pSUP301 (Simon et al., BiolTechnology 1: 784-791 (1983)), pK18mob oder pK19mob (Schäfer et al., Gene 145: 69-73 (1994)), pGEM-T (Promega Corporation, Madison, Wisconsin, USA), pCR2.1-TOPO (Shuman, Journal of Biological Chemistry 269: 32678-84 (1994)), pCR^{®}Blunt (Invitrogen, Groningen, Niederlande), pEM1 (Schrumpf et al., Journal of Bacteriology 173: 4510-4516)) oder pBGS8 (Spratt et al., Gene 41: 337-342 (1986)) in Frage. Der Plasmidvektor, der das zu amplifizierende Gen enthält, wird anschließend durch Konjugation oder Transformation in den gewünschten Stamm von *Corynebacterium glutamicum* überführt. Die Methode der Konjugation ist beispielsweise bei Schäfer et al., Applied and Environmental Microbiology 60: 756-759 (1994) beschrieben. Methoden zur Transformation sind beispielsweise bei Thierbach et al., Applied Microbiology and Biotechnology 29: 356-362 (1988), Dunican und Shivnan, Bio/Technology 7: 1067-1070 (1989) und Tauch et al., FEMS Microbiology Letters 123: 343-347 (1994) beschrieben. Nach homologer Rekombination mittels eines "cross-over"-Ereignisses enthält der resultierende Stamm mindestens zwei Kopien des betreffenden Gens.

Unter der vorstehend und in den nachfolgenden Ausführungen verwendeten Formulierung *"eine gegenüber ihrem Wildtyp gesteigerte Aktivität eines Enzyms Eₓ*" ist vorzugsweise stets eine um ein en Faktor von mindestens 2, besonders bevorzugt von mindestens 10, darüber hinaus bevorzugt von mindestens 100, darüber hinaus noch mehr bevorzugt von mindestens 1.000 und am meisten bevorzugt von mindestens 10.000 gesteigerte Aktivität des jeweiligen Enzyms Eₓ zu verstehen. Weiterhin umfasst die erfindungsgemäße Zelle, welche *"eine gegenüber ihrem Wildtyp gesteigerte Aktivität eines Enzyms Eₓ"* aufweist, insbesondere auch eine Zelle, deren Wildtyp keine oder zumindest keine nachweisbare Aktivität dieses Enzyms Eₓ aufweist und die erst nach Erhöhung der Enzymaktivität, beispielsweise durch Überexpression, eine nachweisbare Aktivität dieses Enzyms Eₓ zeigt. In diesem Zusammenhang umfasst der Begriff *"Überexpression"* oder die in den nachfolgenden Ausführungen verwendete Formulierung *erhöhung der Expression"* auch den Fall, dass eine Ausgangszelle, beispielsweise eine Wildtyp-Zelle, keine oder zumindest keine nachweisbare Expression aufweist und erst durch rekombinante Verfahren eine nachweisbare Expression des Enzyms Eₓ induziert wird.

Ein bevorzugtes Enzym E₁ ist die vom *alk*BGT-Gen kodierte Alkanmonoxygenase aus *Pseudomonas putida* GPO1. Die Isolierung der *alk*BGT-Gensequenz ist beispielsweise von van Beilen et al. in "Functional Analysis of Alkane Hydroxylases from Gram-Negative and Gram-Positive Bacteria", Journal of Bacteriology, Vol. 184 (6), Seiten 1.733-1.742 (2002) beschrieben. Weiterhin können als Alkanmonoxygenasen auch Cytochrom-P450-Monoxygenasen aus *Candida tropicalis* eingesetzt werden. Die Gensequenzen geeigneter Cytochrom-P450-Monoxygenasen aus *Candida tropicalis* sind beispielsweise in WO-A-00/20566 offenbart.

Ein bevorzugtes Enzym E_{II} ist insbesondere die vom *alk*J*-*Gen kodierte Alkohol-Dehydrogenase aus *Pseudomonas pactida* GPo1. Die Gensequenzen der vom *alk*J*-*Gen kodierten Alkoholdehydrogenase aus *Pseudomonas pactida* GPo1, *Alcanivorax borkumensis, Bordetella parapertussis, Bordetella bronchiseptica* oder aus *Roseobacter denitrificans* können beispielsweise der KEGG-Gendatenbank entnommen werden.

Geeignete ω-Transaminasen sind beispielsweise diejenigen ω-Transaminasen, die in US-A-2007/0092957 durch die Sequenz-Nummern 248, 250, 252 und 254 charakterisiert werden.

Ein bevorzugtes Enzym E_{III}, insbesondere eine bevorzugte ω-Transaminase ist insbesondere die ω**-**Transaminase aus *Chromobacterium violaceum* DSM30191 (Kaulmann et al, 2007; "Substrate spectrum of ω-transaminase from Chromobacterium violaceum DSM30191 and its potential for biocatalysis", Enzyme and Microbial Technology, Vol. 41, Seiten 628-637), welche von der Gensequenz gemäß SEQ.-ID.-Nr. 01 kodiert wird.
Es kann von Vorteil sein, als Enzym E_{III} ω-Transaminasen einzusetzen, die sich aus Planzen isolieren lassen. Bevorzugt werden hier ω-Transaminasen aus Pflanzen ausgewählt aus der Gruppe enthaltend *Arabidopsis thaliana, Avena sativa, Beta vulgaris, Glycine max, Hordeum vulgare, Lotus japonicus, Solanum lycopersicum, Manihot esculenta, Oryza sativa, Traeticum aestivum, Zea mays, Spinacia oleracea, Arum maculatum, Mercurialis perennis* und *Urtica dioica,* wobei *Arabidopsis thaliana* besonders bevorzugt ist. Als ω-Transaminasen eignen sich insbesondere Enzyme die durch Nukelinsäuren kodiert werden, die 90 %, bevorzugt 95 %, besonders bevorzugt 99 und ganz besonders bevorzug 100 % Identität zu der Sequenz gemäß SEQ.-ID.-Nr. 39 aufweisen. Die "Nukleotid-Identität" relativ zur SEQ.-ID-Nr. 39 wird hierbei mit Hilfe bekannter Verfahren bestimmt. Generell werden besondere Computerprogramme mit Algorithmen unter Berücksichtigung spezieller Erfordernisse verwendet. Bevorzugte Verfahren zur Bestimmung der Identität erzeugen zunächst die größte Übereinstimmung zwischen den zu ver-gleichenden Sequenzen. Computer-Programme zur Bestimmung der Identität umfassen, sind jedoch nicht beschränkt auf, das GCG- Programmpaket, einschließlich
- GAP (Deveroy, J. et al., Nucleic Acid Research 12 (1984), Seite 387, Genetics Computer Group University of Wisconsin, Medicine (Wi), und
- BLASTP, BLASTN und FASTA (Altschul, S. et al., Journal of Molecular Biology 215 (1990), Seiten 403-410. Das BLAST-Programm kann erhalten werden vom National Center For Biotechnology Information (NCBI) und aus weiteren Quellen (BLAST Handbuch, Altschul S. et al., NCBI NLM NIH Bethesda ND 22894; Altschul S. et al., vorstehend).

Der bekannte Smith-Waterman Algorithmus kann ebenso zur Be-stimmung der Nukleotid-Identität verwendet werden.

Bevorzugte Parameter zum Nukleotidvergleich umfassen die nachfolgenden:
- Algorithmus Needleman und Wunsch, Journal of Molecular Biology 48 (1970), Seiten 443-453
- Vergleichsmatrix
Matches = + 10
Mismatches = 0
Gap penalty = 50
Gap length penalty = 3
Das GAP-Programm ist ebenso zur Verwendung mit den vorste-henden Parametern geeignet. Die vorstehenden Parameter sind die default-Parameter im Nukleotidsequenzvergleich.

Des Weiteren sind Enzyme aus der Untergruppe der β-Ala:Pyruvat-Transaminasen geeignet. Hierzu gehören z.B. Transaminasen aus *Pseudomonas putida* W619 (gi: 119860707, gi: 119855857, gi: 119856278), aus *Pseudomonas putida* KT2440 (gi: 24984391), aus *Pseudomonas aeruginosa* PA01 (gi 15595330, gi: 15600506, gi 15595418, gi 9951072); *Streptomyces coelicolor* A3(2) (gi: 3319731), *Streptomyces avermitilis* MA 4680 (gi: 29831094, gi: 29829154) und *Chromobacterium violaceum* ATCC 12472 (gi 34102747). Die Aminosäuresequenzen der vorstehend genannten Transaminasen sind in den Sequenzen gemäß SEQ.-ID.-Nr. 19 bis SEQ.-ID.-Nr. 30 wiedergegeben.

Für den Fall, dass die erfindungsgemäßen Zellen eingesetzt werden sollen, um ω-Aminocarbonsäuren, ω-Aminocarbonsäureester oder auf ω- Aminocarbonsäuren basierende Lactame ausgehend von Carbonsäureestern herzustellen, ist es weiterhin vorteilhaft, wenn die erfindungsgemäße Zelle neben der gesteigerten Aktivität der Enzyme E_{I} und E_{III} oder E_{I}, E_{II} und E_{III} auch eine gesteigerte Aktivität eines Enzyms E_{IV} aufweist, welches die Umsetzung von ω-Aminocarbonsäureestern zu den entsprechenden ω-Aminocarbonsäuren katalysiert, wobei es sich bei diesem Enzym E_{IV} vorzugsweise um eine Esterase handelt, welche vorzugsweise von der Zelle sezerniert wird. Die Sezernierung der Esterase durch die Zelle hat den Vorteil, dass die Esterbindung erst außerhalb der Zelle gespalten wird. Auf diese Weise kann sichergestellt werden, dass infolge der besseren Membranpermeabilität des ω-Aminocarbonsäureesters im Vergleich zur ω-Aminocarbonsäure ausreichend Zielprodukt die Zelle verlässt und in das die Zelle umgebende Nährmedium übertreten kann.

Erfindungsgemäß bevorzugte Esterasen sind insbesondere Lipase, wobei als Beispiel einer geeigneten Lipase die Lipase LipA aus *Pseudomonas fluoreszenz* HU380 (ACC Code Q76D26, Kojima und Shimizu, "purification and Characterization of the Lipase from Pseudomonas flacorescens HU380", Journal of Bioscience and Bioengineering, Volume 96 (3), Seiten 219-226 (2003)) genannt sei. Um sicherzustellen, dass die Esterasen sezerniert werden, können sie in dem Fachmann bekannter Weise mit entsprechenden Signalsequenzen versehen werden, die ein Sezernieren sicherstellen. Wird beispielsweise die vorstehend genannte Lipase LipA aus *Pseudomonas fluoreszenz* HU380 in *E*. *coli* überexpremiert, so kann sie vorteilhafterweise mit Signalsequenzen von EstA, einer Esterase, die natürlicherweise auf der Zelloberfläche von *Pseudomonas aeruginosa* vorkommt (Becker et al., "A generic system for the Escherichia coli cell-sacrface display of lipolytic enzymes", FEBS Letters, Vol. 579, Seiten 1177-1182 (2005)), versehen werden. Weitere geeignete Enzyme sind Lipasen aus C. *antarctica, M. miehei* und P. *cepacia* (Vaysse et al., "Enzyme and Microbial Technology", Vol. 31, Seiten 648-655 (2002)). Alternativ kann der sezernierte ω-Aminocarbonsäureester auch konventionell gespalten werden, um die ω-Aminocarbonsäure zu erhalten, so z.B. durch Verseifung, d.h. Hydrolyse des ω-Aminocarbonsäureesters durch die wässrige Lösung eines Hydroxids, z. B. durch Natriumhydroxid.

Weiterhin kann es sich erfindungsgemäß als vorteilhaft erweisen, wenn die erfindungsgemäße Zelle neben der gesteigerten Aktivität der Enzyme E_{I} und E_{III} oder E_{I}, E_{II} und E_{III} und gegebenenfalls auch neben einer gesteigerten Aktivität des vorstehend beschriebenen Enzyms E_{IV} auch eine gesteigerte Aktivität eines Enzyms Eᵥ aufweist, welches die Umsetzung von ω-Aminocarbonsäuren zu den entsprechenden Lactamen katalysiert, wobei es auch hier vorteilhaft sein kann, wenn dieses Enzym Eᵥ von der Zelle sezeniert wird. Auf diese Weise kann erreicht werden, dass die von der Zelle unmittelbar gebildete ω-Aminocarbonsäuren oder aber die erst nach extrazellulärer Spaltung von ω-Aminocarbonsäureestern gebildete ω-Aminocarbonsäure in das entsprechende Lactam überführt wird, um so gegebenenfalls die Aufreinigung des Zielproduktes zu erleichtern.

Gemäß einer weiteren, besonderen Ausführungsform der erfindungsgemäßen Zelle weist diese neben der erhöhten Aktivität eines oder mehrerer der Enzyme E_{I}, E_{II} oder E_{III} sowie gegebenenfalls der erhöhten Aktivität des Enzyms E_{IV} und/oder Ev auch eine erhöhte Aktivität eines Enzyms E_{VI} auf, welches die Umsetzung einer α-Ketocarbonsäure zu einer Aminosäure katalysiert, wobei es sich bei diesem Enzym E_{VI} vorzugsweise um eine Aminosäure-Dehydrogenase handelt. Eine solche Modifizierung der Zelle hätte den Vorteil, dass die im Falle eines Einsatzes von Aminosäuren als Donor für die NH₂-Gruppe, welche bei der durch die Transaminase (E_{III}) vermittelten Reaktion einer ω-Oxocarbonsäure oder eines ω-Oxocarbonsäureesters zur entsprechenden ω-Aminocarbonsäure, zum entsprechenden ω-Aminocarbonsäureester bzw. zum entsprechenden ω-Aminocarbonsäureester verbraucht wird, entsprechend regeneriert werden kann. Als Aminosäure-Dehydrogenase bevorzugt ist die Alanin-Dehydrogenase aus *B. subtilis* (EC Nr. 1.4.1.1; Gene ID: 936557), welche von der Gensequenz gemäß SEQ.-ID.-Nr. 02 kodiert. Weitere geeignete Aminosäure-Dehydrogenasen sind Serin-Dehydrogenasen, Aspartat-Dehydrogenasen, Phenylalanin-Dehydrogenasen und Glutamat-Dehydrogenasen.

Einen weiteren Beitrag zur Lösung der eingangs genannten Zellen leistet ein Verfahren zur Herstellung von ω-Aminocarbonsäuren, von ω-Aminocarbonsäureestern oder von aus ω-Aminocarbonsäuren abgeleiteten Lactamen, beinhaltend die Verfahrensschritte:
I) in Kontakt bringen einer erfindungsgemäßen Zelle mit einem Kulturmedium beinhaltend eine Carbonsäure oder einen Carbonsäureester oder mit einem Kulturmedium angrenzend an eine organische Phase beinhaltend eine Carbonsäure oder einen Carbonsäureester unter Bedingungen, die es der Zelle ermöglichen, aus der Carbonsäure oder aus dem Carbonsäureestern ω-Aminocarbonsäuren, ω-Aminocarbonsäureester oder aus ω-Aminocarbonsäuren abgeleitete Lactame zu bilden;
II) gegebenenfalls Isolierung der gebildeten ω-Aminocarbonsäuren, der ω-Aminocarbonsäureester oder der aus ω-Aminocarbonsäuren abgeleiteten Lactame.

Im Verfahrensschritt I) des erfindungsgemäßen Verfahrens werden die Zellen zunächst mit einem Kulturmedium beinhaltend eine Carbonsäure oder einen Carbonsäureester oder mit einem Kulturmedium angrenzend an eine organische Phase beinhaltend eine Carbonsäure oder einen Carbonsäureester in Kontakt gebracht, wobei dieses in Kontakt bringen unter Bedingungen erfolgt, die es der Zelle ermöglichen, aus der Carbonsäure oder aus dem Carbonsäureestern ω-Aminocarbonsäuren, ω-Aminocarbonsäureester oder aus ω-Aminocarbonsäuren abgeleitete Lactame zu bilden.

Die erfindungsgemäßen, gentechnisch veränderten Zellen können kontinuierlich oder diskontinuierlich im *batch*-Verfahren (Satzkultivierung) oder im *fed-batch*-Verfahren (Zulaufverfahren) oder *repeated-fed-batch*-Verfahren (repetitives Zulaufverfahren) zum Zwecke der Produktion von ω-Aminocarbonsäuren oder von aus ω**-**Aminocarbonsäuren abgeleiteten Lactamen mit dem Nährmedium in Kontakt und somit kultiviert werden. Denkbar ist auch ein semi-kontinuierliches Verfahren, wie es in der GB-A-1009370 beschrieben wird. Eine Zusammenfassung über bekannte Kultivierungsmethoden sind im Lehrbuch von Chmiel ("Bioprozesstechnik 1. Einführung in die Bioverfahrenstechnik" (Gustav Fischer Verlag, Stuttgart, 1991)) oder im Lehrbuch von Storhas ("Bioreaktoren und periphere Einrichtungen", Vieweg Verlag, Braunschweig/Wiesbaden, 1994) beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch *"*Manual of Methods for General Bacteriology" der American Society for Bacteriology (Washington D. C., USA, 1981) enthalten.

Als Kohlenstoffquelle können, neben den Carbonsäuren oder Carbonsäureestern, Kohlehydrate wie z. B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Methanol, Kohlenwasserstoffe wie Methan, Aminosäuren wie L-Glutamat oder L-Valin oder organische Säuren wie z. B. Essigsäure verwendet werden. Diese Stoffe können einzeln oder als Mischung verwendet werden. Besonders bevorzugt ist der Einsatz von Kohlehydraten, insbesondere von Monosacchariden, Oligosacchariden oder Polysacchariden, wie dies in US 6,01,494 und US 6,136,576 beschrieben ist, von C₅-Zuckern oder von Glycerin.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten, werden Sauerstoff oder Sauerstoff-haltige Gasmischungen wie z. B. Luft in die Kultur eingetragen. Die Temperatur der Kultur liegt normalerweise bei 20°C bis 45°C und vorzugsweise bei 25°C bis 40°C.

Gemäß einer besonders bevorzugten Ausführungsform des erfindungsgemäßen Verfahrens zur Herstellung von ω-Aminocarbonsäuren, von ω-Aminocarbonsäureestern oder von aus ω-Aminocarbonsäuren abgeleiteten Lactamen, bei dem als erfindungsgemäße Zelle eine rekombinante Zelle eingesetzt wird, die sich von einer *E. coli-Zelle* ableitet, wird als Nährmedium ein mit Ampicillin, Chloramphenicol und Kanamycin supplemetiertes Mineralsalzmedium nach Riesenberg et al., "High cell density fermentation of recombinant Escherichia coli expressing human interferon alpha 1", Appl Microbiol and Biotechnololgy, Vol. 34 (1), Seiten 77-82 (1990)) eingesetzt.

Das in Kontakt bringen der erfindungsgemäßen Zellen mit dem Kulturmedium im Verfahrensschritt I) erfolgt vorzugsweise unter Bedingungen, die es der Zelle ermöglichen, aus der Carbonsäure oder aus dem Carbonsäureestern ω-Aminocarbonsäuren, ω-Aminocarbonsäureester oder aus ω-Aminocarbonsäuren abgeleitete Lactame zu bilden. Als Carbonsäuren bzw. Carbonsäureester kommen insbesondere Carbonsäuren mit einer Kohlenstoffzahl in einem Bereich von 6 bis 20, besonders bevorzugt von 6 bis 15, insbesondere von 6 bis 12, in Betracht, wobei Laurinsäure als Carbonsäure besonders bevorzugt ist. Als Carbonsäureester kommen insbesondere die Methyl- oder Ethylester der vorstehend genannten Carbonsäuren in Betracht, wobei der Methylester der Laurinsäure als Carbonsäureester besonders bevorzugt ist.

Bei der Herstellung der ω-Aminocarbonsäuren, der ω-Aminocarbonsäureester oder der aus ω-Aminocarbonsäuren abgeleitete Lactame im Verfahrensschritt I) sind unterschiedliche Vorgehensweisen denkbar.

Gemäß einer Ausführungsform des erfindungsgemäßen Verfahrens werden die Zellen zunächst zum Zwecke der Biomasseproduktion in einem Nährmedium kultiviert, welches keine Carbonsäuren oder Carbonsäureester, insbesondere keine der vorstehend genannten, bevorzugten Carbonsäuren oder Carbonsäureester enthält. Erst nach Erhalt einer bestimmten Biomasse werden dann die Carbonsäuren oder die Carbonsäureester dem Nährmedium zugesetzt bzw. werden die Zellen mit einem neuen Nährmedium beinhaltend die Carbonsäuren oder Carbonsäureester in Kontakt gebracht. In diesem Zusammenhang ist es insbesondere bevorzugt, dass der Gehalt an Carbonsäuren bzw. an Carbonsäureestern während der Bildung von ω-Aminocarbonsäuren, von ω-Aminocarbonsäureester oder von ω-Aminocarbonsäuren abgeleiteten Lactamen vorzugsweise in einem Bereich von 1 bis 200 g/l, besonders bevorzugt in einem Bereich von 20 bis 200 g/l liegt.

Gemäß einer anderen Ausführungsform des erfindungsgemäßen Verfahrens wird dieses in einem Zwei-Phasen-System durchgeführt, beinhaltend
A) eine wässrige Phase, sowie
B) eine organische Phase,
wobei die Bildung der ω-Aminocarbonsäuren, der ω-Aminocarbonsäureester oder der aus ω-Aminocarbonsäuren abgeleiteten Lactame durch die Zellen im Verfahrensschritt I) in der wässrigen Phase erfolgt und sich die gebildeten ω-Aminocarbonsäuren, die gebildeten ω-Aminocarbonsäureester oder die gebildeten, aus ω**-**Aminocarbonsäuren abgeleiteten Lactame in der organischen Phase anreichern. Auf dieses Weise ist es möglich, die gebildeten ω-Aminocarbonsäuren, die gebildeten ω-Aminocarbonsäureester oder die gebildeten, aus ω-Aminocarbonsäuren abgeleiteten Lactame *in situ* zu extrahieren.

Auch bei dieser Ausführungsform des erfindungsgemäßen Verfahrens kann es sich als vorteilhaft erweisen, die Zellen zunächst zum Zwecke der Biomasseproduktion in einem Nährmedium zu kultivieren, welches keine Carbonsäuren oder Carbonsäureester, insbesondere keine der vorstehend genannten, bevorzugten Carbonsäuren oder Carbonsäureester enthält. Erst nach Erhalt einer bestimmten Biomasse wird dann die Zellsuspension als wässrige Phase A) mit der organischen Phase B) in Kontakt gebracht, wobei insbesondere die organische Phase B) die Carbonsäure oder den Carbonsäureester vorzugsweise in einer Menge in einem Bereich von 1 bis 200 g/l, besonders bevorzugt in einem Bereich von 20 bis 200 g/l beinhaltet. Werden jedoch als Carbonsäuren oder Carbonsäureester Substrate eingesetzt, die für die verwendeten Zellen nicht toxisch sind, wie etwa Laurinsäuremethylester, so kann der Gehalt der organischen Phase an diesen Carbonsäuren oder Carbonsäureester auch signifikant höher sein. In einem solchen Fall kann es sich auch anbieten, als organische Phase die reine Carbonsäure bzw. den reinen Carbonsäureester, beispielsweise reinen Laurinsäuremethylester, einzusetzen.

Als organische Phase können Alkane mittlerer Kettenlänge, vorzugsweise solche mit einem logP-Wert von mehr als 4 (wenig Schaumbildung), oder physikalisch ähnliche Aromaten oder Aromatenester eingesetzt werden, vorzugsweise jedoch, wie vorstehend ausgeführt, Laurinsäureester, besonders bevorzugt Laurinsäuremethylester, BEHP (Bis(2-ethylhexyl)phthalat) oder langkettige Fettsäureester (Biodiesel).

Weiterhin ist es erfindungsgemäß bevorzugt, dass das im Verfahrensschritt I) eingesetzte Kulturmedium zumindest während der Phase der Bildung von ω-Aminocarbonsäuren, von ω-Aminocarbonsäureester oder von aus ω**-**Aminocarbonsäuren abgeleiteten Lactamen Aminogruppen-Donatoren, wie etwa Ammoniak bzw. Ammonium-Ionen oder aber Aminosäuren, insbesondere jedoch Alanin oder Aspartat enthält, die als Amin-Donor bei der durch die Transaminase katalysierten Umsetzung der ω-Oxocarbonsäuren oder der ω-Oxocarbonsäureester zu den entsprechenden ω**-**Aminocarbonsäuren oder ω-Aminocarbonsäureestern fungieren.

Im Verfahrensschritt II) des erfindungsgemäßen Verfahrens werden die gebildeten ω-Aminocarbonsäuren, die gebildeten ω-Aminocarbonsäureester oder die aus den ω-Aminocarbonsäuren abgeleiteten Lactame gegebenenfalls isoliert, wobei es bevorzugt ist, dass diese Isolierung über ein mindestens zweistufiges Aufreinigungsverfahren erfolgt, umfassend
a) einen Extraktionsschritt, bei dem die ω-Aminocarbonsäuren, die ω-Aminocarbonsäureester oder die aus ω**-**Aminocarbonsäuren abgeleiteten Lactame aus dem Kulturmedium extrahiert werden, sowie
b) einen Feinreinigungsschritt, bei dem der im Verfahrensschritt a) erhaltene Extrakt über Destillationsverfahren oder eine selektive Kristallisation unter Erhalt einer ω-Aminocarbonsäure-Phase, einer ω-Aminocarbonsäureester-Phase oder einer Lactam-Phase mit einer Reinheit von mindestens 99,8 % weiter aufgereinigt wird.

Die Extraktion im Verfahrensschritt a) kann insbesondere als sogenannte *"in situ*"-Extraktion ausgestaltet sein, bei der die Verfahrensschritt I) und II) des erfindungsgemäßen Verfahrens zur Herstellung von ω-Aminocarbonsäuren, von ω-Aminocarbonsäureestern oder von aus ω-Aminocarbonsäuren abgeleiteten Lactamen zeitgleich durchgeführt werden. Diese *"in situ"-*Extraktion wurde bereits vorstehend beschrieben.

Die Feinreinigung im Verfahrensschritt II) kann beispielsweise über eine Destillation oder Kristallisation erfolgen.

In einer besonderen Ausführungsform erfindungsgemäßen Verfahrens zur Herstellung von ω-Aminocarbonsäuren, von ω-Aminocarbonsäureestern oder von aus ω-Aminocarbonsäuren abgeleiteten Lactamen werden die in Verfahrensschritt I) gebildeten ω-Aminocarbonsäureester in einem weiteren Verfahrensschritt mit konventionellen chemischen Verfahren zu ω-Aminocarbonsäuren umgesetzt; bevorzugtes, konventionelles chemisches Verfahren stellt die Verseifung dar, bei der der ω-Aminocarbonsäureester mir einer wässrigen Lösung einer Base, bevorzugt eines Hydroxides, bevorzugt Natriumhydroxid, zu der ω-Aminocarbonsäure umgesetzt wird.
Bevorzugt wird dieses Verfahren zur Herstellung von ω-Aminolaurinsäure aus Laurinsäureestern, bevorzugt Laurinsäuremethylester, eingesetzt;

Einen Beitrag zur Lösung der eingangs genannten Aufgaben leistet auch ein Verfahren zur Herstellung von auf ω-Aminocarbonsäuren basierenden Polyamiden, umfassend die Verfahrensschritte:
(α1) Herstellung von ω-Aminocarbonsäuren durch eines der vorstehend beschriebene Verfahren zur Herstellung ω-Aminocarbonsäuren, insbesondere durch die vorstehend beschriebenen Verfahren zur Herstellung von ω-Aminolaurinsäure aus Laurinsäure oder Laurinsäureestern;
(α2) Polymerisation der ω-Aminocarbonsäure unter Erhalt eines Polyamids.

Im Verfahrensschritt (α2) des erfindungsgemäßen Verfahrens zur Herstellung von auf ω-Aminocarbonsäuren basierenden Polyamiden werden die im Verfahrensschritt (α1) erhaltenenen ω-Aminocarbonsäuren, insbesondere die im Verfahrensschritt (α1) erhaltene ω-Aminolaurinsäuren in einer Polymeristaion zu einem Polyamid umgesetzt, wobei gegebenenfalls auch Mischungen aus verschiedenen ω-Aminocarbonsäuren eingesetzt werden können, von denen zumindest eines der ω-Aminocarbonsäuren, gegebenenfalls aber auch alle ω-Aminocarbonsäuren durch das erfindungsgemäße Verfahren zur Herstellung ω-Aminocarbonsäuren hergestellt wurden.

Die Herstellung der Polyamide aus den ω-Aminocarbonsäuren kann durch an sich bekannte Verfahren erfolgen, wie sie beispielsweise in L. Notarbartolo, Ind. Plast. Mod. 10(1958)2, S. 44, JP 01-074224, JP 01-051433, JP63286428, JP58080324 oder JP60179425 beschrieben sind.

Einen Beitrag zur Lösung der eingangs genannten Aufgaben leistet auch ein Verfahren zur Herstellung von auf Lactamen basierenden Polyamiden, umfassend die Verfahrensschritte:
(β1) Herstellung von Lactamen durch das vorstehend beschriebene Verfahren zur Herstellung von aus ω-Aminocarbonsäuren abgeleiteten Lactamen, insbesondere durch das vorstehend beschriebene Verfahren zur Herstellung von Laurinlactam aus Laurinsäure oder Laurinsäureestern;
(β2) Ringöffnungspolymerisation oder Polykondensation des Laurinlactams unter Erhalt eines Polyamids.

Im Verfahrensschritt (β2) des erfindungsgemäßen Verfahrens zur Herstellung von auf Lactamen basierenden Polyamiden werden die im Verfahrensschritt (β1) erhaltenenen Lactame, insbesondere das im Verfahrensschritt (β1) erhaltene Laurinlactam in einer Ringöffnungspolymerisation oder durch Polykondensation zu einem Polyamid umgesetzt, wobei gegebenenfalls auch Mischungen aus verschiedenen Lactamen, beispielsweise Mischungen aus Laurinlactam und ε-Caprolactam eingesetzt werden können, von denen zumindest eines der Lactame, gegebenenfalls aber auch alle Lactame durch das erfindungsgemäße Verfahren zur Herstellung von aus ω-Aminocarbonsäuren abgeleiteten Lactamen hergestellt wurden.

Die Herstellung der Polyamide aus den Lactamen kann durch an sich bekannte Verfahren erfolgen, wie sie beispielsweise in der DE-A-14 95 198, der DE-A-25 58 480, der EP-A-0 129 196 oder aber in *"*Polymerization Processes", Inters- cience, New York, 1977, Seiten 424-467, insbesondere Seiten 444-446, beschrieben sind.

Die Erfindung wird nun anhand nicht limitierender Figuren und Beispiele näher erläutert.

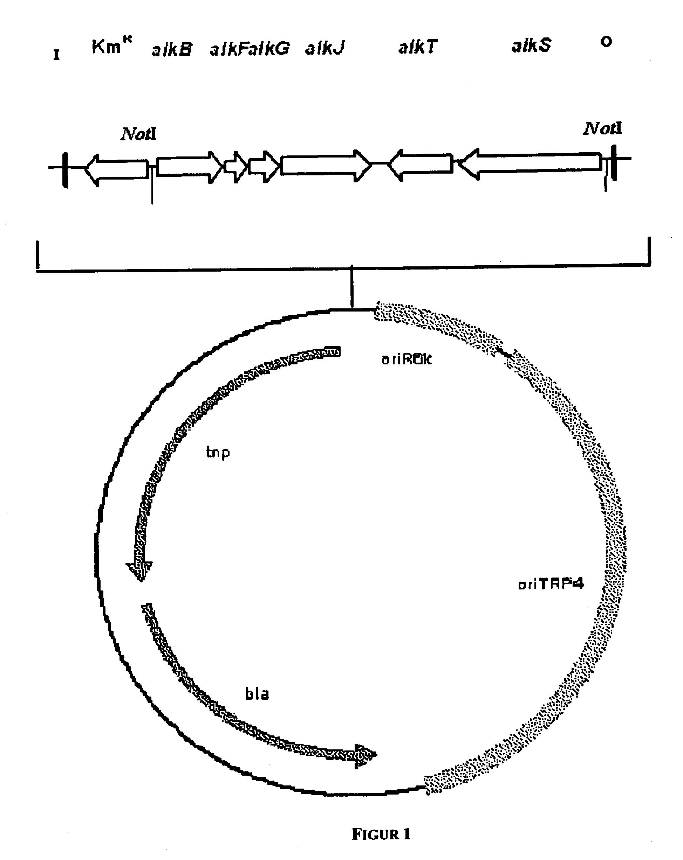

Es zeigt die Figur 1 die schematische Darstellung eines rekombinantes Plasmids für die chromosomale Integration der *alk*-Gene in *Escherichia coli* (tnp: Transposasegen; bla: Ampicillinresistenzgen, oriT RP4: Mobilisierungsregion; I und O markieren die *"inverted repeats"* des mini-Transposons Tn5).

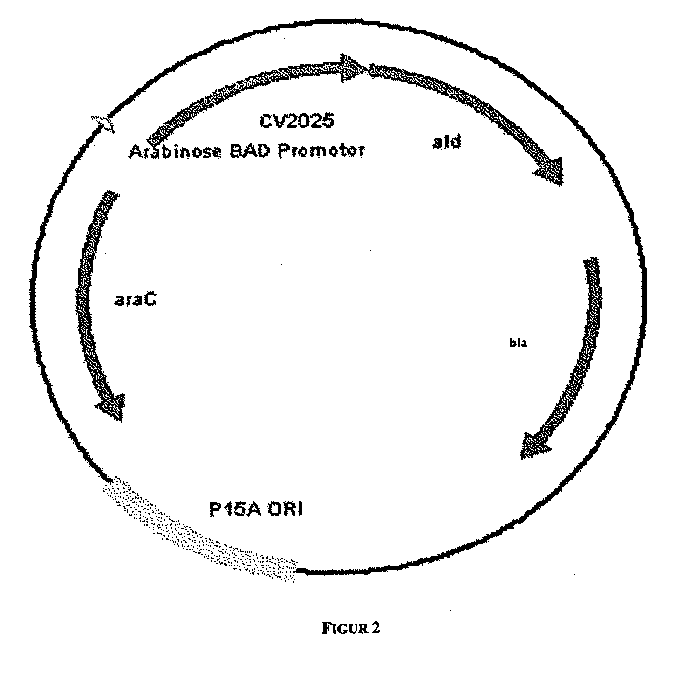

Es zeigt die Figur 2 die schematische Darstellung eines rekombinantes Plasmids für die Expression der Transaminase und der Alanin-Dehydrogenase unter der Kontrolle eines Arabinose induzierbaren Promotors (bla: Ampicillinresistenzgen; CV2025: Gen für ω-Transaminase aus *Chromobacterium violaceum;* ald: Gen für Alanin-Dehydrogenase aus *B. subtilis; araC:* Transkriptionsregulator).

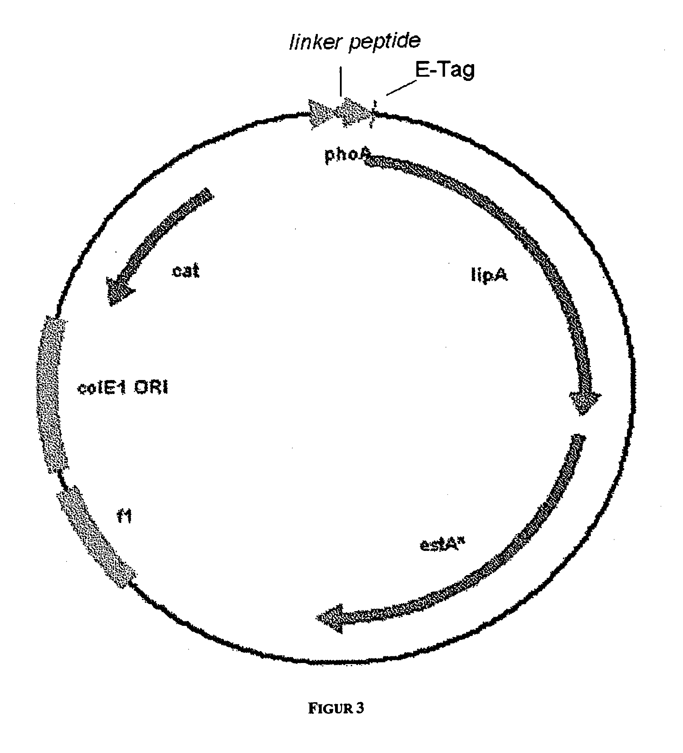

Es zeigt die Figur 3 die schematische Darstellung eines rekombinantes Plasmids für die Expression von LipA in *E. coli* und Darstellung des Enzyms auf der Zelloberfläche (colE1, ColE1: Replikationsursprung; *estA*, estA:* Gen mit Aminosäureaustausch Alanin gegen Serin (Codon #38), cat: Chloramphenicol Resistenzgen; phoA: Gensegment das für die leader-Sequenz der alkalischen Phosphatase kodiert).

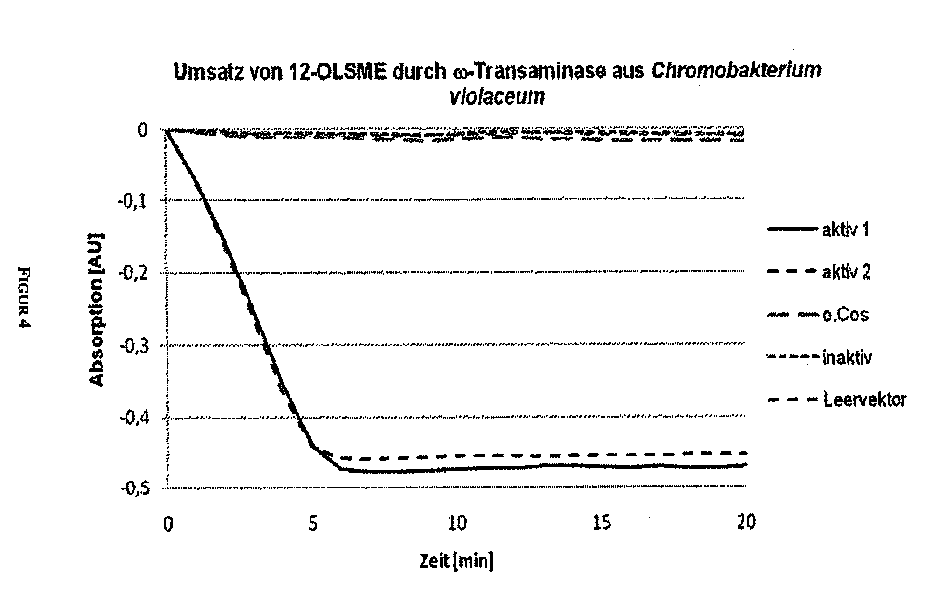

Es zeigt die Figur 4 die Aktivitätsbestimmung der C. *violaceum*-Transaminase aus dem Enzymassay. Die Bestimmung der Aktivität wurde in Doppelbestimmung (aktiv1, aktiv2) am Photometer durchgeführt. Als Negativkontrollen wurde ein Ansatz ohne das Cosubstrat L-Alanin (o.Cos), ein Ansatz mit hitzeinaktiviertem Enzym (inaktiv) und ein Ansatz aus *E. coli* Expressionskultur mit Leervektor (Leervektor), analog zur omega-TA aufgereinigt, verwendet.

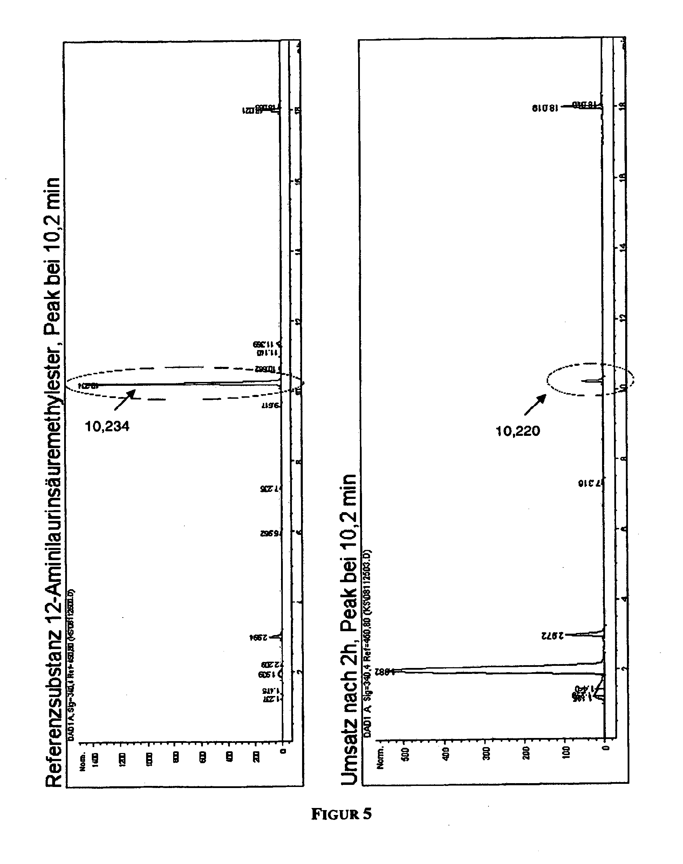

Es zeigt die Figur 5 Chromatogramme des Substrates 12-Aminolaurinsäuremethylester zu Beginn Referenzmessung (oben) und nach 2 h Inkubation mit der aufgereinigten Transaminase (unten).

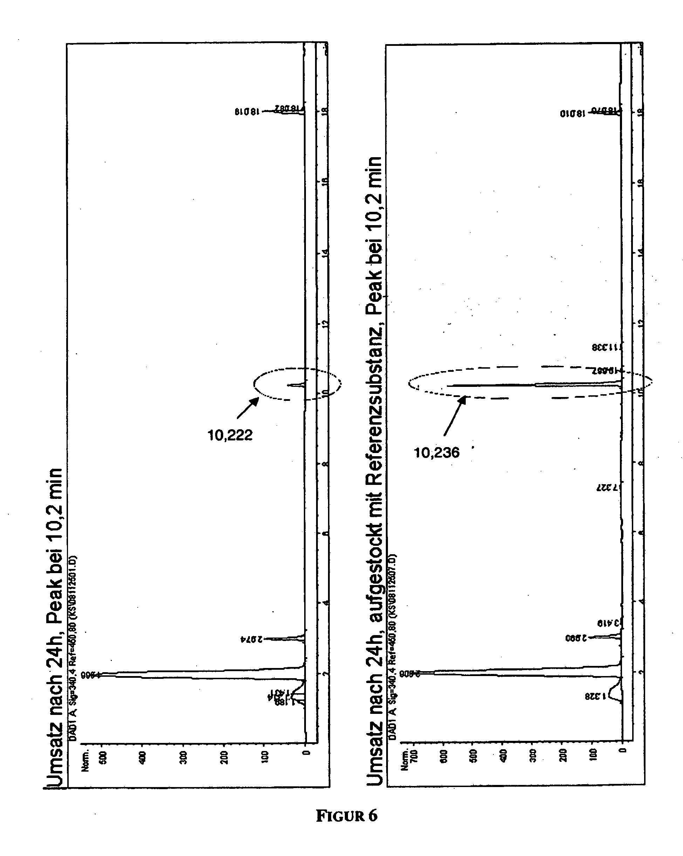

Es zeigt die Figur 6 Chromatogramme des Substrates 12-Aminolaurinsäuremethylester nach 24 h (oben) des Enzymassays und nach Aufstockung des Substrates (Kontrolle, unten).

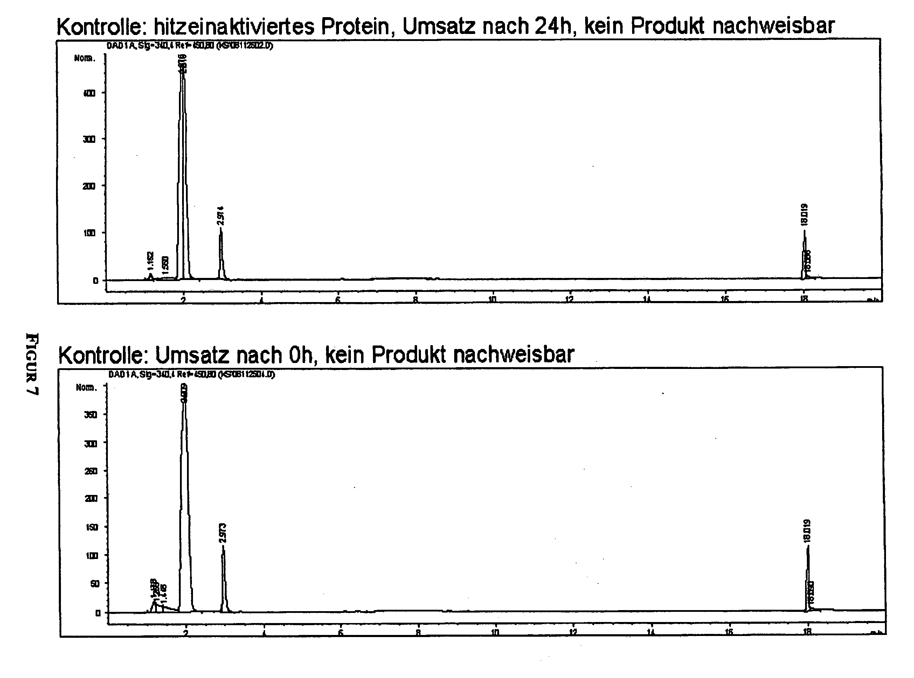

Es zeigt die Figur 7 zeigt 6 Chromatogramme des Substrates 12-Aminolaurinsäuremethylester nach Hitzeinaktivierung des Enzyms (oben) und nach 0h (unten).

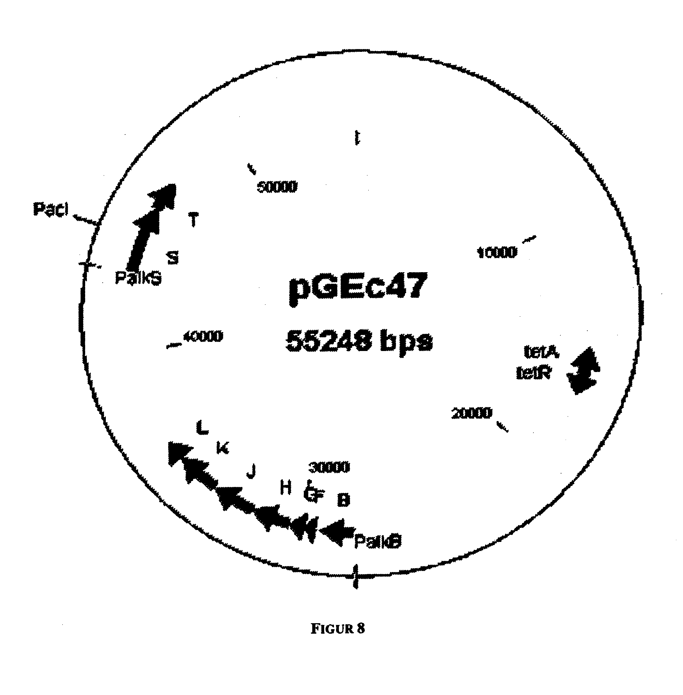

Es zeigt die Figur 8 das Ausgangsplasmid pGEc47, das als Templat für die Amplifizierung von alkBGTS eingesetzt wurde.

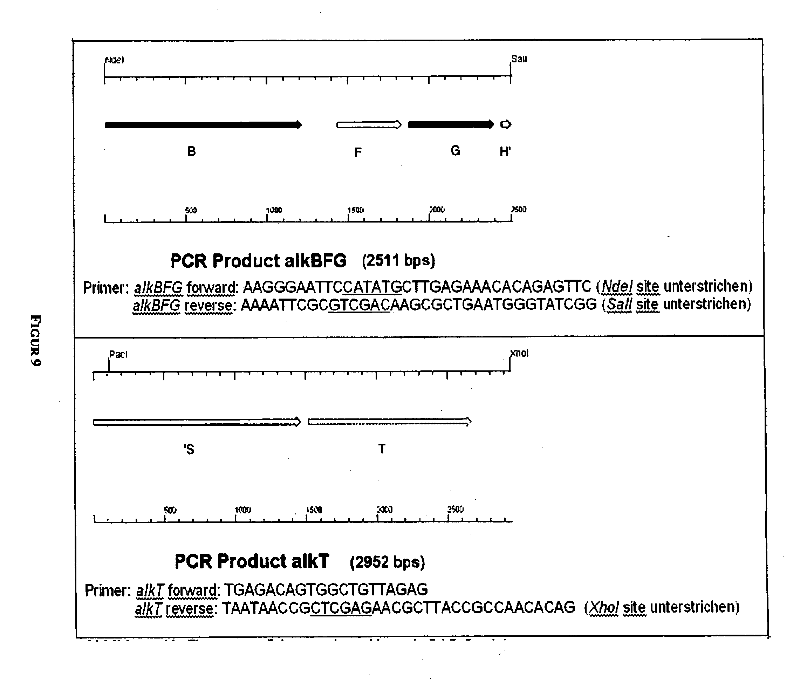

Es zeigt die Figur 9 die eingesetzten Primer und die daraus resultierenden PCR-Produkte alkBFG und alkT.

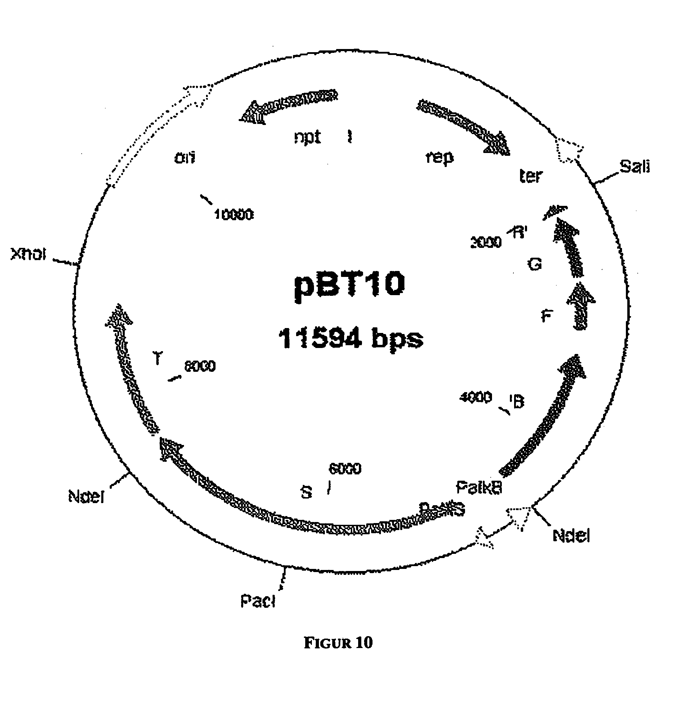

Es zeigt die Figur 10 den rekombinanten Vekor pBT10, der für die Synthese von Hyroxylaurinsäure-methylester und Oxolaurinsäure-methylester eingesetzt wurde.

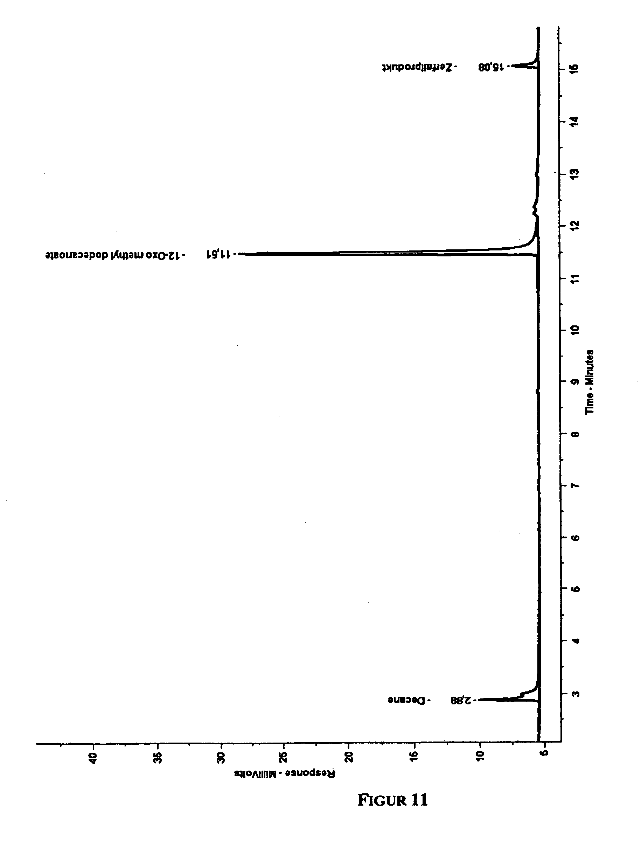

Es zeigt die Figur 11 ein GC-Chromatogramm des Standards 12-Oxo-laurinsäure-methylester.

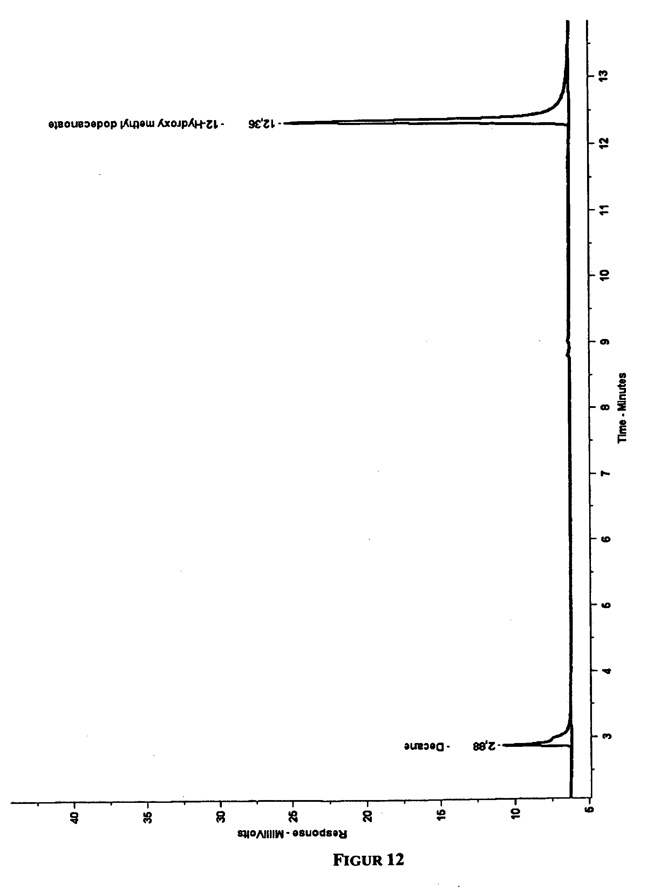

Es zeigt die Figur 12 ein GC-Chromatogramm des Standards 12-Hydroxylaurinsäuremethylester.

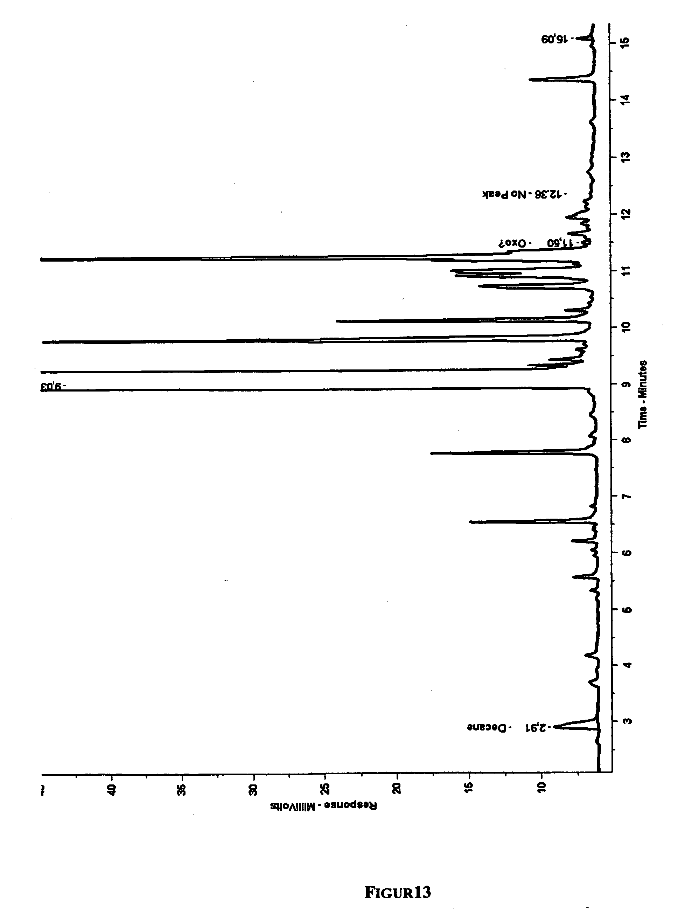

Es zeigt die Figur 13 ein Chromatogramm der organischen Phase aus einer Resting Cell Biotransformation im Bioreaktor von Laurinsäure-methylester, Zeitpunkt 0 min.

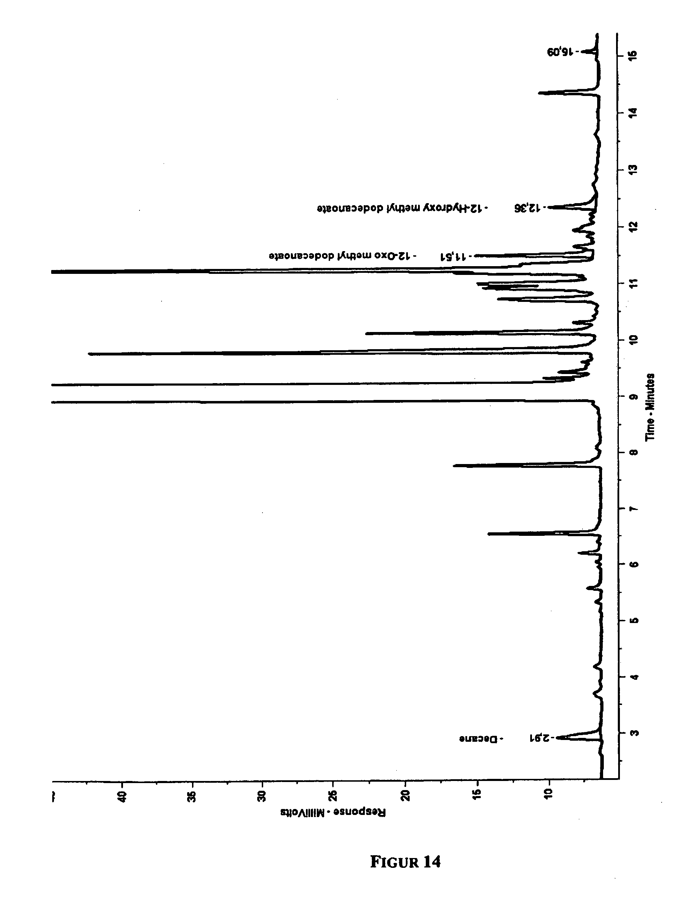

Es zeigt die Figur 14 ein Chromatogramm der organischen Phase aus einer Resting Cell Biotransformation im Bioreaktor von Laurinsäure-methylester, Zeitpunkt 150 min.

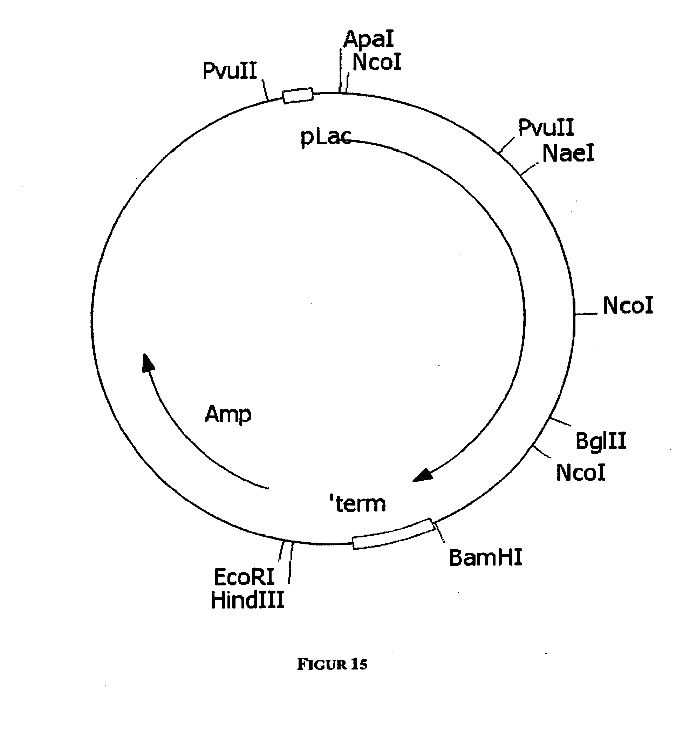

Es zeigt die Figur 15 den Expressionsvektor pGJ3130 mit AT3G22200.

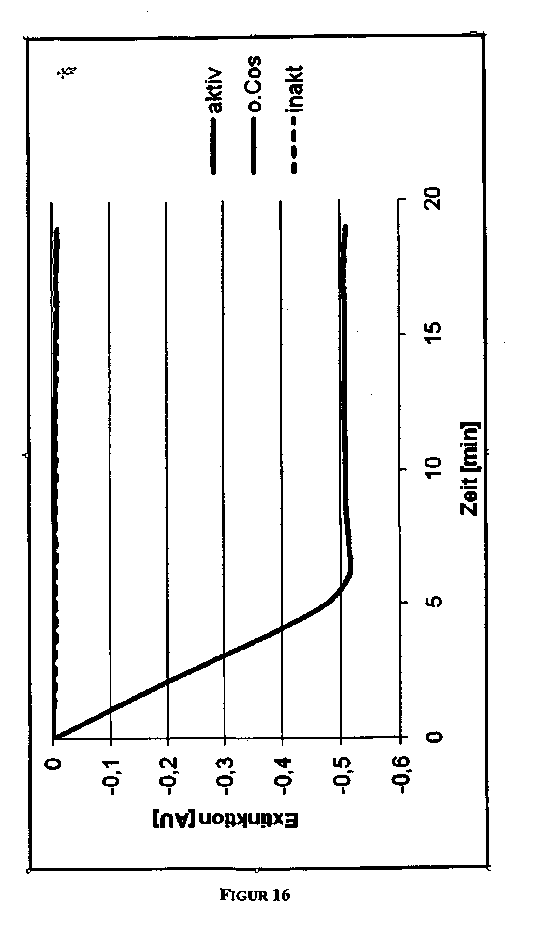

Es zeigt die Figur 16 den Nachweis der Enzymaktivität über den gekoppelten Enzymssay (inaktiv, Hitzeinaktiviertes Protein; o.Cos., ohne Zugabe von Alanin; akt, das aufgereinigte, aktive Enzym).

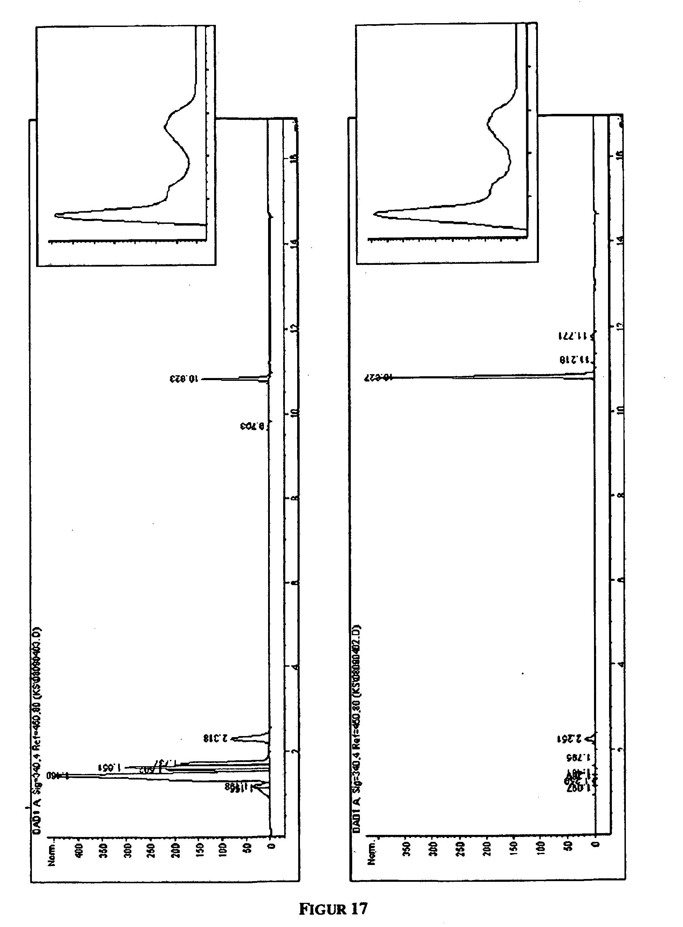

Es zeigt die Figur 17 den Nachweis des heterolog exprimierten Proteins AT3G22200 über HPLC. Oben: Produkt nach 20 min Inkubvation bei 10,8 min; unten: Referenzsubstanz bei 10,8 min.

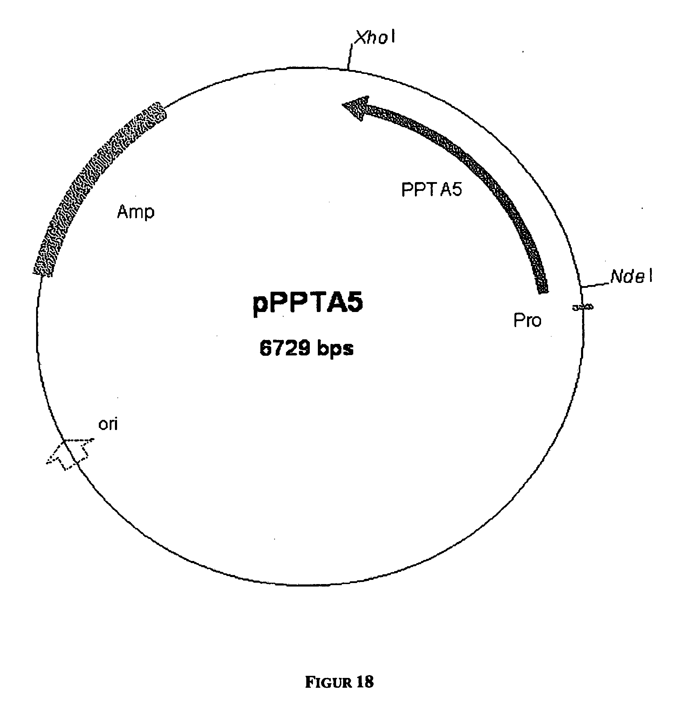

Es zeigt die Figur 18 die Plasmidkarte des Expressionsvektoren pET-21a(+) mit dem Transaminase-Genen ppta5 (pPPTA5).

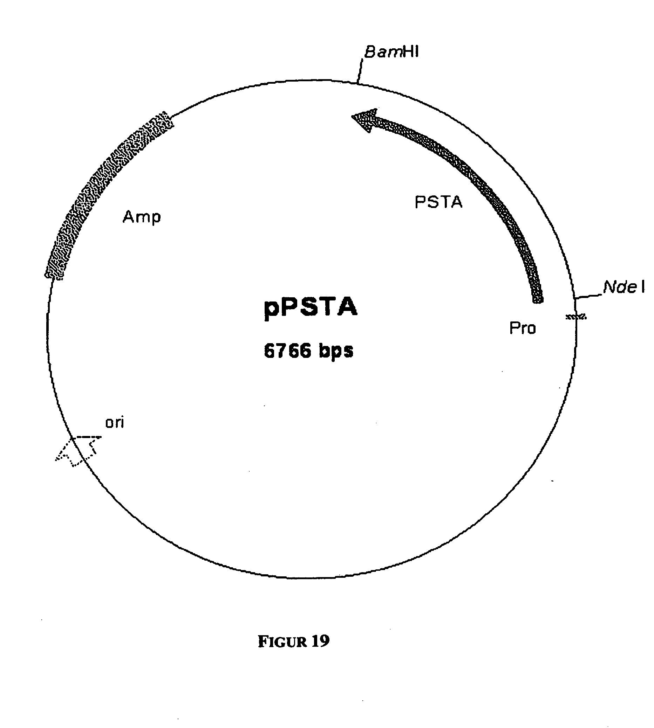

Es zeigt die Figur 19 die Plasmidkarte der Expressionsvektoren pET-21 a(+) mit den Transaminase-Gen psta (pPSTA).

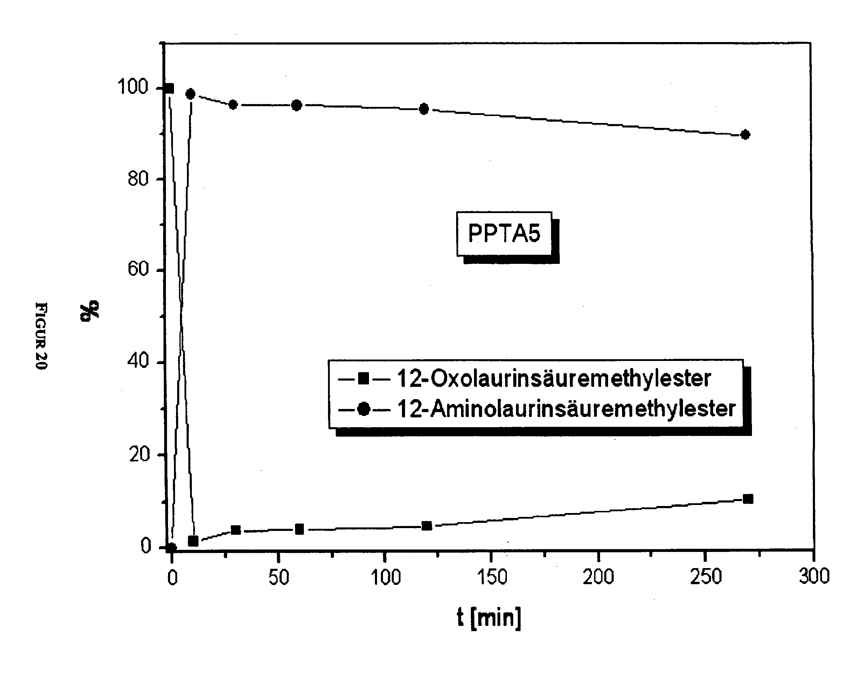

Es zeigt die Figur 20 die Aminierung von 5 mM 12-Oxolaurinsäuremethylester mit den Transaminasen PPTA5. Für die Auswertung wurden die Peakflächen von 12-Oxo- und 12-Aminolaurinsäuremethylester aus den erhaltenen Chromatogrammen der neutralen und sauren Extraktion summiert und der prozentuale Anteil von Edukt bzw. Produkt berechnet.

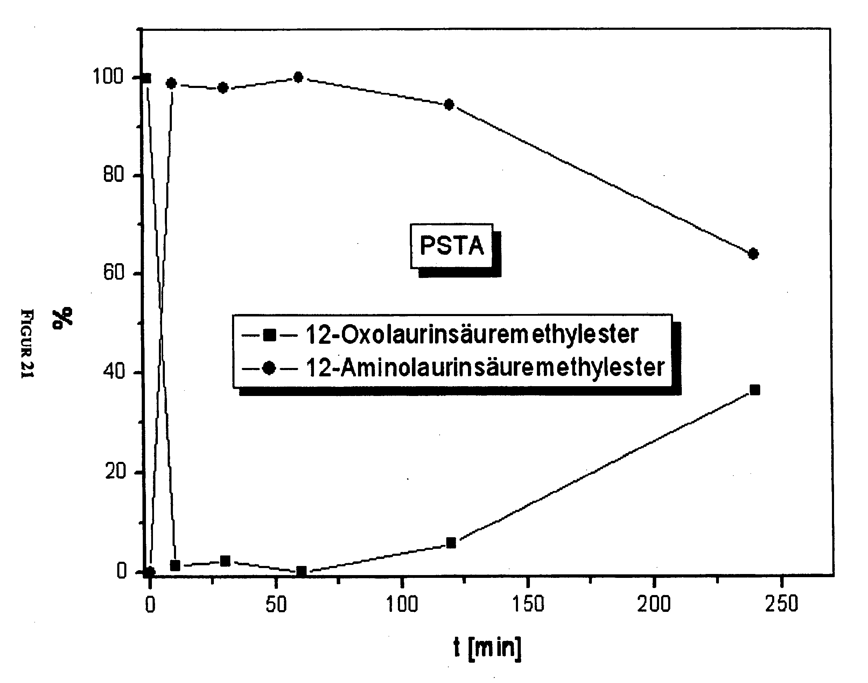

Es zeigt die Figur 21 die Aminierung von 5 mM 12-Oxolaurinsäuremethylester mit den Transaminasen PSTA. Für die Auswertung wurden die Peakflächen von 12-Oxo- und 12-Aminolaurinsäuremethylester aus den erhaltenen Chromatogrammen der neutralen und sauren Extraktion summiert und der prozentuale Anteil von Edukt bzw. Produkt berechnet.

### BEISPIELE

### A. Umsetzung von Laurinsäure bzw. Laurinsäuremethylester zu Laurinlactam

Für die Umsetzung der Laurinsäure bzw. des Laurinsäuremethylesters zum Laurinlactam wird *E*. *coli* mit den erforderlichen Enzymen Monooxygenase, Alkohol-Dehydrogenase, ω-Transaminase, Alanin-Dehydrogenase und einer Lipase komplementiert. Die Enzyme werden in *E. coli* überexpremiert, wobei die Expressionslevel der einzelnen Enzyme von der Kinetik der Einzelreaktionen abhängig sind und optimal aufeinander abgestimmt werden müssen. Die Einstellung des Expressionslevels erfolgt durch die Zugabe der entsprechenden Induktormenge. Die Expression der Monooxygenase und der Alkohol-Dehydrogenase wird mit n-Oktan induziert, die Transaminase und die Alanin-Dehydrogenase werden mit Arabinose und die Lipase mit IPTG induziert.

### Al. Klonierung der einzelnen Enzyme

### Hydroxylierung und Aldehydbildung

Für die Hydroxylierung der Laurinsäure bzw. des Laurinsäuremethylesters wird das Alkanhydroxylase-System *alkBGT* aus *Pseudomonas putida* GPo1 verwendet. Der zweite Schritt zum Aldehyd wird durch die Alkohol-Dehydogenase *alk*J katalysiert.

Die für diese Reaktionen erforderlichen Gene *alkBGJT* werden in *E. coli* durch Insertion in das Mini-Transposon Tn5 chromosomal in das Genom von *E. coli* integriert (de Lorenzo et al., JBacteriol., Vol. 172 (11), Seiten 6568-6572 und 6557-6567 (1990); Panke et al., Appl and Environm. Microbiol., Seiten 5619-5623 (1999)). Dabei sollen die Gene unter der Kontrolle des *alkB*-Promotors und des positiven Regulators *alkS* exprimiert werden. Die Übertragung des Tn5-*alk*BGFJST-Konstrukts auf den *E. coli*-Zielorganismus erfolgt mit Hilfe des mobilisierbaren Plasmids pUT-Km Panke et al., 1999 s. o.).

Der *alkST* Locus mit dem für die Expression relevanten Regulator *alk*S ist außerdehalb des *alkBFGHJKL*-Operons organisiert und im Genom von *Pseudomonas putida* in entgegengesetzter Richtung angeordnet. Die Anordnung der Gene wird bei der Klonierung in das Transposon-tragende Plasmid beibehalten. Die Fragmente *alkST* und *alkBFGJ* werden nacheinander in das Plasmid integriert.

Die zu klonierenden Gene *alkB* (SEQ.-ID.-Nr. 03) *alkG* (SEQ.-ID.-Nr. 04) und *alkJ* (SEQ.-ID.-Nr. 05) sind in *P. putida* zwar gemeinsam in dem Operon *alkBFGHJKL* organisiert, werden aber von dem Gen *alkH* getrennt, das für eine Aldehyd-Dehydrogenase kodiert. Dieses Enzym würde das angestrebte Zwischenprodukt, das Aldehyd der Laurinsäure, wieder abbauen und muss daher aus der Klonierung der *alkBGJ* Gene ausgeschlossen werden.

Zur Vereinfachung der Klonierung von *alkB* und *alkG* wird das dazwischen liegende Gen *alkF* gemeinsam mit *alkB* und *alkG* amplifiziert und kloniert. AlkF ist für die zu katalysierende Reaktion ohne Bedeutung. Die Gene *alkBFG* und *alkJ* werden in zwei getrennten PCR-Ansätzen amplifiziert und über SOE-PCR miteinander fusioniert (Horton et al., Gene, Vol. 77, Seiten 61-68 (1989)). Als Ziel-DNA dient das OCT-Plasmid aus *Pseudomonas putida* GPo1.

### A2. Klonierunesstrategie:

PCR-Amplifikation des Fragments *alkST =* 4077 bp (SEQ.-ID.-Nr. 06 (*alkS*) und SEQ.-ID.-Nr. 07 (*alkT*)) mit *Not*I-Schnittstelle stromaufwärts von *alkT:*
Primer
   *alk*T-foward-*Not*I (SEQ.-ID.-Nr. 08)
   5' ACGTAGCGGCCGCCTAATCAGGTAATTTTATAC
*alk*S-reverse (SEQ.-ID.-Nr. 09)
   5' GAGCGAGCTATCTGGT

Das PCR-Fragment wird in den Transposon-tragenden Vektor pUT-Km kloniert. Dazu wird der Vektor innerhalb von *Tn5* mit *Not*I geschnitten und durch *blund-end* Ligation mit dem *alkST*-Fragment ligiert. Das rekombinante Plasmid trägt die Bezeichnung pUT-Km-*alkST.*

### A3. Synthese alkBFGJ-Konstrukts mit der SOE-PCR-Technik:

Synthese von Fragment 1: *alkBFG* + Promotor stromaufwärts *alkB* und *Not*I-Schnittstelle am 5'-Ende (Produktgröße: 1409 bp):
Primer
   *alk*BFG-foward-*Not*I (SEQ.-ID.-Nr. 10)
   5' TCGTAGCGGCCGCCCAGCAGACGACGGAGCAA
*alk*BFG-reverse-SOE (SEQ.-ID.-Nr. 11)
   5'ATTTTATCTTCTCGAGGCTTTTCCTCGTAGAGCACAT

Synthese von Fragment 3, *alkJ* mit komplementärem Ende zu *alkG* am 5'-Ende und *Not*I-Schnittstelle am 3'-Ende (Produktgröße: 1723 bp):
Primer
   *alk*J-fowward-SOE (SEQ.-ID.-Nr. 12)
   5'TGCTCTAACGAGGAAAAGCCTCGAGAAGATAAAATGTA
*alk*J-reverse-*Not*I (SEQ.-ID.-Nr. 13)
   5'ATTGACGCGGCCGCTTACATGCAGACAGCTATCA

Die zwei Einzelfragmente werden über SOE-PCR miteinander fusioniert. (3 getrennte PCR-Reaktionen erforderlich). Das rekombinante Plasmid pUT-Km-*alkST* und das *alkBFGJ*-Konstrukt werden mit *Not*I geschnitten und ligiert. Das neue rekombinante Plasmid pUT-Km-*alk*BGJST (siehe Figur 1) wird in *E. coli* HB101 transformiert und über konjugativen Plasmidtransfer auf *E. coli* JM101 übertragen.

### A4. Aminierung und Aminodonorregeneration

Für die Aminierung zum ω-Aminolaurinsäureester und die Regeneration des Aminodonors wird der Tn5*:alkBGJST* tragende *E. coli* Stamm JM101 zusätzlich mit dem rekombinanten Plasmid pBAD-CV2025-*ald* transformiert. Dieses Plasmid basiert auf dem Vektor pBAD30 (Guzman et al., "Tight Regulation, Modulation, and High-Level Expression by Vectors Containing the Arabinose PBAD Promotor", J. Bacteriol, Vol. 177 (14), Seiten 4121-4130 (1995)). pBAD-CV2025-*ald* trägt das Gen für die Transaminase CV2025 aus *Chromobacterium violaceum* DSM30191 (SEQ:-ID. Nr. 01; Kaulmann et al., Enzyme and Microbial Technology Vol. 41, Seiten 628-637 (2007) und das Gen *ald,* das für eine Alanin-Dehydrogenase aus *Bacillus subtilis subsp. Subtilis* (SEQ.-ID.-Nr. 02; NP_391071) kodiert. Die Gene stehen unter der Kontrolle eines Arabinose induzierbaren Promotors.

### A5. Klonierungsstrategie

PCR-Amplifizierung des Transaminase-Gens mit chromosomaler DNA aus *Chromobacterium violaceum* DSM30191 (Produktgröße: 1415 bp):
Primer
   CV2025-foward-*SacI*I (SEQ.-ID.-Nr. 14)
      5'CGAGGAGCTCAGGAGGATCCAAGCATGCAGAAGCAACGTACG
   CV2025-reverse-*Kpn*I (SEQ.-ID.-Nr. 15)
      5'GTCATGTACCCCTAAGCCAGCCCGCGCGCCT

Für die Klonierung in den Vektor pBAD30 enthält der *foward-*Primer neben der *Xba*I-Schnittstelle eine Ribosomenbindestelle. Die Ligation in den Vector pBAD30 erfolgt über die Schnittstellen *Sac*I und *Kpn*I*.* Der rekombinante Vektor trägt die Bezeichnung pBAD-CV2025.

PCR-Amplifizierung des Alanin-Dehydrogenase-Gens *ald* mit chromosomaler DNA aus *B. subtilis subsp. Subtilis* (NP_391071) (Produktgröße: 1171 bp).
Primer
   *Ala*DH-foward-XbaI (SEQ.-ID.-Nr. 16)
      5' ACCTATCTAGAAGGAGGACGCATATGATCATAGGGGTTCCT
   *Ala*DH-reverse-PstI (SEQ.-ID.-Nr. 17)
      5'AACCTCTGCAGTTAAGCACCCGCCAC

Für die Klonierung in den rekombinanten Vektor pBAD-CV2025 enthält der *foward-*Primer neben der *Xba*I-Schnittstelle eine Ribosomenbindestelle. Die Klonierung in den Vektor erfolgt über die Schnittstellen *Xba*I und *Pst*I*.* Das resultierende Plasmid trägt die Bezeichnung pBAD-CV2025-*ald* (siehe Figur 2).

### A6. Alternative Klonierung des Gens omega-Transaminase aus Chromobacterium violaceum DSM 30191 für kodonoptimierte Expression in E.coli

Das Gen kodierend für die omega-Transaminase wurde bei der Fa. Geneart für *E. coli* codon-usage optimiert synthetisiert (SEQ.-ID.-Nr. 42) und in den Vektor pGA15 (Geneart) kloniert. Dabei wurde bei der Synthese des Gens die Schnittstellen SacI und KpnI flankierend eingebaut und nach Verdau mit SacI und KpnI in den vorher mit SacI und KpnI linearisierten Vektor pGA15 kloniert. Der resultierende Vektor wurde mit den Restriktionsendonukleasen *Sac*I und *Kpn*I verdaut, das Fragment (Transaminase) aufgereinigt und in den Expressionsvektor paCYCDuet-1 (Novagen) ligiert.
Durch Restriktionsanalyse wurden die korrekten Plasmide überprüft. Der resultierende Vektor heisst paCYCDuet-1::omega Tranaminase.

### A7. Aufreinigung von heterolog exprimiertem Protein über 6xHis-Tag

Nach Transformation des Vektors (paCYCDuet-1::omega Transaminase) in *E*. *coli* XL1blue wurde der transformierte Stamm in doppeltem YT-Medium (dYT) mit dem Antibiotikum Ampicillin (100 µg/ml) bei 28 °C bis zu einer Dichte von OD600 nm=0,3-0,4 kultiviert. Die Expression erfolgt unter der Kontrolle des P*_{lac}*Promotors und wird mit IPTG (Endkonzentration 1 mM) induziert. Lyse der Bakterienexpressionskultur: 50 ml Kultur wurden bei 2360 x g, abzentrifugiert und anschließend in 5 ml Na-Phosphatpuffer (pH 8) mit 5 mM EDTA, 300 mM NaCl und 1 mg/ml Lysozym resuspendiert, 1h bei RT inkubiert. Das Lysat wurde bei 2360 x g für 10 min zentrifugiert und der Überstand wurde über eine Protino Ni-TED 2000 Packed Columns (nach Angaben des Herstellers;
Macherey-Nagel, Düren) aufgereinigt. Die Proteinkonzentration wurde mittels Bradford bestimmt

### A8. Nachweis der Enzymaktivität über einen gekoppelten Assay

Die Aktivität wurde in einem gekoppelten Assay bestimmt, bei dem das als Nebenprodukt der Transaminasereaktion anfallende Pyruvat in einem zweiten Schritt weiter umgesetzt wird, wobei NADH zu NAD+ oxidiert wird. Die Abnahme der NADH-Konzentration (Prinzip: Messung des Extinktionsabfalls) wird im Photometer bei 340 nm gemessen und dient als Maß für die Aktivität.

| Ansatz | |
|---|---|
| 50 mM | Na-Phosphat pH 7,5 |
| 50 mM | L-Alanin |
| 100 µM | Pyridoxalphosphat |
| 250 µg | 12-Oxododecansäuremethylester |
| 1,25 mM | NADH |
| 10 U | Laktatdehydrogenase |
| 10 µg | heterolog exprimiertes Protein |
| Mit H₂O_{bidest} | auf 1 ml auffüllen |

Der Assay wurde durch Zugabe von 5 µl 12-ODME (50 mg/ml) gestartet. Die Messung erfolgt kontinuierlich jede Minute bei 340 nm bei RT bis max. 20 Minuten.
Zur Kontrolle wurde inaktiviertes Protein und ein Ansatz ohne Co-Substrat verwendet. In Figur 4 konnte die Extinktionsänderung photometrisch verfolgt werden

### A9. Nachweis des heterolog exprimierten Proteins über HPLC

| Ansatz | |
|---|---|
| 50 mM | Na-Phosphat pH 7,5 |
| 50 mM | L-Alanin |
| 100 µM | Pyridoxalphosphat |
| 250 µg | 12-Oxododecansäuremethylester |
| 50 µg | heterolog exprimiertes Protein |
| Mit H₂O_{bidest} | auf 500 µl auffüllen |

Nach Inkubation 4h bei RT wurde die Reaktion mit 1 Vol. MeOH abgestoppt
Für die HPLC-Analytik wurde der Ansatz mit o-Phthaldialdehyd (oPA) derivatisiert und davon 250 µl analysiert. Als Laufmittel A wurde 50 mM NaAC pH 4:Acetonitril 4:1 (v:v) verwendet.
Laufmittel B war Acetonitril mit 5 % 50 mM NaAC pH 4. Der Gradient verlief von 30 % B zu 60 % B in 4 min, von 60% B zu 100% B in 2 min. Flussrate betrug 1,2 ml/min.
Auftrennung erfolgte über eine Agilent Zorbax RP18 Säule (Agilent Technologies, USA), Die Säulentemperatur betrug 40 °C.
Figuren 5 und 6 zeigen den Standard sowie die Abnahme des 12-Oxolaurinsäuremethylester. Als Negativkontrolle dient hitzeinaktiviertes Enzym (Figur 7).

### A6. Esterspaltung

Für die Spaltung des ω-Aminolaurinsäuremethylesters zur ω-Aminolaurin-säure wird die Lipase LipA (Q76D26) aus *Pseudomonas fluoreszenz* (Kojima & Shimizu, J. of Bioscience and Bioengin., Vol. 96 (3), Seiten 219-226 (2003)) eingesetzt. Das Gen wird mit den Primern LipA-SfiI-up und LipA-SfiI-down mit chromosomaler DNA von *Pseudomonas fluoreszenz* amplifiziert und über die *Sfi*I-Schnittstellen in den Vektor pEST100 kloniert. Das rekombinante Plasmid trägt die Bezeichnung *pEST-lipA* (siehe Figur 3).

Durch die Klonierung wird *lipA* (SEQ.-ID.-Nr. 18) an die Signalsequenz der alkalischen Phosphatase *phoA* und die Autotransporter Domäne von EstA, einer Esterase aus P. *aeruginosa* fusioniert, so dass die Lipase über die Cytoplasmamembran transferiert und auf der Zelloberfläche von *E. coli* dargestellt wird. Zur Vorgehensweise siehe Becker et al, "A generic system for the Escherichia coli cell-surface display of lipolytic enzymes", FEBS Letters Vol. 579, Seiten 1177-1182 (2005). Die Expression erfolgt unter der Kontrolle des P_{/ac} Promotors und wird mit IPTG (Endkonzentration 1 mM) induziert (Produktgröße: 1894 bp).
Primer
   Primer *lip*A-Sfi-up (SEQ.-ID.-Nr. 19)
      5' AACAA.AAGGGCCGCAATGGCCATGGGTGTGTATGACTAC
   Primer *lip*A-Sfi-down (SEQ.-ID.-Nr. 20)
      5'TACAGGGGCCACCACGGCCTCAGGCGATCACAATTCC

### B Synthese von 12-Hydroxylaurinsäure-methylester und 12-Oxolaurinäure-methylester ausgehend von Laurinsäure-methylester mit dem AlkBGT Alkanhydroxylase-System aus Pseudomonas putida GPo1

### B1. Konstruktion der alkBGT Expressionsvektoren

### Ausgehend von den pCOM

Ausgehend von den pCOM Systemen (Smits et al., 2001 Plasmid 64:16-24) wurde das Konstrukt pBT10 (Figur 10, SEQ.-ID.-Nr.31) hergestellt. das die für die Oxidation zum Aldehyd notwendigen drei Komponenten Alkan-Hydroxylase (AlkB), Rubredoxin (AlkG) und Rubredoxin-Reduktase (AlkT) aus Pseudomonas putida enthält. Für die Expression der drei Gene wurde die alkBFG Gensequenz unter die Kontrolle des alkB-Promotors gestellt und das alkT-Gen unter die des alkS-Promotors.

### B2. Klonierungsstrategie:

Zur Vereinfachung der Klonierung von *alkB* und *alkG* wurde das dazwischen liegende Gen *alkF* gemeinsam mit *alkB* und *alkG* amplifiziert und kloniert. AlkF ist für die zu katalysierende Reaktion ohne Bedeutung.
PCR-Amplifikation des Fragments *alkBFG* = 2524 bp (vgl. SEQ.-ID.-Nr. 03 (*alkB*) und SEQ.-ID.-Nr. 04 (alkG)) mit *Nde*I-Schnittstelle stromaufwärts von *alkB* und *SalI-*Schnittstelle stromabwärts von *alkG*:
Primer: alkBFG forward (SEQ.-ID.-Nr. 32)
   AAGGGAATTCCATATGCTTGAGAAACACAGAGTTC
Primer: alkBFG reverse (SEQ.-ID.-Nr. 33)
   AAAATTCGCGTCGACAAGCGCTGAATGGGTATCGG

PCR-Amplifikation des Fragments *alkT* (2958 bp) (vgl. Seq.ID.-Nr. 07 (*alkT*))
Primer alkT forward (SEQ.-ID.-Nr. 34)
   TGAGACAGTGGCTGTTAGAG
Primer alkT reverse (SEQ.-ID.-Nr. 35)
   TAATAACCGCTCGAGAACGCTTACCGCCAACACAG

Die Amplifikation der Fragmente alkBFG und alkT erfolgte mittels PCR. Als Template wurde das Plasmid pGEc47 (Figur 12) (Eggink et al. (1987) J. Biol. Chem. 262, 17712-17718) eingesetzt.
Die Klonierungen wurden mittels Subklonierungsvektor pSMART® HCKan (Lucigen Corporation) durchgeführt. Dieser zusätzliche Schritt war notwendig, da eine direkte Klonierung zu keinem Erfolg geführt hatte. Dazu wurde der kommerziell erhältliche und schon linerisiert und mit blunt-end versehende Vektor pSMART® HCKan (Lucigen) mit dem jeweiligen Blunt-End PCR-Produkt (Figur 9) ligiert.
Im Anschluss wurden das alkBFG-Fragment mit den Restriktionsenzymen NdeI und SalI und das alkT-Fragment mit den Restriktionsenzymen PacI und XhoI aus den Subklonierungsvektoren heraus geschnitten. Die Fragmente wurden im Agarosegel (1 %) aufgetrennt, aus dem Gel ausgeschnitten und mittels Gelextraktionskit isoliert.
Die Fragmente wurden nacheinander in den Vektor pCOM10 (Smits, T. H. M., Seeger, M. A., Witholt, B. & van Beilen, J. B. (2001) Plasmid 46, 16-24) ligiert. Im ersten Schritt wurde alkBFG über die Schnittstellen NdeI und SalI in den pCOM10 eingebracht, in einem zweiten Schritt wurde dann alkT über die Schnittstellen PacI und XhoI kloniert.

Das rekombinante Plasmid wurde zunächst in E. coli DH5α transformiert. Plasmidtragende Klone wurden auf Kanamycin-haltigem LB-Medium selektioniert. Das daraus isolierte Plasmid wurde über Restriktionsanalyse und Sequenzierung kontrolliert. Es trägt die Bezeichnung pBT10 (Figur 10).

### B3. Biotransformation im Bioreaktor von Laurinsäure-methylester zu Hydroxylaurinsäure-methylester und 12-Oxolaurinsäure-methylester

Für die Biotransforamtion wurde das Plasmid pBT10 durch Hitzschock bei 42°C für 2 min in den chemisch kompetenten Stamm E. coli W3110 transformiert. Für die Synthese von Hydroxylaurinsäure-methylester und 12-Oxolaurinsäure-methylester wurde E. coli W3110-pBT10 über Nacht bei 30°C und 180 rpm in M9 Medium kultiviert und abgeerntet. Die Biomasse wurde in M9 Medium mit 0,5 % Glucose bis zu einer OD450 = 0,2 aufgenommen. Die Expression wurde nach einer Wachstumszeit von 4 h mit Dicyclopropylketon induziert und für 4 weitere Stunden inkubiert. Die Zellen wurden erneut abzentrifugiert, das Zellpellet in KPi-Puffer (50 mM, pH 7,4) resuspendiert und in einen Bioreaktor gegeben. Es wurde eine Biomassekonzentration von etwa 1,8 gCDW/L eingestellt. Unter kräftigem Rühren (1500 min-1) wurde das Substrat Laurinsäuremethylester im Verhältnis 1:3 zur Zellsuspension zugesetzt (100 ml Zellsuspension, 50 ml Laurinsäure-methylester). Die Temperatur wurde bei 30°C konstant gehalten.
Die Bildung der Produkte Hydroxylaurinsäure-methylester und 12-Oxolaurinsäuremethylester wurde durch GC-Analyse des Reaktionsansatzes nachgewiesen. Dazu wurde nach 0 min als Negativkontrolle (Figur 13) und nach 150 min (Figur 14) aus der organischen Phase des Reaktionsansatzes eine Probe genommen und mittel GC (Thermo Trace GC Ultra) analysiert. Als Säule diente eine Varian Inc. FactorFourTM VF-5m, Länge: 30 m, Filmdicke: 0,25 µm, Innendurchmesser: 0,25 mm.
Analysebedingungen:

| | |
|---|---|
| Ofentemperatur | 80 - 280 °C |
| Rampe | 15°C/min |
| Splitratio | 15 |
| Injektionsvolumen | 1 µl |
| Carrierflow | 1,5 ml/min |
| PTV Injektor | 80 - 280°C bei 15°C/s |
| Detektorbasistemperatur: | 320 °C |

Die Detektion von 12-Oxolaurinsäuremethylester (Figur 11) und 12-Hydroxylaurinsäuremethylester (Figur 12) wurde durch Injektion der Reinsubstanzenm gezeigt.

### C Umsetzung von 12-Oxolaurinsäure-methylester zu 12-Aminolaurinsäuremethylester

### C 1: Isolierung und Expression einerAminotransferase aus Arabidopsis thaliana

Es wurde eine bekannte Aminotransferase aus Arabidopsis thaliana analysiert. Dabei zeigte überraschenderweise die 4-Aminobutyrat-Transaminase (at3g22200, SEQ.-ID.-Nr. 38) eine Aktivität von etwa 14 U/mg heterolog exprimiertem Protein gegenüber 12-Oxododekansäuremethylester. Das Produkt 12-Aminododekansäuremethylester wurde mittels HPLC bestätigt.

Wenn nicht anders angegeben wurden alle Methoden ausgeführt nach den Protokollen in Sambrook, J., Fritsch, E. F., & Maniatis, T (1989). Molecular cloning, 2nd edn. New York: Cold Spring Harbor Laboratory Press.

### Isolierung der 4-Aminobutyrattransaminase aus A.thaliana (at3g22200)

Die RNA wurde mit dem RNeasy Mini Kit aus einer ganzen, blühenden Pflanze der Art A. thaliana nach Angaben des Herstellers: QIAGEN GmbH, Hilden, isoliert. Anschließend wurde die cDNA-Synthese mit dem RT Skript Kit (USB Europe GmbH, Staufen) nach Angaben des Herstellers durchgeführt. Die RNA-Qualität/Quantität Bestimmung wurde mittels Nanodrop nach Angaben des Herstellers (Thermo Fisher Scientific Inc. Waltham USA) bestimmt.

### PCR aus A. thaliana cDNA zur Einfügung von Schnittstellen

Mit folgenden Primern wurde die DNA kodierend für die 4-Aminobutyrattransaminase mit der SEQ.-ID.-Nr. 39 in den NaeI, BamHI verdauten Vektor kloniert
Forward-Primer führt Protease-Schnittstelle und NaeI ein, SEQ.-ID.-Nr. 36
   GCCGGCGAGAACCTGTACTTTCAGATGGCAAGTAAGTATGCCACTTG
Reverse-Primer führt BamHI ein, SEQ.-ID.-Nr. 37
   GGATCCTCACTTCTTGTGCTGAGCCTTG
Die PCR wurde nach folgendem Protokoll durchgeführt:

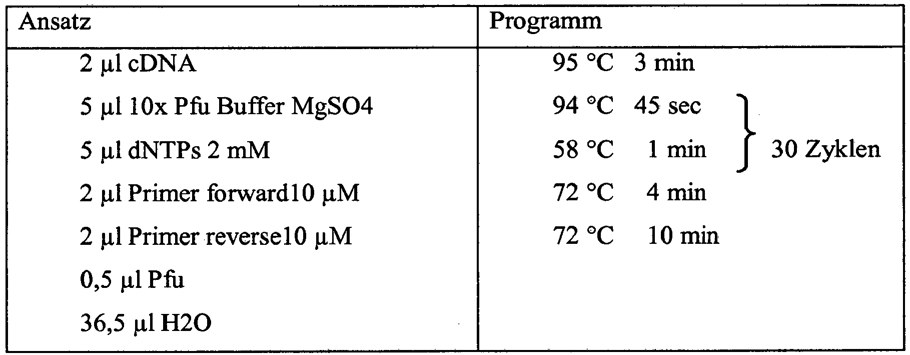

Das resultierende PCR Produkt wurde mit dem NucleoSpin® Extract II Kit (Macherey-Nagel, Deutschland, nach Angaben des Herstellers) aufgereinigt.

### Expression des heterologen Proteins

Unter Verwendung des oben beschriebenen PCR-Produktes wurde der Vektor pGJ3130 (Figur 15, SEQ.-ID.-Nr. 43) durch Standard molekularbiologische Methoden hergestellt und in *E.coli* XLlblue transformiert. Der transformierte *E. coli* Stamm wurde in doppeltem YT-Medium (dYT) mit dem Antibiotika Ampicillin (100 µg/ml) und Zugabe von 0,5 mM IPTG bei 28 °C bis zu einer Dichte von OD600 nm=0,3-0,4 kultiviert.

### Aufreinigung von heterolog exprimiertem Protein über 6xHis-Tag

Lyse der Bakterienexpressionskultur: 50 ml Kultur wurden bei 2360 x g, abzentrifugiert und anschließend in 5 ml Na-Phosphatpuffer (pH 8) mit 5 mM EDTA, 300 mM NaCl und 1 mg/ml Lysozym resuspendiert, 1h bei RT inkubiert. Das Lysat wurde bei 2360 x g für 10 min zentrifugiert und der Überstand wurde über eine Protino Ni-TED 2000 Packed Columns (nach Angaben des Herstellers; Macherey-Nagel, Düren) aufgereinigt. Die Proteinkonzentration wurde mittels Bradford bestimmt

### Nachweis der Enzymaktivität über einen gekoppelten Assay

Die Aktivität wurde in einem gekoppelten Assay bestimmt, bei dem das als Nebenprodukt der Transaminasereaktion anfallende Pyruvat in einem zweiten Schritt weiter umgesetzt wird, wobei NADH zu NAD+ oxidiert wird. Die Abnahme der NADH-Konzentration (Prinzip: Messung des Extinktionsabfalls) wird im Photometer bei 340 nm gemessen und dient als Maß für die Aktivität.

| Ansatz | |
|---|---|
| 50 mM | Na-Phosphat pH 7,5 |
| 50 mM | L-Alanin |
| 100 µM | Pyridoxalphosphat |
| 250 µg | 12-Oxododecansäuremethylester |
| 1,25 mM | NADH |
| 10 U | Laktatdehydrogenase |
| 10 µg | heterolog exprimiertes Protein |
| Mit H₂O_{bidest} | auf 1 ml ausfüllen |

Der Assay wurde durch Zugabe von 5 µl 12-ODME (50 mg/ml) gestartet. Die Messung erfolgt kontinuierlich jede Minute bei 340 nm bei RT bis max. 20 Minuten.
Zur Kontrolle wurde inaktiviertes Protein und ein Ansatz ohne Co-Substrat verwendet.
In Figur 16 konnte die Extinktionsänderung photometrisch verfolgt werden

Nachweis des heterolog exprimierten Proteins über HPLC

| Ansatz | |
|---|---|
| 50 mM | Na-Phosphat pH 7,5 |
| 50 mM | L-Alanin |
| 100 µm | Pyridoxalphosphat |
| 250 µg | 12-Oxododecansäuremethylester |
| 50 µg | heterolog exprimiertes Protein |
| Mit H₂O_{bidest} | auf 500 µl auffüllen |

Nach Inkubation 4h bei RT wurde die Reaktion mit 1 Vol. MeOH abgestoppt
Für die HPLC-Analytik wurde der Ansatz mit o-Phthaldialdehyd (oPA) derivatisiert und davon 250 µl analysiert. Als Laufmittel A wurde 50 mM NaAC pH 4:Acetonitril 4:1 (v:v) verwendet.
Laufmittel B war Acetonitril mit 5 % 50 mM NaAC pH 4. Der Gradient verlief von 30 % B zu 60 % B in 4 min, von 60% B zu 100% B in 2 min. Flussrate betrug 1,2 ml/min. Auftrennung erfolgte über eine Agilent Zorbax RP18 Säule (Agilent Technologies, USA), Die Säulentemperatur betrug 40 °C. Figur 17 (oben) zeigt die Bildung von 12-Aminolaurinsäuremethylester. Die Referenzprobe ist unten in Figur 17 dargestellt.

### D Aminierung von 12-Oxolaurinsäuremethylester mit PPTA5 und PSTA aus Pseudomonas

### D1. Klonierung von PPTA5 und PSTA

Für die Aminierung von 12-Oxolaurinsäuremethylester wurden die Stämme *E. coli* BL21(DE3)/PPTA5 und E. coli BL21(DE3)/PSTA eingesetzt. Diese Stämme wurden wie folgt konstruiert. Zur Klonierung beider Transaminase-Gene wurde der Expressionsvektor pET-21a(+) (Novagen) gewählt. Für das ppta5-Gen, SEQ.-ID.-Nr. 40, wurden Primer konstruiert, die dem Gen die Restriktionsschnittstellen NdeI und XhoI an den Enden anfügen sollten; Primer PPTA5_NdeI:
GGAATTCCATATGAGCGTCAACAACCCGCAAACCCG (SEQ.-ID.-Nr. 44) und Primer PPTA5_XhoI: CCGCTCGAGTTATCGAATCGCCTCAAGGGTCAGGTCC (SEQ.-ID.-Nr. 45). Für das psta-Gen, SEQ.-ID.-Nr. 41, Primermit NdeI und BamHI an den Enden; Primer PSTA_NdeI:
GGAATTCCATATGAGCGATTCGCAAACCCTGCACTGGC (SEQ.-ID.-Nr. 46) und Primer PSTA_BamHI: CGCGGATCCTCAGCCCAGCACATCCTTGGCTGTCG (SEQ.-ID.-Nr. 47)

Diese Primer wurden in PCRs eingesetzt. Die aufgereinigten PCR-Produkte sowie der Vektor pET-21a(+) wurden anschließend einer Restriktion mit den Restriktionsenzymen NdeI und XhoI bzw. NdeI und BamHI unterzogen. Der geschnittene Vektor wurde mit Alkalischer Phosphatase aus Shrimps dephosphoryliert. Mit NdeI und XhoI geschnittener Vektor und ppta5-Gen sowie mit NdeI und BamHI geschnittener Vektor und psta-Gen wurden nach Ligation mit T4 DNA Ligase mit dem kompetenten Expressionsstamm E. *coli* XL1-Blue transformiert. Nach Anzucht einiger Klone erfolgte die Isolierung der Plasmide mit nachfolgender Restriktion und gelelektrophoretischer Analyse. Die Transaminase-Sequenzen der erhaltenen Klone (pPPTA5 bzw. pPSTA) wurden durch Sequenzanalyse bestätigt. Figur 18 zeigt die Plasmidkarten der Expressionsvektoren.

### D2. Expression von PPTA5 und PSTA

Für die Expression wurden die Vektoren pPPTA5 und pPSTA in kompetente *E. coli* BL21(DE3)-Zellen transformiert. Je eine Einzelkolonie wurde in 5 ml LB-Amp-Medium (Ampicillinkonzentration 100µg/ml) überimpft und über Nacht bei 37°C geschüttelt. anschließend wurde 1 %-ig in 200 ml LB-Amp-Medium überimpft, bei 37°C geschüttelt und nach Erreichen einer OD₆₀₀ von 0,5 mit 0,5 mM IPTG die Genexpression induziert. Nach 20-stündigem Schütteln bei 30°C wurden die Zellen geerntet und bei -20°C gelagert.

### Für den Aufschluss der Stämme E. coli BL21(DE3)/pPPTA5 und E. coli

BL21(DE3)/pPSTA wurden je 0,4 g Zellen mit 100 mM Tris-HCI-Puffer pH 7,2 zu 25 %- igen Zellsuspensionen verarbeitet, welche mit für zweimal 90 sec mit Ultraschall (Bandelin Sonoplus HD2070; Sonde MS73; 50 % Intensität) behandelt wurden. Nach Zentrifugation wurden die Überstände abgenommen. Die so erhaltenen Rohextrakte wurden in Umsetzungen von 12-Oxolaurinsäuremethylester eingesetzt. Die 400 µl-Ansätze enthielten 5 mM 12-Oxolaurinsäuremethylester, gelöst in N,N-Dimethylformamid, 500 mM DL-Alanin, 1 mM Pyridoxal-5'-Phosphat und 80 µl Rohextrakt in 10 mM Kpi-Puffer pH 7,0. Es wurde bei 25°C geschüttelt. Nach bestimmten Zeiten wurden je 20 µl Proben entnommen, ein Teil mit 1 µl 1 %-iger NaOH-Lösung in den alkalischen pH-Bereich gebracht und mit 100 µl Essigsäureethylester ausgeschüttelt. Die organischen Phasen wurden gaschromatographisch (Gaschromatograph Fa. Perkin Elmer, Clarus 500 mit Flammen-Ionisations-Detektor) untersucht. Hierzu wurde eine Optima 5-Säule (0,25 µm, 30 m, 0,25 mm, Macherey-Nagel) verwendet mit Programm:

| | |
|---|---|
| 80°C | |
| 25°C/min | 180°C |
| 5°C/min | 215°C |
| 20°C/min | 280°C |

Die Retentionszeiten von 12-Oxo- und 12-Aminolaurinsäuremethylester liegen bei 7,2 bzw. 7,7 min.

Die Ergebnisse der Umsetzungen sind in Figuren 20 und 21 dargestellt. Für die Auswertung wurden die Peakflächen von 12-Oxo- und 12-Aminolaurinsäuremethylester aus den erhaltenen Chromatogrammen der neutralen und sauren Extraktion summiert und der prozentuale Anteil von Edukt bzw. Produkt berechnet.

## Patentansprüche

1. Eine Zelle, welche gegenüber ihrem Wildtyp derart gentechnisch verändert worden ist, dass sie im Vergleich zu ihrem Wildtyp mehr ω-Aminocarbonsäuren, mehr ω-Aminocarbonsäureester oder mehr aus ω-Aminocarbonsäuren abgeleitete Lactame aus Carbonsäuren oder Carbonsäureestern herzustellen vermag, wobei die Zelle eine im Vergleich zu ihrem Wildtyp gesteigerte Aktivität der Enzyme E_{I} und E_{III} oder der Enzyme E_{I}, E_{II} und E_{III} aufweist:
i) eines Enzyms E_{I}, welches die Umsetzung von Carbonsäuren oder Carbonsäureestern zu den entsprechenden ω-Hydroxycarbonsäuren oder ω-Hydroxycarbonsäureestern katalysiert, ausgewählt aus der Gruppe bestehend aus den Alkanmonooxygenasen:
*alkBGT* kodierte Alkanmonoxygenase aus *Pseudomonas putida* GPol und Cytochrom-P450-Monoxygenasen aus *Candida tropicalis;*
ii) eines Enzyms E_{II}, welches die Umsetzung von ω-Hydroxycarbonsäuren oder ω-Hydroxycarbonsäureestern zu den entsprechenden ω-Oxocarbonsäuren oder ω-Oxocarbonsäureestern katalysiert, ausgewählt aus der Gruppe bestehend aus den *alk*J*-*Gen kodierten Alkoholdehydrogenasen;
ii) eines Enzyms E_{III}, welches die Umsetzung von ω-Oxocarbonsäuren oder ω-Oxocarbonsäureestern zu den entsprechenden ω-Aminocarbonsäuren oder ω-Aminocarbonsäureestern katalysiert ausgewählt aus der Gruppe der ω-Transaminasen.

2. Die Zelle nach Anspruch 1,wobei das Enzym E_{I} die von *alkBGT* kodierte Alkanmonoxygenase aus *Pseudomonas putida* GPol ist.

3. Die Zelle nach Anspruch 1 oder 2, wobei das Enzym E_{II} vom *alkJ-*Gen aus Pseudomonas *putida* GPol kodiert wird.

4. Die Zelle nach einem der Ansprüche 1 bis 3, wobei das Enzym E_{III} die ω-Transaminase CV2025 aus *Chromobacterium violaceum* DSM30191 ist.

5. Die Zelle nach einem der vorhergehenden Ansprüche, wobei in der Zelle die Expression eines Enzyms E_{IV} gesteigert ist, welches die Umsetzung von ω-Aminocarbonsäureestern zu den entsprechenden ω-Aminocarbonsäuren katalysiert.

6. Die Zelle nach Anspruch 5, wobei das Enzym E_{IV} die Lipase LipA (Q76D26) aus *Pseudomonas fluoreszenz* ist, welche von der Zelle sezerniert wird.

7. Die Zelle nach einem der vorhergehenden Ansprüche, wobei in der Zelle die Expression eines Enzyms E_{V} gesteigert ist, welches die Umsetzung von ω-Aminocarbonsäuren zu den entsprechenden Lactamen katalysiert.

8. Die Zelle nach Anspruch 7, wobei das Enzym E_{V} von der Zelle sezerniert wird.

9. Die Zelle nach einem der vorhergehenden Ansprüche, wobei die Zelle eine gentechnisch veränderte *Escherichia coli-Zelle,* eine gentechnisch veränderte *Corynebakterium glutamicum*-Zelle oder eine gentechnisch veränderte Pseudomonas *putida-*Zelle ist.

10. Ein Verfahren zur Herstellung von ω-Aminocarbonsäuren, von ω-Aminocarbonsäureestern oder von aus ω-Aminocarbonsäuren abgeleiteten Lactamen, beinhaltend die Verfahrensschritte:
I) in Kontakt bringen einer Zelle nach einem der Ansprüche 1 bis 9 mit einem Kulturmedium beinhaltend eine Carbonsäure oder einen Carbonsäureester oder mit einem Kulturmedium angrenzend an eine organische Phase beinhaltend eine Carbonsäure oder einen Carbonsäureester unter Bedingungen, die es der Zelle ermöglichen, aus der Carbonsäure oder aus dem Carbonsäureestern ω-Aminocarbonsäuren, ω-Aminocarbonsäureester oder aus ω-Aminocarbonsäuren abgeleitete Lactame zu bilden;
II) gegebenenfalls Isolierung der gebildeten ω-Aminocarbonsäuren, der gebildeten ω-Aminocarbonsäureester oder der aus ω-Aminocarbonsäuren abgeleiteten Lactame.

11. Das Verfahren nach Anspruch 10, wobei die in Verfahrensschritt I) gebildeten ω-Aminocarbonsäureester in einem weiteren Verfahrensschritt mit konventionellen chemischen Verfahren zu ω-Aminocarbonsäuren umgesetzt werden.

12. Das Verfahren nach Anspruch 10 oder 11, wobei die Zelle eine gentechnisch veränderte *Escherichia coli-*Zelle, eine gentechnisch veränderte *Corynebakterium glutamicum-*Zelle oder eine gentechnisch veränderte *Pseudomonas putida-Zelle* ist.

13. Das Verfahren nach einem der Ansprüche 10 bis 12, wobei das im Verfahrensschritt I) eingesetzte Kulturmedium Aminosäuren enthält, die als Amin-Donor bei der durch die Transaminase katalysierten Umsetzung der ω-Oxocarbonsäuren oder der ω-Oxocarbonsäureester zu den entsprechenden ω-Aminocarbonsäuren oder ω-Aminocarbonsäureestern fungieren.

14. Das Verfahren nach einem der Ansprüche 10 bis 13, wobei das Verfahren in einem Zwei-Phasen-System durchgeführt wird, beinhaltend
A) eine wässrige Phase, sowie
B) eine organische Phase,
wobei die Bildung der ω-Aminocarbonsäuren, der ω-Aminocarbonsäureester oder der aus ω-Aminocarbonsäuren abgeleiteten Lactame durch die Zellen im Verfahrensschritt I) in der wässrigen Phase erfolgt und sich die gebildeten ω-Aminocarbonsäuren, die gebildeten ω-Aminocarbonsäureester oder die gebildeten, aus ω-Aminocarbonsäuren abgeleiteten Lactame in der organischen Phase anreichern.

15. Das Verfahren nach einem der Ansprüche 10 bis 14, wobei die Isolierung der gebildeten ω-Aminocarbonsäuren, der gebildeten ω-Aminocarbonsäureester oder der aus ω-Aminocarbonsäuren abgeleiteten Lactame über ein mindestens zweistufiges Aufreinigungsverfahren erfolgt, umfassend
a) einen Extraktionsschritt, bei dem die ω-Aminocarbonsäuren, die ω-Aminocarbonsäureester oder die aus ω-Aminocarbonsäuren abgeleiteten Lactame aus dem Kulturmedium extrahiert werden, sowie
b) einen Feinreinigungsschritt, bei dem der im Verfahrensschritt a) erhaltene Extrakt über Destillationsverfahren oder weitere Extraktionsverfahren unter Erhalt einer ω-Aminocarbonsäure-Phase, einer ω-Aminocarbonsäureester-Phase oder einer Lactam-Phase mit einer Reinheit von mindestens 99,8 % weiter aufgereinigt wird.

16. Das Verfahren nach Anspruch 15, wobei es sich bei der Extraktion im Verfahrensschritt a) um eine Reaktivextraktion handelt.

17. Das Verfahren nach einem der Ansprüche 10 bis 16, wobei die Carbonsäure Laurinsäure oder der Carbonsäureester Laurinsäuremethylester ist und wobei die Laurinsäure oder der Laurinsäuremethylester im Verfahrensschritt II) zu Laurinlactam umgesetzt wird.

18. Ein Verfahren zur Herstellung von auf ω-Aminocarbonsäuren basierenden Polyamiden, umfassend die Verfahrensschritte:
(αl) Herstellung von ω-Aminocarbonsäuren durch ein Verfahren nach einem der Ansprüche 10 bis 17;
(α2) Polymerisation der ω-Aminocarbonsäure unter Erhalt eines Polyamids.

19. Ein Verfahren zur Herstellung von auf Lactamen basierenden Polyamiden, umfassend die Verfahrensschritte:
(β1) Herstellung von Lactamen durch ein Verfahren nach einem der Ansprüche 10 bis 17;
(β2) Ringöffnungspolymerisation oder Polykondensation des Lactams unter Erhalt eines Polyamids.

## Claims

1. Cell, which has been genetically modified relative to its wild type so that, in comparison with its wild type, it is able to produce more ω-aminocarboxylic acids, more ω-aminocarboxylic acid esters or more lactams derived from ω-aminocarboxylic acids, starting from carboxylic acids or carboxylic acid esters, wherein the cell has increased activity, in comparison with its wild type, of the enzymes E_{I} and E_{III} or of the enzymes E_{I}, E_{II} and E_{III}:
i) an enzyme E_{I}, which catalyses the conversion of carboxylic acids or carboxylic acid esters to the corresponding ω-hydroxycarboxylic acids or ω-hydroxycarboxylic acid esters, selected from the group consisting of the alkane monooxygenases:
*alkBGT*-encoded alkane monooxygenase from *Pseudomonas putida* GPo1 and cytochrome P450 monooxygenases from *Candida tropicalis*;
ii) an enzyme E_{II}, which catalyses the conversion of ω-hydroxycarboxylic acids or ω-hydroxycarboxylic acid esters to the corresponding ω-oxocarboxylic acids or ω-oxocarboxylic acid esters, selected from the group consisting of the alcohol dehydrogenases encoded by the *alk*J gene;
iii) an enzyme E_{III}, which catalyses the conversion of ω-oxocarboxylic acids or ω-oxocarboxylic acid esters to the corresponding ω-aminocarboxylic acids or ω-aminocarboxylic acid esters, selected from the group of the ω-transaminases.

2. Cell according to Claim 1, wherein the enzyme E_{I} is the *alkBGT*-encoded alkane monooxygenase from *Pseudomonas putida* GPo1.

3. Cell according to Claim 1 or 2, wherein the enzyme E_{II} is encoded by the *alkJ* gene from *Pseudomonas putida* GPo1.

4. Cell according to one of Claims 1 to 3, wherein the enzyme E_{III} is the ω-transaminase CV2025 from *Chromobacterium violaceum* DSM30191.

5. Cell according to one of the preceding claims, wherein the expression of an enzyme E_{IV}, which catalyses the conversion of ω-aminocarboxylic acid esters to the corresponding ω-aminocarboxylic acids, is increased in the cell.

6. Cell according to Claim 5, wherein the enzyme E_{IV} is the lipase LipA (Q76D26) from *Pseudomonas fluorescens,* which is secreted by the cell.

7. Cell according to one of the preceding claims, wherein the expression of an enzyme E_{V}, which catalyses the conversion of ω-aminocarboxylic acids to the corresponding lactams, is increased in the cell.

8. Cell according to Claim 7, wherein the enzyme E_{V} is secreted by the cell.

9. Cell according to one of the preceding claims, wherein the cell is a genetically modified *Escherichia coli* cell, a genetically modified *Corynebacterium glutamicum* cell or a genetically modified *Pseudomonas putida* cell.

10. Method for the production of ω-aminocarboxylic acids, of ω-aminocarboxylic acid esters or of lactams derived from ω-aminocarboxylic acids, containing the process steps:
I) contacting a cell according to one of Claims 1 to 9 with a culture medium containing a carboxylic acid or a carboxylic acid ester or with a culture medium contiguous with an organic phase containing a carboxylic acid or a carboxylic acid ester in conditions that make it possible for the cell to form ω-aminocarboxylic acids, ω-aminocarboxylic acid esters or lactams derived from ω-aminocarboxylic acids, starting from carboxylic acid or from carboxylic acid esters;
II) optionally isolation of the resultant ω-aminocarboxylic acids, the resultant ω-aminocarboxylic acid esters or the lactams derived from ω-aminocarboxylic acids.

11. Method according to Claim 10, wherein the ω-aminocarboxylic acid esters formed in step I) are converted in another process step by conventional chemical methods to ω-aminocarboxylic acids.

12. Method according to Claim 10 or 11, wherein the cell is a genetically modified *Escherichia coli* cell, a genetically modified *Corynebacterium glutamicum* cell or a genetically modified *Pseudomonas putida* cell.

13. Method according to one of Claims 10 to 12, wherein the culture medium used in step I) contains amino acids, which function as amine donor in the transaminase-catalysed conversion of the ω-oxocarboxylic acids or the ω-oxocarboxylic acid esters to the corresponding ω-aminocarboxylic acids or ω-aminocarboxylic acid esters.

14. Method according to one of Claims 10 to 13, wherein the method is carried out in a two-phase system, containing
A) an aqueous phase, and
B) an organic phase,
where the formation of the ω-aminocarboxylic acids, the ω-aminocarboxylic acid esters or the lactams derived from ω-aminocarboxylic acids by the cells in step I) takes place in the aqueous phase and the resultant ω-aminocarboxylic acids, the resultant ω-aminocarboxylic acid esters or the resultant lactams derived from ω-aminocarboxylic acids accumulate in the organic phase.

15. Method according to one of Claims 10 to 14, wherein the isolation of the resultant ω-aminocarboxylic acids, the resultant ω-aminocarboxylic acid esters or the lactams derived from ω-aminocarboxylic acids takes place by an at least two-stage purification process, comprising
a) an extraction step, in which the ω-aminocarboxylic acids, the ω-aminocarboxylic acid esters or the lactams derived from ω-aminocarboxylic acids are extracted from the culture medium, and
b) a fine purification step, in which the extract obtained in step a) is purified further by distillation methods or additional extraction processes, obtaining an ω-aminocarboxylic acid phase, an ω-aminocarboxylic acid ester phase or a lactam phase with a purity of at least 99.8%.

16. Method according to Claim 15, wherein the extraction in step a) is a reactive extraction.

17. Method according to one of Claims 10 to 16, wherein the carboxylic acid is lauric acid or the carboxylic acid ester is methyl laurate and in that the lauric acid or the methyl laurate is converted in step II) to laurinlactam.

18. Method for the production of polyamides based on ω-aminocarboxylic acids, comprising the process steps:
(α1) production of ω-aminocarboxylic acids by a method according to one of Claims 10 to 17;
(α2) polymerization of the ω-aminocarboxylic acid, obtaining a polyamide.

19. Method for the production of polyamides based on lactams, comprising the process steps:
(β1) production of lactams by a method according to one of Claims 10 to 17;
(β2) ring opening polymerization or polycondensation of the lactam, obtaining a polyamide.

## Revendications

1. Cellule qui a subi une modification génétique par rapport à son type sauvage de façon qu'elle puisse, par comparaison avec son type sauvage, fabriquer plus d'acides ω-aminocarboxyliques, plus d'esters d'acides ω-aminocarboxyliques ou plus de lactames dérivés d'acides ω-aminocarboxyliques, obtenus à partir d'acides carboxyliques ou d'esters d'acides carboxyliques, la cellule présentant une activité des enzymes E_{I} et E_{III}, ou des enzymes E_{I}, E_{II} et E_{III}, augmentée par comparaison avec celles de leur type sauvage :
i) d'une enzyme E_{I}, qui catalyse la conversion d'acides carboxyliques ou d'esters d'acides carboxyliques en les acides ω-hydroxycarboxyliques ou les esters d'acides ω-hydroxycarboxyliques correspondants, choisie dans le groupe consistant en les alcanemonooxygénases : l'alcanemonooxygénase codée par *alkBGT* de *Pseudomonas putida* GPo1, et les cytochromes-P450-monoxygénases de *Candida tropicalis ;*
ii) d'une enzyme E_{II}, qui catalyse la conversion d'acides ω-hydroxycarboxyliques ou d'esters d'acides ω-hydroxycarboxyliques en les acides ω-oxocarboxyliques ou les esters d'acides ω-oxocarboxyliques correspondants, choisie dans le groupe consistant en les alcool-déshydrogénases codées par le gène *alkJ ;*
iii) d'une enzyme E_{III}, qui catalyse la conversion des acides ω-oxocarboxyliques ou des esters d'acides ω-oxocarboxyliques en les acides ω-aminocarboxyliques ou les esters d'acides ω-aminocarboxyliques correspondants, choisie dans le groupe des ω-transaminases.

2. Cellule selon la revendication 1, l'enzyme E_{I} étant l'alcane monoxygénase de *Pseudomonas putidia* GPo1 codée par *alkBGT.*

3. Cellule selon la revendication 1 ou 2, l'enzyme E_{II} étant codée par le gène *alkJ* de *Pseudomonas putida* GPo1.

4. Cellule selon l'une des revendications 1 à 3, l'enzyme E_{III} étant l'ω-transaminase CV2025 de *Chromobacterium violaceum* DSM30191.

5. Cellule selon l'une des revendications précédentes, cellule dans laquelle est augmentée l'expression d'une enzyme E_{IV} qui catalyse la conversion d'esters d'acides ω-aminocarboxyliques en les acides ω-aminocarboxyliques correspondants.

6. Cellule selon la revendication 5, l'enzyme E_{IV} étant la lipase LipA (Q76D26) de *Pseudomonas fluoreszens,* qui est sécrétée par la cellule.

7. Cellule selon l'une des revendications précédentes, cellule dans laquelle est augmentée l'expression d'une enzyme E_{V} qui catalyse la conversion d'acides ω-aminocarboxyliques en les lactames correspondants.

8. Cellule selon la revendication 7, l'enzyme E_{V} étant sécrétée par la cellule.

9. Cellule selon l'une des revendications précédentes, la cellule étant une cellule d*'Escherichia coli* génétiquement modifiée, une cellule de *Corynebacterium glutamicum* génétiquement modifiée, ou une cellule de *Pseudomonas putida* génétiquement modifiée.

10. Procédé de préparation d'acides ω-aminocarboxyliques, d'esters d'acides ω-aminocarboxyliques ou de lactames dérivés d'acides ω-aminocarboxyliques, comprenant les étapes suivantes :
I) mise en contact d'une cellule selon l'une des revendications 1 à 9 avec un milieu de culture contenant un acide carboxylique ou un ester d'acide carboxylique, ou avec un milieu de culture délimitant une phase organique contenant un acide carboxylique ou un ester d'acide carboxylique, dans des conditions permettant à la cellule de former, à partir de l'acide carboxylique ou de l'ester de l'acide carboxylique, des acides ω-aminocarboxyliques, des esters d'acides ω-aminocarboxyliques ou des lactames dérivés d'acides ω-aminocarboxyliques ;
II) éventuellement, isolement des acides ω-carboxyliques formés, des esters d'acides ω-aminocarboxyliques formés ou des lactames dérivés d'acides ω-aminocarboxyliques.

11. Procédé selon la revendication 10, dans lequel les esters d'acides ω-aminocarboxyliques formés dans l'étape I) sont, dans une autre étape, convertis par des procédés chimiques classiques en des acides ω-aminocarboxyliques.

12. Procédé selon la revendication 10 ou 11, dans lequel la cellule est une cellule d'*Escherichia coli* génétiquement modifiée, une cellule de *Corynebacterium glutamicum* génétiquement modifiée ou une cellule de *Pseudomonas putida* génétiquement modifiée.

13. Procédé selon l'une des revendications 10 à 12, dans lequel le milieu de culture utilisé dans l'étape I) contient des acides aminés qui agissent comme des donneurs d'amines lors de la conversion, catalysée par la transaminase, des acides ω-oxocarboxyliques ou des esters d'acides ω-oxocarboxyliques en les acides ω-aminocarboxyliques ou les esters d'acides ω-aminocarboxyliques correspondants.

14. Procédé selon l'une des revendications 10 à 13, le procédé étant mis en oeuvre dans un système diphasique, comprenant :
A) une phase aqueuse, ainsi que
B) une phase organique,
la formation des acides ω-aminocarboxyliques, des esters d'acides ω-aminocarboxyliques ou des lactames dérivés d'acides ω-aminocarboxyliques par les cellules dans l'étape I) ayant lieu en phase aqueuse, et les acides ω-aminocarboxyliques formés, les esters d'acides ω-aminocarboxyliques formés ou les lactames formés, dérivés d'acides ω-aminocarboxyliques, s'enrichissant dans la phase organique.

15. Procédé selon l'une des revendications 10 à 14, dans lequel l'isolement des acides ω-aminocarboxyliques formés, des esters d'acides ω-aminocarboxyliques formés ou des lactames dérivés d'acides ω-aminocarboxyliques s'effectue par un procédé de purification en au moins deux étapes, comprenant :
a) une étape d'extraction, dans laquelle les acides ω-aminocarboxyliques, les esters d'acides ω-aminocarboxyliques ou les lactames dérivés d'acides ω-aminocarboxyliques sont extraits du milieu de culture, ainsi que
b) une étape de purification fine, dans laquelle l'extrait obtenu dans l'étape a) est soumis à une purification plus poussée par des procédés par distillation ou d'autres procédés par extraction, avec obtention d'une phase acide ω-aminocarboxylique, d'une phase ester d'acide ω-aminocarboxylique ou d'une phase lactame, avec une pureté d'au moins 99,8 %.

16. Procédé selon la revendication 15, dans lequel, en ce qui concerne l'extraction dans l'étape a), il s'agit d'une extraction réactive.

17. Procédé selon l'une des revendications 10 à 16, dans lequel l'acide carboxylique est l'acide laurique, ou l'ester d'acide carboxylique est le laurate de méthyle, et l'acide laurique ou le laurate de méthyle est converti dans l'étape II) en laurinelactame.

18. Procédé de préparation de polyamides à base d'acides ω-aminocarboxyliques, comprenant les étapes suivantes :
(α1) préparation d'acides ω-aminocarboxyliques par un procédé selon l'une des revendications 10 à 17 ;
(α2) polymérisation de l'acide ω-aminocarboxylique, avec obtention d'un polyamide.

19. Procédé de préparation de polyamides à base de lactames, comprenant les étapes suivantes :
(β1) préparation de lactames par un procédé selon l'une des revendications 10 à 17 ;
(β2) polymérisation par décyclisation ou polycondensation du lactame avec obtention d'un polyamide.
